# EUROPEAN PATENT SPECIFICATION

(11) **EP 2 664 619 B1**
(45) Date of publication and mention of the grant of the patent: **12.07.2017**
(21) Application number: 12305550.1
(22) Date of filing: 16.05.2012
(51) Int. Cl.: C07D 473/04, C07D 473/16, C07D 473/18, C07D 473/24, C07D 473/34, G01N 33/00

(54) **Purine derivatives as tools for screening anti-Alzheimer compounds**
Purinderivate als Werkzeuge zum Abtasten von Verbindungen gegen Alzheimer
Dérivés de purine en tant qu'outils pour le screening de composés anti-Alzheimer

(43) Date of publication of application: 20.11.2013
(73) Proprietor: Manros Therapeutics, 29680 Roscoff (FR); Centre National de la Recherche Scientifique, 75016 Paris (FR); The Rockefeller University, New York, NY 10065 (US); Université Paris Descartes, 75270 Paris Cedex 06 (FR)
(72) Inventor: Oumata, Nassima, 29680 Roscoff (FR); Meijer, Laurent, 29680 Roscoff (FR); Flajolet, Marc, New York, NY 10065 (US); Greengard, Paul, New York, NY 10065 (US); Bettayeb, Karima, New York, NY 10065 (US); Galons, Hervé, 75014 Paris (FR)
(74) Representative: Nony

(56) References cited:
- WO-A1-01/49688
- WO-A1-98/05335
- WO-A1-99/32660
- BETTAYEB K. ET AL.: "Small-molecule inducers of Abeta-42 peptide production share a common mechanism of action", THE FACEB JOURNAL, vol. 26, no. 12, 1 December 2012 (2012-12-01), pages 5115-5123, XP009165735,
- TANG LI ET AL.: "Crystal structure of pyridoxal kinase in complex with roscovitine and derivatives", THE JOURNAL OF BIOLOGICAL CHEMISTRY, vol. 280, no. 35, 2 September 2005 (2005-09-02), pages 31220-31229, XP002689291,
- RIVKIN A ET AL: "Purine derivatives as potent gamma-secretase modulators", BIOORGANIC & MEDICINAL CHEMISTRY LETTERS, PERGAMON, ELSEVIER SCIENCE, GB, vol. 20, no. 7, 1 April 2010 (2010-04-01), pages 2279-2282, XP026971060, ISSN: 0960-894X [retrieved on 2010-02-06]
- MARIE KNOCKAERT ET AL: "p42/p44 MAPKs are intracellular targets of the CDK inhibitor purvalanol", ONCOGENE, NATURE PUBLISHING GROUP, GB, vol. 21, 1 January 2002 (2002-01-01), pages 6413-6424, XP003027277, ISSN: 0950-9232
- HARMSE L ET AL: "STRUCTURE-ACTIVITY RELATIONSHIPS AND INHIBITORY EFFECTS OF VARIOUS PURINE DERIVATIVES ON THE IN VITRO GROWTH OF PLASMODIUM FALCIPARUM", BIOCHEMICAL PHARMACOLOGY, PERGAMON, OXFORD, GB, vol. 62, no. 3, 1 January 2001 (2001-01-01), pages 341-348, XP001066310, ISSN: 0006-2952, DOI: 10.1016/S0006-2952(01)00644-X
- RAMZAEVA N. ET AL.: "Synthesis and N-alkylation of 6-benzyloxypurine under phase-transfer conditions", SYNTHETIC COMMUNICATIONS, vol. 19, no. 9,10, 1 January 1989 (1989-01-01), pages 1669-1676, XP009165745,
- DAWN E. VERDUGO ET AL: "Discovery of Estrogen Sulfotransferase Inhibitors from a Purine Library Screen", JOURNAL OF MEDICINAL CHEMISTRY, vol. 44, no. 17, 1 August 2001 (2001-08-01), pages 2683-2686, XP055047807, ISSN: 0022-2623, DOI: 10.1021/jm010171u
- BARALDI P G ET AL: "An efficient one-pot synthesis of 6-alkoxy-8,9-dialkylpurines via reaction of 5-amino-4-chloro-6-alkylaminopyrimidines with N,N-dimethylalkaneamides and alkoxide ions", TETRAHEDRON, ELSEVIER SCIENCE PUBLISHERS, AMSTERDAM, NL, vol. 58, no. 38, 16 September 2002 (2002-09-16), pages 7607-7611, XP004379382, ISSN: 0040-4020, DOI: 10.1016/S0040-4020(02)00867-0

## Description

The present invention relates to derivatives of purine and to their use as pharmacological tools to investigate Alzheimer's disease, more particularly in cells and animal models and to screen active ingredients for the treatment of Alzheimer's disease as well as reference compounds in screening methods designed to detect Alzheimer-inducing compounds among molecules constituting the human chemical exposome and to measure their potency.

The present invention more particularly focuses on providing compounds triggering γ-secretase dependent massive production of Aβ₄₂ and down-regulation of Aβ₃₈ in cultured cells and animal models while leaving Aβ₄₀ levels unaffected.

Alzheimer's disease (AD) is an irreversible disease which is irremediably fatal and constitutes a major threat to health systems of the whole world. There are an estimate 25 million patients in the world and 4.6 million new cases each year (1 every 7 seconds). In the absence of new therapeutic agents, projections are 65.7 million cases in 2030, and 115.4 million cases in 2050. The worldwide cost of AD is estimated to reach 248 billion $ each year.

Despite numerous investigations, the causes and mechanisms underlying the initiation and development of this disease still remain poorly understood. There is only relatively mild consensus on the respective roles and importance of the two best described events associated with AD, namely (1) the release of amyloid β peptides by proteolytic processing of the amyloid precursor protein (APP) and their extracellular aggregation in extracellular plaques and (2) the hyperphosphorylation of the microtubule-binding protein Tau and its subsequent intracellular aggregation into paired helical filaments.

Amyloid β peptides are the consequence of the action of two successive proteolytic actions catalyzed first by β-secretase (BACE 1, beta-site APP cleaving enzyme 1) and then by γ-secretase, a complex of four proteins: presenilin-1/2 (PSEN1/PSEN2) (the catalytic subunit, a di-aspartyl protease), nicastrin, APH-1 (anterior pharynx-defective 1) and PEN-2 (presenilin enhancer 2). The action of β-secretase on APP leads to a soluble extracellular fragment and a membrane bound fragment known as C99 or βCTF (β-carboxyl-terminal fragment). γ-Secretase then acts on this C-terminal fragment of APP, leading to the generation of amyloid β peptides of various lengths, such as Aβ₃₈, Aβ₄₀, Aβ₄₂ and Aβ₄₃. Aβ₄₀ is the main amyloid P peptide.

However the generation of Aβ₄₂ is strongly correlated with the onset and development of AD. In autosomal-dominant AD (ADAD), which only represents less than 1% of all AD cases, mutations affect APP, PSEN1 and PSEN2. These mutations all lead to enhanced production of Aβ₄₂ or increase in the Aβ₄₂/Aβ₄₀ ratio, a critical factor in the onset of AD. Aβ₄₂ is much more toxic than Aβ₄₀ and this appears to be linked to its higher hydrophobicity and ability to oligomerize and aggregate in plaques.

Up to now several therapeutics approaches have targeted the effects of neurodegeneration but none of them was found to be able to block the neurodegenerative process. Further compared to other disease such as cancer very few drugs are undergoing clinical tests.

Several models of Alzheimer's disease have been described most of them being based on transgenic rodents which present some aspects of AD's phenotype. However, these models may differ by the mechanism on the onset of the pathology and therefore be essentially helpful for the isolation of compounds which can improve cognitive impairments rather than treat the basic mechanism of the disease.

The lack of molecular markers and of an efficient model which could mimic some of the key steps of AD constitute the two major difficulties in the development of effective treatments of AD.

The inventors have surprisingly found that compounds of formula (I) as described hereinafter are effective tools to overcome these difficulties and therefore are well adapted as pharmacological tools to investigate Alzheimer's disease. Said compounds may be named as Aftins (Amyloid β Forty Two Inducers) within the framework of the present invention.

Said Aftins bind specific mitochondrial proteins (mitofilins, VDAC 1, prohibitin) and reversibly alter mitochondrial structure. They also bind APP and PSEN1. They constitute new pharmacological tools to investigate the molecular mechanism underlying the modified Aβ₄₂/Aβ₄₀/Aβ₃₈ ratio associated with Alzheimer's disease (AD). Aftins can be used in cell models to screen for inhibitors of Aβ₄₂ production and activators of Aβ₃₈ production (potential "anti-AD compounds" - therapeutic applications), and as reference compounds in cellular screens designed to identify natural or anthropogenic products that trigger Aβ₄₂ production and down-regulate Aβ₃₈ production (potential 'pro-AD compounds' - preventive applications).

Aftins can be used in animals to trigger the Aβ₄₂/Aβ₄₀/Aβ₃₈ ratio changes that are observed in Alzheimer's disease patients, thereby constituting a chemical animal model of the disease. Aftins can be used in normal mice but also in genetically modified AD models (APP/PS for ex.) to accelerate the production of Aβ₄₂ and reducing the production of Aβ₃₈, and their subsequent cognitive effects. Disclosed is a compound of formula (I): 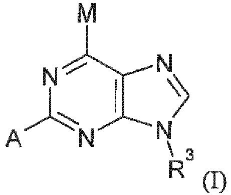 in which
M represents a NR¹R² group, an OR¹ group or a SR¹ group,
A represents a NR⁴R⁵ group, an OR¹⁰ group or a hydrogen atom,
R¹ is an aryl group, a heteroaryl group, a -CH₂-aryl group or a -CH₂-heteroaryl group, said aryl and heteroaryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group, an azido group (-N₃), an aryl group, a heteroaryl group and a (C₁-C₆)alkyl group,
R² is a (C₁-C₆)alkyl group or a (C₁-C₆)cycloalkyl group said alkyl and cycloalkyl being optionally substituted with one or more substituents chosen from a halogen atom, a hydroxyl group and a NH₂ group,
or R¹ forms together with R² and with the nitrogen atom that bears R¹ and R² an heterobicyclic ring,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group, said alkyl being optionally substituted with one or more substituents chosen from a halogen atom, a hydroxyl group and a R⁸ group,
R⁸ represents a hydroxyl group, a NH₂ group, a N(CH₃)₂ group, a 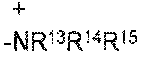 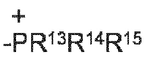 group or a group,
R¹³, R¹⁴ and R¹⁵ represent independently of each other an aryl group or a (C₁-C₆)alkyl group,
x is an integer ranging from 0 to 4,
y is an integer ranging from 2 to 3,
z is an integer ranging from 1 to 10,
p is an integer ranging from 2 to 9,
q is an integer ranging from 2 to 8,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom, a hydroxyl group and a NH₂ group,
R⁴ is a hydrogen atom, a (C₁-C₆)alkyl group, a (C₁-C₆)cycloalkyl group, an aryl group or a heteroaryl group, said alkyl and cycloalkyl being optionally substituted with one or more substituents chosen from a halogen atom, a hydroxyl group and a NH₂ group, said aryl and heteroaryl being optionally substituted with one or more substituents chosen from a halogen atom, a hydroxyl group, a NH₂ group and a NH-R⁹ group,
R⁹ is a heteroaryl group optionally substituted with one or more substituents chosen from a halogen atom, a hydroxyl group, a NH₂ group and a heteroaryl group,
R⁵ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group, an azido group and a NH₂ group, one or more of the carbon atoms of said alkyl or cycloalkyl being optionally replaced by a nitrogen atom,
R¹⁰ is a hydrogen atom, a (C₁-C₆)alkyl group, a (C₁-C₆)cycloalkyl group, an aryl group, a heteroaryl group, a -CH₂-aryl group or -CH₂-heteroaryl group, said aryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group and an azido group (N₃),
with the proviso that the compound of formula (I) is different from the following compounds Aftin-3, Aftin-4, and 30, 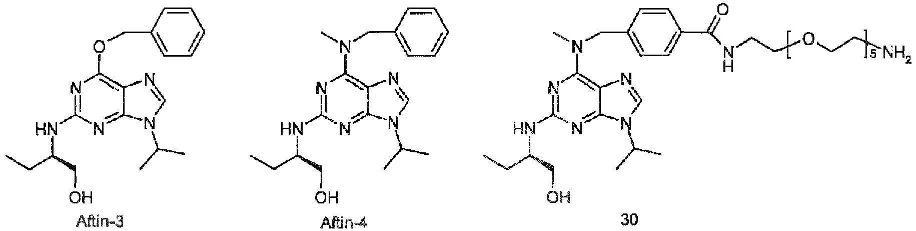
and their addition salts with pharmaceutically acceptable acids.

According to a first aspect, a subject-matter of the present invention relates to the use of a compound of formula (I): 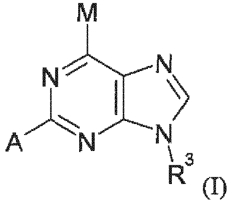 in which
M represents a NR¹R² group, an OR¹ group or a SR¹ group,
A represents a NR⁴R⁵ group, an OR¹⁰ group or a hydrogen atom,
R¹ is an aryl group, a heteroaryl group, a -CH₂-aryl group, a -CH₂-heteroaryl group, said aryl and heteroaryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group, an azido group (-N₃), an aryl group, a heteroaryl group and a (C₁-C₆)alkyl group,
R² is a (C₁-C₆)alkyl group or a (C₁-C₆)cycloalkyl group said alkyl and cycloalkyl being optionally substituted with one or more substituents chosen from a halogen atom , a hydroxyl group and a NH₂ group,
or R¹ forms together with R² and with the nitrogen atom that bears R¹ and R² an heterobicyclic ring,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group, said alkyl being optionally substituted with one or more substituents chosen from a halogen atom, a hydroxyl group and a R⁸ group,
R⁸ represents a hydroxyl group, a NH₂ group, a N(CH₃)₂ group, a 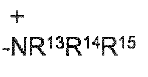 group or a 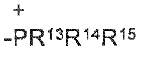 group,
R¹³, R¹⁴ and R¹⁵ represent independently of each other an aryl group or a (C₁-C₆)alkyl group,
x is an integer ranging from 0 to 4,
y is an integer ranging from 2 to 3,
z is an integer ranging from 1 to 10,
p is an integer ranging from 2 to 9,
q is an integer ranging from 2 to 8,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom , a hydroxyl group and a NH₂ group,
R⁴ is a hydrogen atom, a (C₁-C₆)alkyl group, a (C₁-C₆)cycloalkyl group, an aryl group or a heteroaryl group, said alkyl and cycloalkyl being optionally substituted with one or more substituents chosen from a halogen atom, a hydroxyl group and a NH₂ group, said aryl and heteroaryl being optionally substituted with one or more substituents chosen from a halogen atom, a hydroxyl group, a NH₂ group and a NH-R⁹ group,
R⁹ is a heteroaryl group optionally substituted with one or more substituents chosen from a halogen atom, a hydroxyl group, a NH₂ group and a heteroaryl group,
R⁵ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group, an azido group and a NH₂ group, one or more of the carbon atoms of said alkyl or cycloalkyl being optionally replaced by a nitrogen atom,
R¹⁰ is a hydrogen atom, a (C₁-C₆)alkyl group, a (C₁-C₆)cycloalkyl group, an aryl group, a heteroaryl group, a -CH₂-aryl group or -CH₂-heteroaryl group, said aryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group and an azido group (N₃),
in particular Aftin-3, Aftin-4 and Aftin-5, 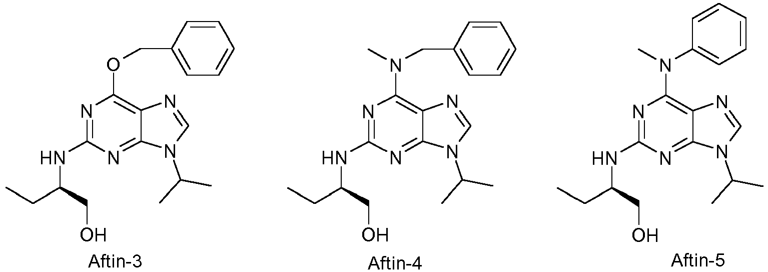 and their addition salts with pharmaceutically acceptable acids,
   as an inducer agent that increases the Aβ₄₂ amyloid β peptides production for *in vitro* screening and *in vivo* screening in animal models, said animal being different from a human being, of compounds that modulate the production of Aβ₄₂ amyloid β peptides.

The compounds Aftin-3, Aftin-4 and 30 are disclosed in Tang et al. J. Biol. Chem. 280, 31220-21229 (2005). In this publication these compounds are used as negative control in a kinase activity test.

In the context of the present invention, the term "halogen" is understood to mean chlorine, fluorine, bromine or iodine.

The term "alkyl" as used herein refers to a linear or branched, saturated aliphatic hydrocarbon group. For instance a (C₁-C₆)alkyl group denotes a linear or branched carbon chain of 1 to 6 carbon atoms. Examples are, but are not limited to, methyl, ethyl, propyl, isopropyl, butyl, methylbutyl.

The term "cycloalkyl" refers to a cyclic alkyl group that may be substituted by one or more (C₁-C₆)alkyl groups. Examples are, but are not limited to cyclopropyl, cyclobutyl, cyclopentyl, methylcyclopropyl, methylcyclobutyl.

The term "aryl" refers to a mono or polycyclic aromatic hydrocarbon radical of 6-20 atoms derived by the removal of one hydrogen from a carbon atom of a parent aromatic ring system. Typical aryl groups include, but are not limited to 1 ring or 2 or 3 rings fused together. Said radical is typically derived from the rings selected from benzene, naphthalene, anthracene, and the like. "Aryl" preferably refers to radicals such as phenyl.

The term "heteroaryl" denotes a 5- or 6-membered aromatic ring comprising 1 or 2 heteroatoms or a bi- or tricyclic aromatic nucleus comprising from 1 to 4 heteroatoms, and at least one of the rings of which has 6 ring members, the other fused ring or rings having 5 or 6 ring members.

The term "heteroatom" is understood to mean nitrogen, oxygen or sulphur.

Preferably, the heteroaryl comprises at least one nitrogen atom. In particular, the heteroaryl does not comprise an oxygen atom. Finally, very particularly preferably, the heteroaryl comprises only nitrogen as heteroatom(s). Thus, advantageously, the heteroaryl comprises from 1 to 4 nitrogen atoms.

Mention may be made of pyridine, thiazole, pyrazole and triazole.

In the context of the present invention, the terms "aromatic ring", "aryl", and "heteroaryl" include all the positional isomers.

The term "heterobicyclic ring" refers to 8 to 14-membered bicyclic radical that comprises at least one heteroatom. Preferably one ring from the heterobicyclic ring is aromatic. In particular the heterobicyclic ring comprises only nitrogen as heteroatom(s). Finally, very particularly the heterobicyclic ring may be chosen from these 2 following radicals:

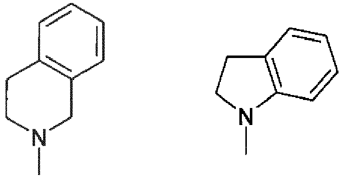

Among the compounds of general formula (I), a first subgroup of compounds is formed from compounds for which M represents a NR¹R² group, an OR¹ group or a SR¹ group;
R¹ is an aryl group or a -CH₂-aryl group, said aryl being optionally substituted with one or more substituents chosen from a halogen atom, a hydroxyl group, an OR⁶ group, a CONR⁶R⁷ group, an azido group (-N₃), an aryl group, a heteroaryl group and a (C₁-C₆)alkyl group,
R² is a (C₁-C₆)alkyl group,
or R¹ forms together with R² and with the nitrogen atom that bears R¹ and R² an heterobicyclic ring,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group,
R⁸ represents a hydroxyl group, a NH₂ group or a 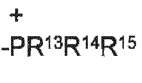 group,
R¹³, R¹⁴ and R¹⁵ represent independently of each other an aryl group,
x is an integer ranging from 0 to 4,
y is an integer ranging from 2 to 3,
z is an integer ranging from 1 to 10,
p is an integer ranging from 2 to 9,
q is an integer ranging from 2 to 8.

Among the compounds of general formula (I), a second subgroup of compounds is formed from compounds for which M represents a NR¹R² group, an OR¹ group or a SR¹ group;
R¹ is an aryl group or a -CH₂-aryl group, said aryl being optionally substituted with one or more substituents chosen from a halogen atom, an OR⁶ group, a CONR⁶R⁷ group, an aryl group, a heteroaryl group and a (C₁-C₆)alkyl group,
R² is a (C₁-C₆)alkyl group,
or R¹ forms together with R² and with the nitrogen atom that bears R¹ and R² an heterobicyclic ring,
R⁶ and R⁷ represent independently of each other a hydrogen atom, a (C₁-C₆)alkyl group or a (CH₂)₂[O(CH₂)₂]₅-NH₂ group.

Among the compounds of general formula (I), a third subgroup of compounds is formed from compounds for which M represents a NR¹R² group, an OR¹ group or a SR¹ group;
R¹ is a phenyl group or a benzyl group, said phenyl and benzyl groups being optionally substituted with one substituent chosen from a fluorine atom, a chloride atom, an OR⁶ group, a CONR⁶R⁷ group, a phenyl group, a pyridinyl group and a methyl group,
R² is a methyl group, an ethyl group, a *n*-propyl group or an isopropyl group,
or R¹ forms together with R² and with the nitrogen atom that bears R¹ and R² an heterobicyclic ring chosen from these 2 following radicals: 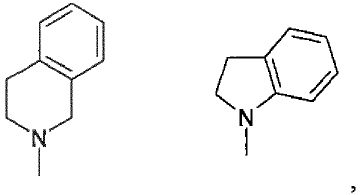 R⁶ and R⁷ represent independently of each other a hydrogen atom, a methyl group or a (CH₂)₂[O(CH₂)₂]₅-NH₂ group.

Among the compounds of general formula (I), a fourth subgroup of compounds is formed from compounds for which A represents a NR⁴R⁵ group, an OR¹⁰ group or a hydrogen atom;
R⁴ is a hydrogen atom or a (C₁-C₆)alkyl group, said alkyl being optionally substituted with one hydroxyl group,
R⁵ is a hydrogen atom, a (C₁-C₆)alkyl group or a (C₁-C₆)cycloalkyl group, said alkyl being optionally substituted with one or two hydroxyl group, one of the carbon atoms of said alkyl or cycloalkyl being optionally replaced by a nitrogen atom,
R¹⁰ is a -CH₂-aryl group.

Among the compounds of general formula (I), a fifth subgroup of compounds is formed from compounds for which A represents a NR⁴R⁵ group, an OR¹⁰ group or a hydrogen atom;
R⁴ is a hydrogen atom or a 2-hydroxyethyl group,
R⁵ is a hydrogen atom, a 1-hydroxybutan-2-yl group, a 1-hydroxy-3-methylbutan-2-yl group, a 1,2-dihydroxypropan-3-yl group, a N-ditehylaminoeth-2-yl group, a piperidin-4-yl group or a 2-hydroxyethyl group,
R¹⁰ is a benzyl group.

Among the compounds of general formula (I), a sixth subgroup of compounds is formed from compounds for which R³ represents a (C₁-C₆)alkyl group, a (C₁-C₆)cycloalkyl group or a -CH₂-aryl group.

Among the compounds of general formula (I), a seventh subgroup of compounds is formed from compounds for which R³ represents a cyclopentyl group, an isopropyl group or a benzyl group.

Among the compounds of general formula (I), an eighth subgroup of compounds is formed by the compounds of general formula (I) in which, simultaneously, A and/or M and/or R³ are as defined in the above groups with the proviso that compounds of general formula (I) are different from Aftin-3, Aftin-4 and compound 30.

According to a particular embodiment, the present invention is directed to the use of a compound of formula (Ia): 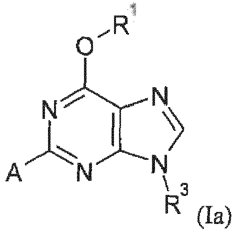 in which
A represents a NR⁴R⁵ group or an OR¹⁰ group,
R¹ is defined as in the compound of formula (I), preferably R¹ is a -CH₂-aryl group and more preferably a benzyl group,
R⁵ is as defined as in the compound of formula (I), preferably R⁵ is a (C₁-C₆)alkyl group or a (C₁-C₆)cycloalkyl group, said alkyl and cycloalkyl being optionally substituted with one hydroxyl group, more preferably R⁵ is a methyl, ethyl, cyclopropyl, cyclobutyl, methylcyclopropyl, methylcyclobutyl or 1-hydroxybutan-2-yl group in particular a 1-hydroxybutan-2-yl group,
R¹⁰ is as defined as in the compound of formula (I), preferably R¹⁰ is a -CH₂-aryl group, more preferably a benzyl group,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom , a hydroxyl group and a NH₂ group, preferably R³ is a (C₁-C₆)alkyl group or a (C₁-C₆)cycloalkyl group, more preferably a methyl, ethyl, cyclopropyl, cyclobutyl, methylcyclopropyl, methylcyclobutyl or isopropyl group, in particular an isopropyl group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group, preferably R⁴ is a hydrogen atom,
and their addition salts with pharmaceutically acceptable acids.

According to a particular embodiment, the present invention relates to the use of a compound of formula (Ia) as defined above in which,
A represents a NR⁴R⁵ group or an OR¹⁰ group,
R¹ is a -CH₂-aryl group, preferably a benzyl group,
R³ is a (C₁-C₆)alkyl group or a (C₁-C₆)cycloalkyl group, preferably a methyl, ethyl, cyclopropyl, cyclobutyl, methylcyclopropyl, methylcyclobutyl or isopropyl group, in particular an isopropyl group,
R⁴ is a hydrogen atom,
R⁵ is a (C₁-C₆)alkyl group or a (C₁-C₆)cycloalkyl group, said alkyl and cycloalkyl being optionally substituted with one hydroxyl group, preferably R⁵ is a methyl, ethyl, cyclopropyl, cyclobutyl, methylcyclopropyl, methylcyclobutyl or 1-hydroxybutan-2-yl group in particular a 1-hydroxybutan-2-yl group,
R¹⁰ is a -CH₂-aryl group, preferably a benzyl group,
and their addition salts with pharmaceutically acceptable acids.

An additional subject matter of the present invention is the use of a compound of formula (Ib): 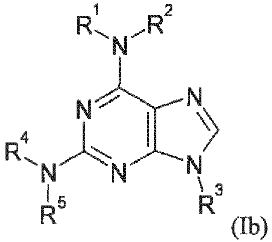 in which
R¹, R² and R⁵ are as defined above,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom, a hydroxyl group and a NH₂ group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group,
and their addition salts with pharmaceutically acceptable acids.

According to a particular embodiment, the present invention relates to the use of a compound of formula (Ib) as defined above, in which, the NR¹R² radicals are chosen from the following radicals: 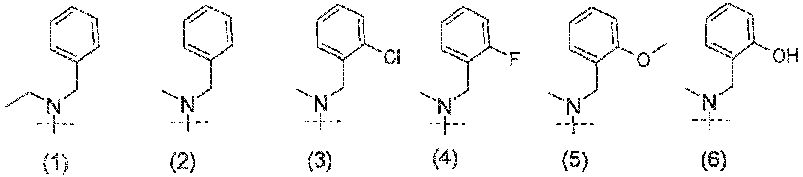 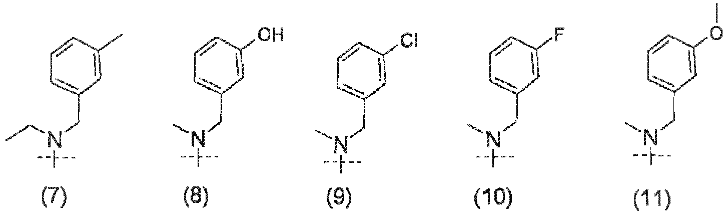 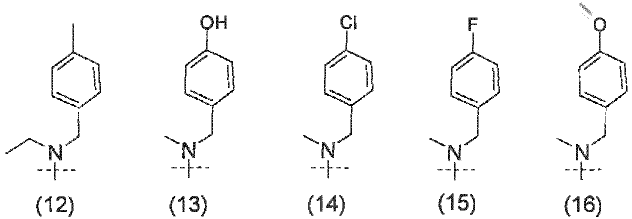 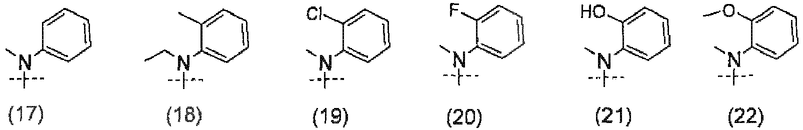 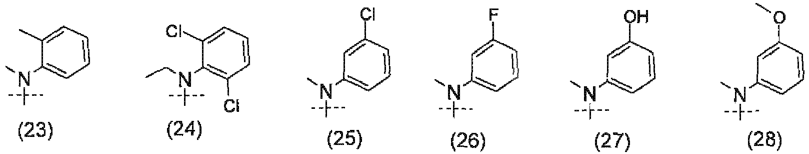 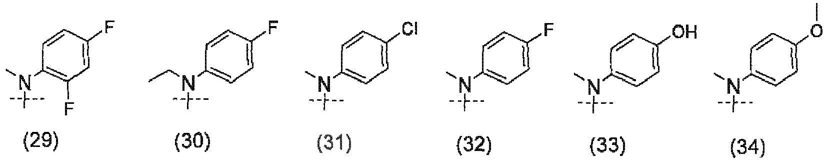 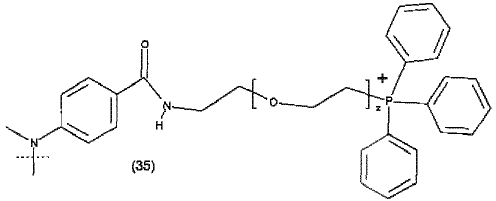 wherein z is ranging from 2 to 9, 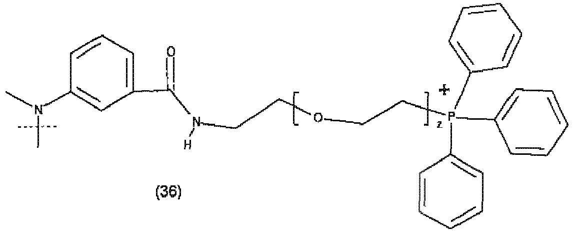 wherein z is ranging from 2 to 9, 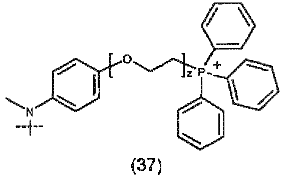 wherein z is ranging from 2 to 9, 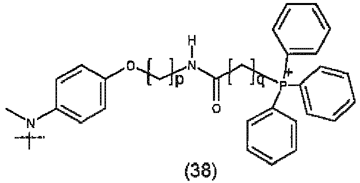 wherein p is ranging from 2 to 9 and q is ranging from 2 to 8, 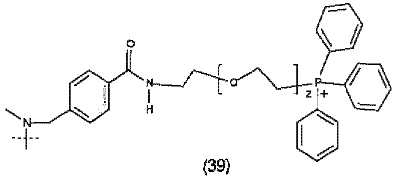 wherein z is ranging from 2 to 9, 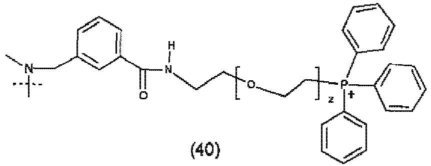 wherein z is ranging from 2 to 9, 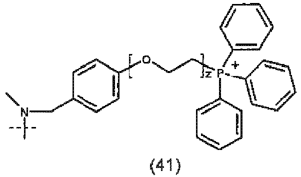 wherein z is ranging from 2 to 9, 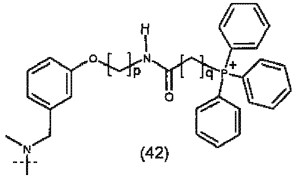 wherein p is ranging from 2 to 9 and q is ranging from 2 to 8,

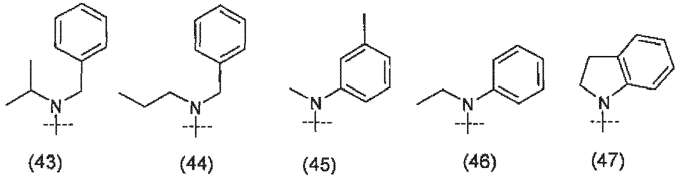

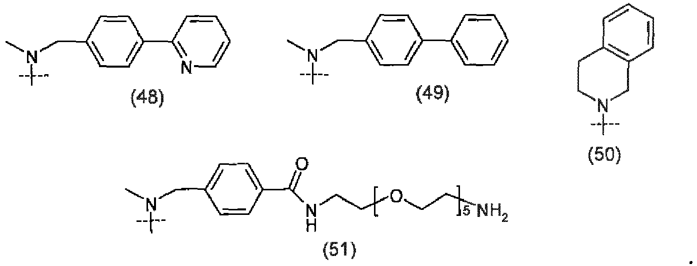

According to a particular embodiment, the present invention relates to the use of a compound of formula (Ib) as defined above, in which,
the NR¹R² radical is as defined above, preferably, NR¹R² radical is chosen from radicals of formulae (1), (2), (11), (15), (17), (25), (32), (34) and (43) to (51) as defined above,
R³ is a (C₁-C₆)alkyl group, a (C₁-C₆)cycloalkyl group or a -CH₂-aryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R⁴ is a hydrogen atom, a (C₁-C₆)alkyl group or a (C₁-C₆)cycloalkyl group, said alkyl and cycloalkyl being optionally substituted with one or more hydroxyl group, preferably R⁴ is a methyl, ethyl, cyclopropyl, cyclobutyl, methylcyclopropyl, methylcyclobutyl or 2-hydroxyethyl group or a hydrogen atom, in particular R⁴ is a 2-hydroxyethyl group or a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said alkyl and cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl, 1-hydroxy-3-methylbutan-2-yl, 1,2-dihydroxypropan-3-yl, N-ditehylaminoeth-2-yl, piperidin-4-yl or 2-hydroxyethyl group, or a hydrogen atom, in particular R⁵ is a 1-hydroxybutan-2-yl, 1-hydroxy-3-methylbutan-2-yl, 1,2-dihydroxypropan-3-yl, N-diethylaminoeth-2-yl, piperidin-4-yl or 2-hydroxyethyl group, or a hydrogen atom,
and their addition salts with pharmaceutically acceptable acids.

According to a particular embodiment, the present invention relates to the use of a compound of formula (Ic): 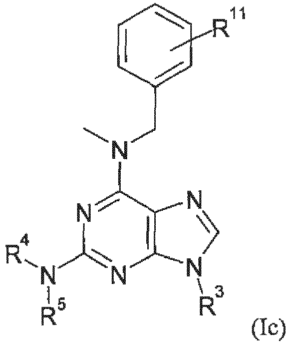 in which,
R³ is a (C₁-C₆)alkyl group, a (C₁-C₆)cycloalkyl group, a -CH₂-aryl group or a -CH₂-heteroaryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R⁴ is a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl, 1,2-dihydroxypropan-3-yl or piperidin-4-yl group, or a hydrogen atom, in particular R⁵ is a 1-hydroxybutan-2-yl, 1,2-dihydroxypropan-3-yl, or piperidin-4-yl group, or a hydrogen atom,
R¹¹ is chosen from a halogen or a hydrogen atom, an OR⁶ group, a CONR⁶R⁷ group, an aryl group and a heteroaryl group, preferably R¹¹ is an OR⁶ CONR⁶R⁷, pyridinyl, phenyl group, or a fluorine or a hydrogen atom,
R⁶ and R⁷ represent independently of each other a hydrogen atom, a (C₁-C₆)alkyl group or a (CH₂)ₓ[O(CH₂)_{y}]_{z}-R⁸ group as defined above, preferably R⁶ and R⁷ represent a hydrogen atom, or a methyl or a (CH₂)₂[O(CH₂)₂]₅-NH₂ group,
and their addition salts with pharmaceutically acceptable acids.

According to a particular embodiment, the present invention relates to the use of a compound of formula (Id): 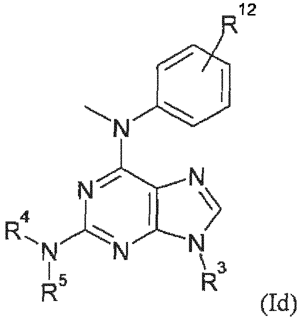 in which,
R³ is a (C₁-C₆)alkyl group, a CH₂-aryl group, a -CH₂-heteroaryl group or a (C₁-C₆)cycloalkyl group, preferably R³ is a methyl, ethyl, cyclopropyl, cyclobutyl, methylcyclopropyl, methylcyclobutyl, isopropyl, cyclopentyl group, in particular R³ is an isopropyl or cyclopentyl group,
R⁴ is a hydrogen atom, a (C₁-C₆)alkyl group or a (C₁-C₆)cycloalkyl group, said alkyl and cycloalkyl being optionally substituted with one or more hydroxyl group, preferably R⁴ is a methyl, ethyl, cyclopropyl, cyclobutyl, methylcyclopropyl, methylcyclobutyl or 2-hydroxyethyl group or a hydrogen atom, in particular R⁴ is a 2-hydroxyethyl group or a hydrogen atom,
R⁵ is a (C₁-C₆)alkyl group or a (C₁-C₆)cycloalkyl group, said alkyl and cycloalkyl being optionally substituted with one or more hydroxyl group, preferably R⁵ is a methyl, ethyl, cyclopropyl, cyclobutyl, methylcyclopropyl, methylcyclobutyl, 1-hydroxybutan-2-yl or 2-hydroxyethyl group, in particular R⁵ is a 1-hydroxybutan-2-yl or 2-hydroxyethyl group,
R¹² is chosen from a halogen or a hydrogen atom, an OR⁶ group and a (C₁-C₆)alkyl group, preferably R¹² is a hydrogen, fluorine or chloride atom, or an OR⁶ or methyl group,
R⁶ is a (C₁-C₆)alkyl group, preferably, R⁶ is a methyl group,
and their addition salts with pharmaceutically acceptable acids.

According to a particular embodiment, the present invention relates to the use of a compound of formula (Ie): 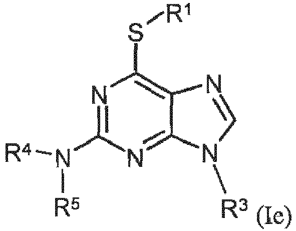 in which
R¹ and R⁵ are as defined above,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom, a hydroxyl group and a NH₂ group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group,
and their addition salts with pharmaceutically acceptable acids.

According to a particular embodiment, the present invention relates to the use of a compound of formula (Ie) as defined above in which,
R¹ is a -CH₂-aryl group, preferably a benzyl group,
R³ is a (C₁-C₆)alkyl group, preferably an isopropyl group,
R⁴ is a hydrogen atom,
R⁵ is a (C₁-C₆)alkyl group being optionally substituted with one or more hydroxyl group, preferably R⁵ is a 1-hydroxybutan-2-yl group,
and their addition salts with pharmaceutically acceptable acids.

According to a second aspect, a subject-matter of the present invention relates to a compound of formula (Ia): 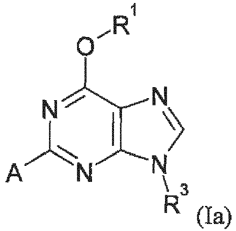 in which
A represents a NR⁴R⁵ group or an OR¹⁰ group,
R¹ is an aryl group, a heteroaryl group, a -CH₂-aryl group, a -CH₂-heteroaryl group, said aryl and heteroaryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group, an azido group (-N₃), an aryl group, a heteroaryl group and a (C₁-C₆)alkyl group, preferably R¹ is a -CH₂-aryl group, more preferably a benzyl group,
R⁵ is a (C₁-C₆)alkyl group or a (C₁-C₆)cycloalkyl group, said alkyl and cycloalkyl being optionally substituted with one or more substituents chosen from a halogen atom, a hydroxyl group, an azido group and a NH₂ group, one or more of the carbon atoms of said alkyl or cycloalkyl being optionally replaced by a nitrogen atom, preferably R⁵ is a (C₁-C₆)alkyl group or a (C₁-C₆)cycloalkyl group, said alkyl and cycloalkyl being optionally substituted with one hydroxyl group, more preferably R⁵ is a methyl, ethyl, cyclopropyl, cyclobutyl, methylcyclopropyl, methylcyclobutyl or 1-hydroxybutan-2-yl group in particular a 1-hydroxybutan-2-yl group,
R¹⁰ is a hydrogen atom, a (C₁-C₆)alkyl group, a (C₁-C₆)cycloalkyl group, an aryl group, a heteroaryl group, a -CH₂-aryl group or -CH₂-heteroaryl group, said aryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group and an azido group (N₃), preferably R¹⁰ is a -CH₂-aryl group, more preferably a benzyl group,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom , a hydroxyl group and a NH₂ group, preferably R³ is a (C₁-C₆)alkyl group or a (C₁-C₆)cycloalkyl group, more preferably a methyl, ethyl, cyclopropyl, cyclobutyl, methylcyclopropyl, methylcyclobutyl or isopropyl group, in particular an isopropyl group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group, preferably R⁴ is a hydrogen atom,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group, said alkyl being optionally substituted with one or more substituents chosen from a halogen atom, a hydroxyl group and a R⁸ group,
R⁸ represents a hydroxyl group, a NH₂ group, a N(CH₃)₂ group, a 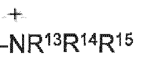 group or a 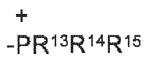 group,
R¹³, R¹⁴ and R¹⁵ represent independently of each other an aryl group or a (C₁-C₆)alkyl group,
x is an integer ranging from 0 to 4,
y is an integer ranging from 2 to 3,
z is an integer ranging from 1 to 10,
p is an integer ranging from 2 to 9,
q is an integer ranging from 2 to 8,
with the proviso that the compound of formula (Ia) is different from Aftin-3, 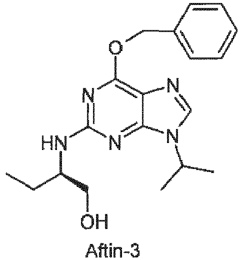 and their addition salts with pharmaceutically acceptable acids.

According to a third aspect, a subject-matter of the present invention relates to a compound of formula (Ib): 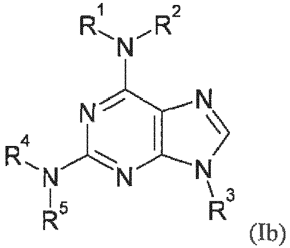 in which,
the NR¹R² radical is chosen from radicals of formulae (1), (2), (11), (15), (17), and (43) to (48), (50) and (51) as defined above,
R³ is a (C₁-C₆)alkyl group, a (C₁-C₆)cycloalkyl group or a -CH₂-aryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R4 is a hydrogen atom, a (C₁-C₆)alkyl group or a (C₁-C₆)cycloalkyl group, said alkyl and cycloalkyl being optionally substituted with one or more hydroxyl group, preferably R⁴ is a methyl, ethyl, cyclopropyl, cyclobutyl, methylcyclopropyl, methylcyclobutyl or 2-hydroxyethyl group or a hydrogen atom, in particular R⁴ is a 2-hydroxyethyl group or a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said alkyl and cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl,1-hydroxy-3-methylbutan-2-yl, 1,2-dihydroxypropan-3-yl, N-ditehylaminoeth-2-yl, piperidin-4-yl or 2-hydroxyethyl group, or a hydrogen atom, in particular R⁵ is a 1-hydroxybutan-2-yl, 1-hydroxy-3-methylbutan-2-yl, 1,2-dihydroxypropan-3-yl, N-diethylaminoeth-2-yl, piperidin-4-yl or 2-hydroxyethyl group, or a hydrogen atom,
with the proviso that the compound of formula (Ib) is different from Aftin-4 and compound 30, 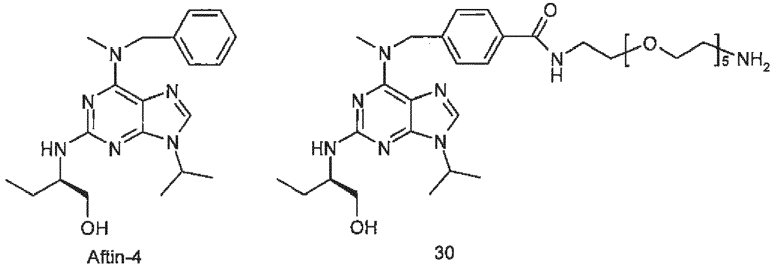
and their addition salts with pharmaceutically acceptable acids.

According to a fourth aspect, a subject-matter of the present invention relates to a compound of formula (Ic): 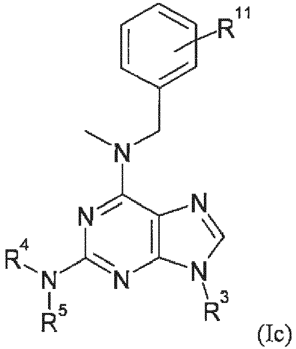 in which,
R³ is a (C₁-C₆)alkyl group, a (C₁-C₆)cycloalkyl group, a -CH₂-aryl group or a -CH₂-heteroaryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R⁴ is a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl, 1,2-dihydroxypropan-3-yl or piperidin-4-yl group, or a hydrogen atom, in particular R⁵ is a 1-hydroxybutan-2-yl, 1,2-dihydroxypropan-3-yl, or piperidin-4-yl group, or a hydrogen atom,
R¹¹ is chosen from a halogen or a hydrogen atom, an OR⁶ group, a CONR⁶R⁷ group, an aryl group and a heteroaryl group, preferably R¹¹ is an OR⁶, CONR⁶R⁷, pyridinyl, phenyl group, or a fluorine or a hydrogen atom,
R⁶ and R⁷ represent independently of each other a hydrogen atom, a (C₁-C₆)alkyl group or a (CH₂)ₓ[O(CH₂)_{y}]_{z}-R⁸ group as defined above, preferably R⁶ and R⁷ represent a hydrogen atom, or a methyl or a (CH₂)₂[O(CH₂)₂]₅-NH₂ group,
with the proviso that the compound of formula (Ic) is different from Aftin-4 and compound 30, 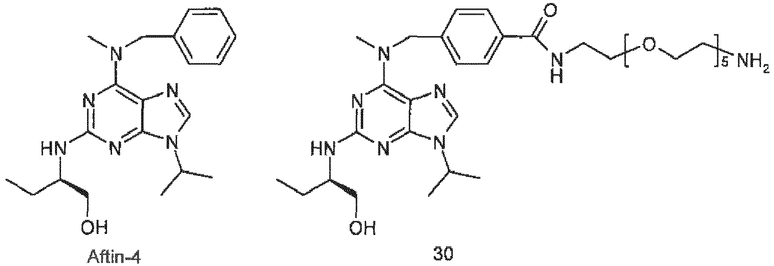 and their addition salts with pharmaceutically acceptable acids.

According to a fifth aspect, a subject-matter of the present invention relates to a compound of formula (Ie): 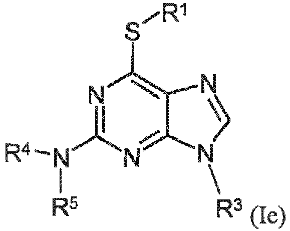 in which
R¹ is an aryl group, a heteroaryl group, a -CH₂-aryl group, a -CH₂-heteroaryl group, said aryl and heteroaryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group, an azido group (-N₃), an aryl group, a heteroaryl group and a (C₁-C₆)alkyl group,
R⁵ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group, an azido group and a NH₂ group, one or more of the carbon atoms of said alkyl or cycloalkyl being optionally replaced by a nitrogen atom,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group, said alkyl being optionally substituted with one or more substituents chosen from a halogen atom, a hydroxyl group and a R⁸ group,
R⁸ represents a hydroxyl group, a NH₂ group, a N(CH₃)₂ group, a 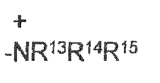 group or a 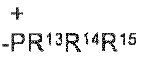 group,
R¹³, R¹⁴ and R¹⁵ represent independently of each other an aryl group or a (C₁-C₆)alkyl group,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom, a hydroxyl group and a NH₂ group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group,
with the proviso that the compound of formula (Ie) is different from the following compound, 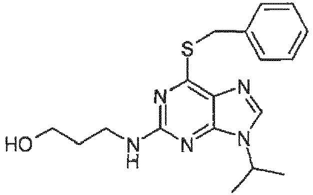 and their addition salts with pharmaceutically acceptable acids.

According to a preferred embodiment of the present invention, the compound is chosen from those disclosed in Table 1, with the proviso that said compound is different from Aftin-3, Aftin-4, compounds 1, 2, 21, 30 and 43, in particular is Aftin-5.

The compounds of formulae (I), (Ia), (Ib), (Ic), (Id) and (Ie) can comprise one or more asymmetric carbon atoms. They can thus exist in the form of enantiomers or of diastereoisomers. These enantiomers, diastereoisomers and their mixtures, including the racemic mixtures, form part of the invention.

The pharmaceutically acceptable salts of the compounds of formulae (I), (Ia), (Ib), (Ic), (Id) and (Ie) include the addition salts with pharmaceutically acceptable acids, such as inorganic acids, for example hydrochloric, hydrobromic, phosphoric or sulphuric acid and organic acids, such as acetic, trifluoroacetic, propionic, oxalic, succinic, fumaric, malic, tartaric, citric, ascorbic, maleic, glutamic, benzoic, toluenesulphonic, methanesulphonic, stearic and lactic acid.

The compounds of formulae (I), (Ia), (Ib), (Ic), (Id) and (Ie) or their salts can form solvates (namely hydrates); the invention includes such solvates.

### GENERAL PROCESS FOR THE PREPARATION OF THE COMPOUNDS

The compounds of the invention may be prepared according to various methods, illustrated by the schemes that follow.

### Compounds of general formula (Ia):

When A is represented by a NR⁴R⁵ group, the synthesis of compounds of general formula (Ia) may be performed according to Scheme 1.

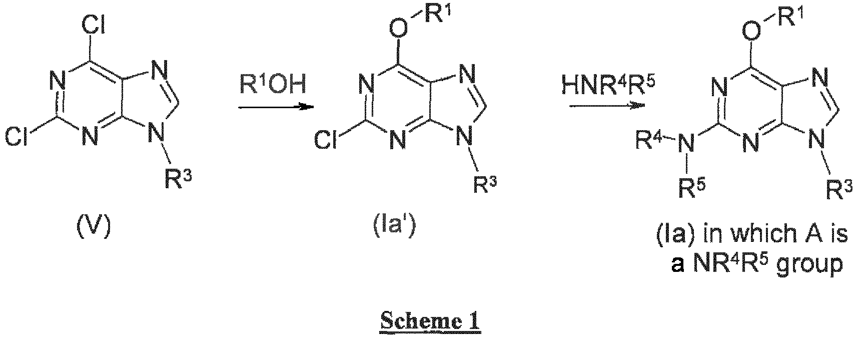

An alcohol R¹OH may be added under nitrogen to a cold (for example 0 °C) suspension of sodium hydride NaH in THF. The mixture can be stirred for example during 0.5 hours for example between 0 °C and 20°C. Compound (V) as defined in Scheme 4 beneath may be slowly added in THF. The mixture can be stirred for example during 2 hours for example at 20 °C. After cooling to 0 °C, NH₄Cl can be carefully added followed by 5 mL water.

After isolation and optional purification, compound (Ia') can be heated in HNR₄R₅, without any added solvent, for example at 120-160 °C for example during 4-6 hours.

When A is represented by an OR¹⁰ group, the synthesis of compounds of general formula (Ia) may be performed according to Scheme 2 starting from the intermediate (Ia') described in Scheme 1.

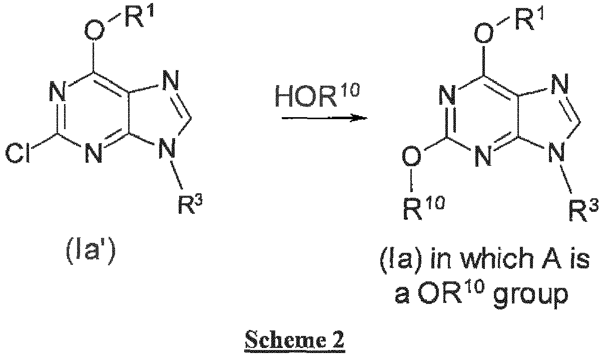

An alcohol R¹⁰OH may be added under nitrogen to a cold (for example 0 °C) suspension of a hydride such as sodium hydride NaH for instance in THF.

Compound (Ia') in a solvent such as THF was slowly added and the mixture may be stirred for example during 6 hours for example between 60 °C and 80 °C.

### Compounds of general formula (Ib):

When R⁴ and R⁵ are represented by a hydrogen atom, compounds of formula (Ib) may be prepared according to the method disclosed in Scheme 3.

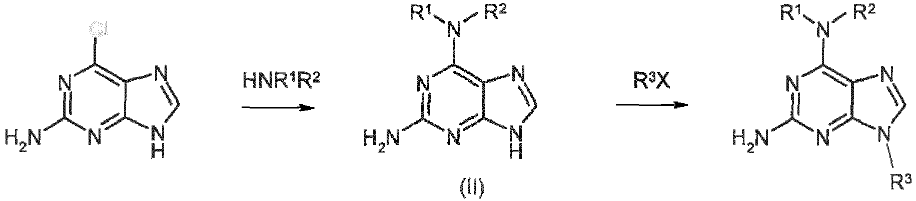

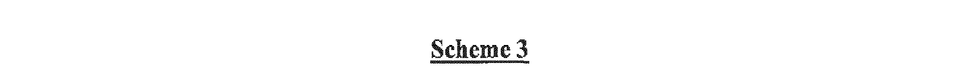

This method consists in adding HNR¹R², wherein R¹ and R² are as defined above, and NEt₃ to a solution of 2-amino-6-chloropurine for example in *n*-butanol, ethanol, *n*-propanol or *iso*-propanol. The mixture can be stirred for example at 80-100 °C for example during 2-6 hours.

The alkylation step may be achieved upon stirring compound (II) (after isolation and optional purification) in a dipolar aprotic solvent such as DMSO with an alkylhalide R³X (X=Cl, Br or I) and a base such as K₂CO₃.

When R⁴ and R⁵ are not both represented by a hydrogen atom, compounds of formula (Ib) may be prepared according to the two routes disclosed in Scheme 4 starting from 2,6-dichloropurine.

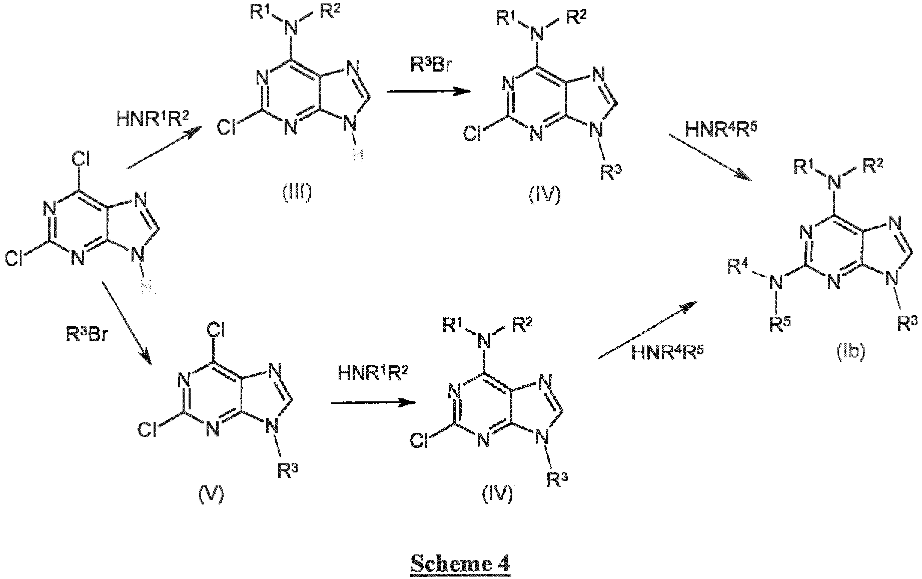

The amination of position 6 may be first performed upon heating 2,6-dichloropurine for example at a temperature comprised between 80 and 100°C with HNR¹R²in an alcohol (for example ethanol, propanol or butanol). Alkylation of (III) may be achieved by using an alkylhalide such as R³Br, wherein R³ is as defined above in a dipolar aprotic solvent such as DMSO in the presence of a basis such as K₂CO₃. In the last step, the amination may be achieved upon heating for instance at a temperature comprised between 140 and 160°C the derivative (IV) with HNR⁴R⁵.

Alternatively, the alkylation leading to (V), may be first performed followed by the two animation steps.

### Compounds of formula (Ie):

They may be prepared according to Scheme 5.

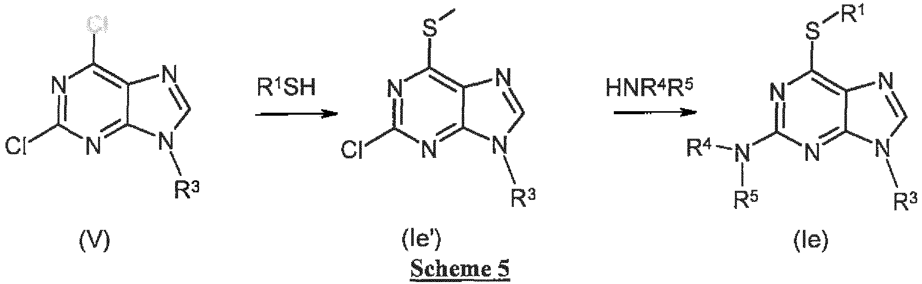

A mixture of compound (V) as defined above, R¹SH, wherein R¹ is as defined above and NEt₃ in an alcohol such as EtOH, propanol or butanol can be heated for example at 70-100 °C for 2 hours. The reaction can be monitored by thin layer chromatography.

After isolation and optional purification, compound (Ie') can be heated in HNR⁴R⁵, wherein R⁴ and R⁵ are as defined above, without any added solvent, for example at 100-120 °C for example during 2-6 hours.

### Compounds for which A represent a hydrogen atom and M represents a NR¹R² group (compounds (If)):

They may be prepared via a two step procedure starting from 6-chloropurine according to Scheme 6.

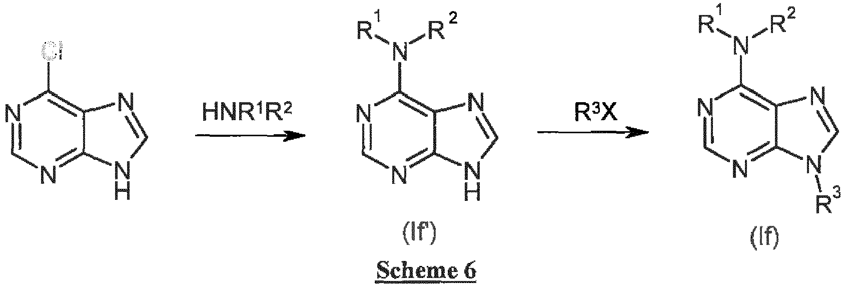

The amination of position 6 may be first performed upon heating 6-chloropurine for example at a temperature comprised between 80 and 100°C with HNR¹R²in an alcohol (for example ethanol, propanol or butanol) in the presence of a base such as NEt₃ for 2 h.

Alkylation of (If) may be achieved by using an alkylhalide R³X (X = Cl, Br or I), wherein R³ is as defined above in a dipolar aprotic solvent such as DMSO in the presence of a basis such as K₂CO₃.

### SCREENING METHOD

As already specified elsewhere herein, the inventors have shown that a compound selected from the group consisting of the compounds of formulae (I), (Ia), (Ib) (Ic), (Id) and (le) used according to the present invention *i.e.* that include Aftin-3, Aftin-4 and compound 30, which are shown herein to modulate the production of β-amyloid peptides, and notably to increase the production of Aβ₄₂ peptide, may be used for the *in vitro* and the *in vivo* screening of compounds that modulate the production of β-amyloid peptides, and more particularly for the *in vitro* and the *in vivo* screening of compounds that inhibit the production of Aβ₄₂-amyloid peptide.

It is recalled that compounds that inhibit the production of Aβ₄₂-amyloid peptide are notably useful for treating Alzheimer's disease.

In this context, this invention also relates to methods for the *in vitro* or the *in vivo* screening of compounds that modulate the production of β-amyloid peptides, and more particularly for the *in vitro* or the *in vivo* screening of compounds that inhibit the production of Aβ₄₂-amyloid peptide, and optionally increase the level of Aβ₃₈-amyloid peptide.

The present invention provides an *in vitro* method for the screening of candidate compounds that modulate the production of a β-amyloid peptide, comprising the steps of :
a) incubating cells able to produce a β-amyloid peptide with an inducing compound selected from the group consisting of the compounds of formulae (I), (Ia), (Ib), (Ic), (Id) and (Ie) used according to the present invention *i. e.* that include Aftin-3, Aftin-4 and compound 30, and further with a candidate compound to be tested, whereby a test sample is provided,
b) measuring the level of a β-amyloid peptide in the test sample obtained at the end of step a),
c) comparing the level of the said β-amyloid peptide measured at step b) with a reference value of the said β-amyloid peptide when step a) is performed in the absence of the said inducing compound,
d) selecting the candidate compound when the said candidate compound modulates the level of the said β-amyloid peptide.

The test sample obtained in step a) does not contain the incubated cells used in the method according to the invention.

In some embodiments, the cells which are able to produce a β-amyloid peptide are selected from the group consisting of neuroblastoma cells, which encompass the N2a cell line and the N2a-APP695 cell line.

In some embodiments, step b) comprises measuring the level of a β-amyloid peptide selected from the group consisting of Aβ₃₈, Aβ₄₀ and Aβ₄₂.

In some embodiments, step b) comprises measuring the level of Aβ₄₂.

In some embodiments, a candidate compound is selected at step d) when the said candidate compound inhibits the level of Aβ₄₂.

This invention further relates to an *in vitro* method for the screening of candidate compounds that modulate the production of a β-amyloid peptide, comprising the steps of:
a) providing a test sample, wherein the said test sample was previously obtained from a non-human mammal that has been administered an inducing compound selected from the group consisting of the compounds of formulae (I), (Ia), (Ib), (Ic), (Id) and (Ie) used according to the present invention *i.e*. that include Aftin-3, Aftin-4 and compound 30, and further with a candidate compound to be tested,
b) measuring the level of a β-amyloid peptide in the test sample obtained at the end of step a),
c) comparing the level of the said β-amyloid peptide measured at step b) with a reference value of the said β-amyloid peptide when step a) is performed in the absence of the said inducing compound,
d) selecting the candidate compound when the said candidate compound modulates the level of the said β-amyloid peptide.

In some embodiments, the test sample provided at step a) consists of a body fluid, which encompasses blood, serum and plasma.

In some embodiments, the test sample was previously obtained from a non-human mammal that has been genetically engineered so as to constitutively produce a β-amyloid peptide, or alternatively so as to over-produce a β-amyloid peptide. In some embodiments, the said non-human mammal is transgenic for constitutive production or overproduction of a β-amyloid peptide.

In some embodiments, step b) comprises measuring the level of a β-amyloid peptide selected from the group consisting of Aβ₃₈, Aβ₄₀ and Aβ₄₂.

In some embodiments, step b) comprises measuring the level of Aβ₄₂.

In some embodiments, a candidate compound is selected at step d) when the said candidate compound inhibits the level of Aβ₄₂.

As intended herein, the "level" of a β-amyloid peptide consists of a quantitative value of the said peptide in a sample, e.g. in a sample collected from cells able to produce the said β-amyloid peptide, particularly after their incubation with an inducing compound selected from the group consisting of the compounds of formulae (I), (Ia), (Ib), (Ic), (Id) and (Ie) used according to the present invention *i.e.* including Aftin-3, Aftin-4 and compound 30. In some embodiments, the said quantitative value does not consist of an absolute value that is actually measured, but rather consists of a fmal value resulting from the taking into consideration of a signal to noise ratio occurring with the assay format used, and/or the taking into consideration of calibration reference values that are used to increase reproducibility of the measures of the level of the said β-amyloid peptide, from assay-to-assay. In some embodiments, the "level" of a β-amyloid peptide is expressed as arbitrary units, since what is important is that the same kind of arbitrary units are compared (i) from assay-to-assay, or (ii) from assays performed at distinct time periods, or (iii) between the β-amyloid peptide level measured in the test sample and a predetermined reference value (which may also be termed a "cut-off" value herein).

Indeed, whatever the kind of numerical units are used for expressing the level of a β-amyloid peptide in a sample, the measured value reflects the amount of β-amyloid peptide that is contained in the tested sample, or most preferably the concentration of β-amyloid peptide in the said sample or the Aβ₃₈/Aβ₄₀/Aβ₄₂ ratios.

At step b) of an *in vitro* screening method according to the invention, the level of a β-amyloid peptide may be measured by any protein detection method that is well known from the one skilled in the art. Thus, the level of a β-amyloid peptide may be measured by any kind of immunoassays including, but not limited to, ELISA, radioimmunoassays, fluorescence immunoassays, immunoaffinity chromatography, immuno-precipitation, mass spectrometry and the like.

In certain embodiments, the level of a β-amyloid peptide is measured by an immunosorbent assay, which encompasses an ELISA method.

At step b) of an *in vitro* screening method according to the invention, the level of a β-amyloid peptide is expressed as a value that refers to an actual signal that is generated, directly or indirectly, by the complexes formed between (i) anti-β-amyloid peptide antibodies used as baits in the antibody detection method and (ii) the β-amyloid peptide present in the patient's sample tested.

As it is conventional when performing antibody detection methods, e.g. immunoassays like ELISA, the signal level (i.e. the β-amyloid peptide level) is expressed as Arbitrary Units (AU). Arbitrary Units are generally determined after substracting the background noise signal, the latter being measured from a sample of the same kind than the test sample that is known to be exempt from the said β-amyloid peptide of interest. Generally, the Arbitrary Units are determined after calibration of the antibody detection method, using a serial of calibration standards (e.g. a serial of samples wherein each sample contains a known amount of the β-amyloid peptide of interest, i.e. Aβ₄₂).

At step c) of the *in vitro* screening method of the invention, the quantification value of the level of the β-amyloid peptide of interest that is measured at step b) is compared to a predetermined reference value (i.e. a predetermined reference value that was previously measured in the absence of the candidate compound to be tested).

Thus, an *in vitro* screening method according to the invention is preferably performed by known immunodetection methods. The examples herein illustrate embodiments of performing a screening method of the invention by an enzyme-linked immunosorbent assay (ELISA), which is a highly conventional immunodetection method. Like other immunodetection methods, an ELISA may be performed either as a non-competitive immunoassay or as a competitive immunoassay.

In a non-competitive ELISA, unlabeled antibody (e.g. an anti-β-amyloid peptide antibody) is bound to a solid support or reaction vessel, such as the surface of a microtiter plate or biochip.

Then, the biological sample is combined with the antibody bound to the reaction vessel, and the β-amyloid peptides present in the biological sample are allowed to bind to the antibodies immobilized on the solid support, thus forming immune complexes.

After the immune complexes have formed, excess biological sample is removed and the vessel is washed to remove non-specifically bound β-amyloid peptide. The immune complexes are then reacted with an appropriate enzyme-labeled anti-β-amyloid peptide antibody (which may be also termed "secondary antibody", which secondary antibody binds to an epitope of the β-amyloid peptide distinct from the epitope recognized by the antibody immobilized on the support). The secondary antibody reacts with β-amyloid peptide in the immune complexes, not with other antigens bound to the reaction vessel. Secondary antibodies specific for binding β-amyloid peptides are well known in the art and are commercially available.

After a second wash step, the enzyme substrate is added. The enzyme linked to the secondary antibody catalyzes a reaction that converts the substrate into a detectable product. When excess antigen is present, the amount of product is directly proportional to the amount of the β-amyloid peptide of interest present in the test sample.

In some embodiments, the antibody which is immobilised on a solid support consists of an antibody possessing the ability to bind to any one of the Aβ₄₂, Aβ₄₀ and Aβ₃₈ amyloid peptides. In these embodiments, the immobilized antibody may consists of an antibody that binds to a common peptide region that is present in each of the Aβ₄₂, Aβ₄₀ and Aβ₃₈ amyloid peptides, e.g. the N-terminal end of these β-amyloid peptides, like the 1-16 N-terminal region of these β-amyloid peptides. Illustratively, the immobilized antibody may be the monoclonal antibody "6E10" (marketed under Ref NE1003 by Calbiochem).

In some embodiment, the second antibody used for performing the ELISA Assay specifically binds to only one of the Aβ₄₂, Aβ₄₀ and Aβ₃₈ amyloid peptides. Illustratively, an anti-Aβ₄₂ antibody may consist of the antibody marketed under Ref "Anti-beta Amyloid 1-42 antibody" by Abcam. Illustratively, an anti-Aβ₄₀ antibody may consist of the antibody which is marketed under Ref PA3-16760 by Pierce. Illustratively, an anti-Aβ₃₈ antibody may consists of the antibody which is marketed under Ref SIG-39162 by Covance.

In some embodiments, the product is fluorescent or luminescent, which can be measured using technology and equipment well known in the art. In some embodiments, the enzyme reaction results in the conversion of the enzyme substrate into a coloured product, which can be measured spectrophotometrically.

Typical enzymes that can be linked to secondary antibodies include horseradish peroxidise, glucose oxidase, glucose-6-phosphate dehydrogenase, alkaline phosphatase, beta-galactosidase and urease.

For performing ELISA, suitable solid supports include organic and inorganic polymers, e.g. dextrans, natural or modified celluloses, polyethylene, polystyrene, polyacrylamides, etc.

Specific embodiments of an ELISA assay which may be useful for performing an *in vitro* screening method of the invention are further detailed hereafter.

An ELISA assay consists of a method where a first antibody, also known as the capture antibody, is attached to a solid support by its constant region. This antibody is diluted in a buffer and can be attached passively to the solid support, or actively. An active attachment can for example be illustrated with the use of a biotinylated antibody which is added to a streptavidine-coated solid support.

This step is followed by the binding of the Aβ₄₂ amyloid β peptides present in said biological sample to the variable regions of this antibody under given proper conditions.

A washing step follows in order to only conserve the Aβ₄₂ amyloid β peptides retained on the solid support through the attached antibody as the unbound products are removed by the wash.

Then a labeled second antibody, also called the detection antibody, diluted in a buffer, is allowed to bind to the Aβ₄₂ amyloid β peptides.

The amount of the antigen is then quantified by measuring the amount of labeled second antibody bound to the matrix. In case of a detecting antibody linked to an enzyme a colorimetric assay can be performed and a change in color determined. It is thus possible to determine in a qualitative and quantitative way the presence of Aβ₄₂ amyloid β peptides in the tested sample.

In one embodiment of the invention, when the detecting antibody is enzyme linked, the method of the invention relates to a sandwich ELISA method.

Several possible combinations of antibodies can be envisaged when a sandwich technique is employed as described here-above, i.e. when a capture antibody or antibodies immobilized on a solid support are used in association with a labeled detecting antibody or antibodies (See The ELISA Guidebook; Second Edition; Methods in Molecular Biology; John R. Crowther; November 19 2010).

The capture antibody can be any antibody having affinity towards the Aβ₄₂ amyloid β peptides and which antibody will bind Aβ₄₂ amyloid β peptides.

As described above, one way of detecting binding of the detecting antibody to the Aβ₄₂ amyloid β peptides is by ELISA in which an enzyme conjugated antibody is employed and the amount of antibody bound is determined by a colorimetric assay. In particular, the ELISA is a sandwich ELISA. However, other means for detecting antibody binding can be employed as well.

One well known technique for monitoring biomolecular interactions between antigens, such as the Aβ₄₂ amyloid β peptides, and an antibody is by surface plasmon resonance (SPR) (R.B.M. Schasfoort and Anna J. Tudos Handbook of Surface Plasmon Resonance, 2008).

Other possible means suitable for detecting interactions between antibodies and antigen is by piezo electric biosensors in which the parameter which is measured is resistance, current or voltage (Ashok Kumar et al., "Design and Implementation of a Piezoelectric Biosensors for Multifunctional Applications", Integrated Design and Process Technology, IDPT-Vol. 1 (1998), pp. 35-41).

A biological sample according to the invention can be chosen among a body fluid, such as blood, plasma, leukocyte extract, saliva, urine, bile, tears, or breast milk, or the supernatant of a culture medium, a lysate or a cellular or tissue extract.

In particular, as the present screening methods are used in order to determine compounds that are able to modulate the production of Aβ₄₂ amyloid β peptides, said biological sample is obtained either from an animal, different from a human being, treated with Aftins used according to the invention, *i.e.* that include Aftin-3, Aftin-4 and compound 30 or from a culture medium of neural cells, in particular from a mouse, for example Mouse neuroblastoma cells (N2a cells) or N2a-APP695 cells (N2a cell line stably transfected with APP695, an amyloid precursor protein), primary neuronal cells, or any cell line expressing the amyloid precursor protein APP, treated with Aftins used according to the invention *i.e.* that include Aftin-3, Aftin-4 and compound 30.

A screening method according to the invention can also be performed *in vivo* in an animal different from a human being.

This screening method allows evaluating the effect of tested compounds, according to standards procedures known to the art, on the brain neuropathophysiology and the animal cognitive abilities, this animal being preferentially previously exposed to compounds according to the invention. Standards procedures are notably disclosed in HuangY, Mucke L (2012) Alzheimer mechanisms and therapeutics strategies. Cell 148, 1204-1222 and Selkoe DJ, Mandelkow E, Holtzman DM (editors) (2012) The Biology of Alzheimer Disease Cold Spring Harbor Press, Cold Spring Harbor, New York, 511 pp.

As indicated previously, a screening method according to the invention can also be used in order to determine the presence and quantity of other antigens, such as Aβ₃₈ and Aβ₄₀ peptides, and thus determine the activity of a tested compound on the production of those peptides. The antibodies used in the methods must obviously be chosen in accordance with the peptides concerned.

Accordingly, the present invention relates to the use of a compound of formula (I), (Ia), (Ib), (Ic), (Id) and/or (Ie) used according to the invention, *i.e.* that includes Aftin-3, Aftin-4 and compound 30, as an inducer agent that increases the Aβ₄₂ amyloid β peptides production, and decreases the Aβ₃₈ amyloid β peptides production for *in vitro* and *in vivo* screening of compounds that modulate the production of Aβ₄₂ amyloid β peptides and that consequently modifies the Aβ₃₈/Aβ₄₀/Aβ₄₂ ratios.

More particularly, the present invention relates to the use of a compound disclosed in Table 1 as an inducer agent that increases the Aβ₄₂ amyloid β peptides production and decreases the Aβ₃₈ amyloid β peptides production for *in vitro* and *in vivo* screening of compounds that modulate the production of Aβ₄₂ amyloid β peptides and the Aβ₃₈/Aβ₄₀/Aβ₄₂ ratios, in particular the present invention relates to the use of Aftin-3, Aftin-4 or Aftin-5.

According to a preferred embodiment, compounds used according to the present invention can be used to screen compounds aimed at treating Alzheimer's disease.

According to another embodiment, compounds used according to the present invention can be used as reference compounds in *in vitro* screening methods and *in vivo* screening methods in animal models, said animal being different from a human being, designed to detect Alzheimer-inducing compounds among molecules constituting the human chemical exposome and to measure their potency.

Indeed compounds used according to the present invention can be used as reference compounds in the very same cell culture and amyloid detection system as those used to detect Alzheimer-inducing compounds, namely compounds that up-regulate Aβ₄₂ and down-regulate Aβ₃₈, and thus modulate the Aβ₃₈/Aβ₄₀/Aβ₄₂ ratios.

These compounds represent potential hazards as possible 'pro-Alzheimer' compounds (Alzheimer inducing compounds) deserve careful handling, and controlled/regulated exposure. These 'pro-Alzheimer' compounds potentially comprise natural products but also anthropic molecules man is exposed to (the so-called 'human chemical exposome': herbicides, pesticides, fungicides, flame retardants, paint ingredients, household products, plastics and plasticizers, monomers and polymerization catalyzers for polymers used for food packaging, industrial by-products, metals, cosmetic ingredients, sunscreens, analgesics, 'comfort' medicines, dental amalgams, food additives (E series), food preservatives, drugs on the market, in phase 2 or 3 clinical trials...).

It is believed the simple screening test according to the invention, using Aftins as positive reference compounds, should be included in safety screening methods designed to detect potentially hazardous molecules among the large number of compounds that are already on or being brought to the market. These molecules indeed may constitute environmental factors contributing to the onset, development and acceleration of Alzheimer disease. Their detection and identification would constitute a first step in a preventive action to reduce or even eradicate late onset Alzheimer disease which accounts for more than 99 % of all Alzheimer disease cases.

### DESCRIPTION OF FIGURES

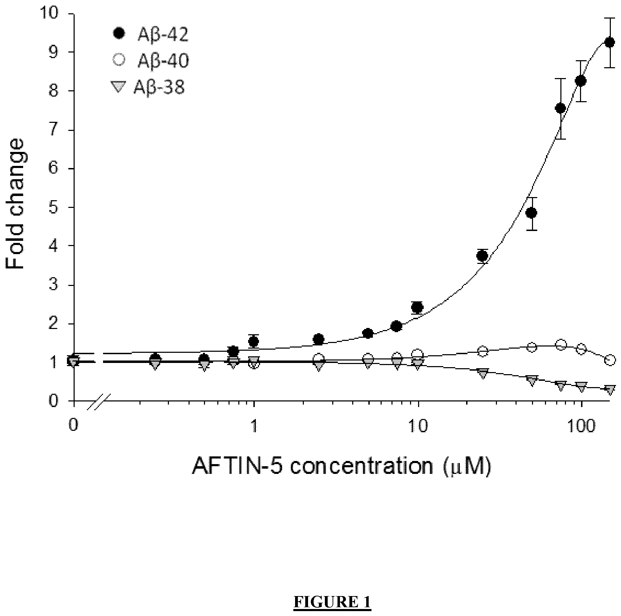

**Figure 1**: Aftin-5 dose-dependent effect on Aβ₃₈, Aβ₄₀ and Aβ₄₂ levels. Cells were exposed to various concentrations of AFTIN-5 for 18 hours. Aβ₃₈, Aβ₄₀ and Aβ₄₂ levels were measured by an ELISA assay and are expressed as fold change over the level of control, vehicle-treated cells.

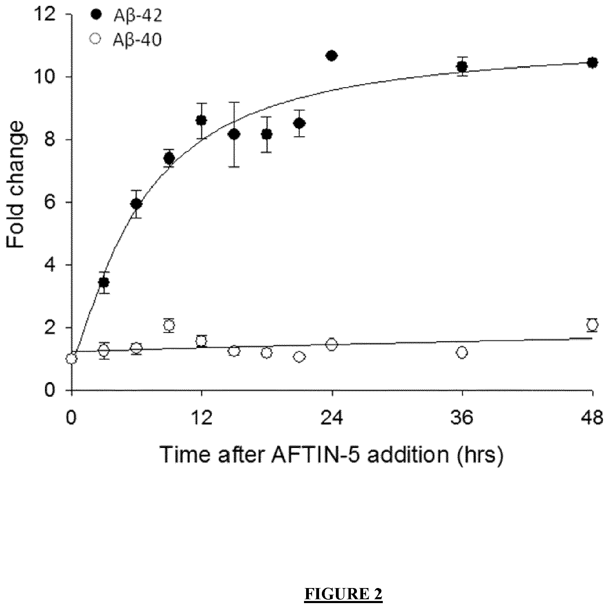

**Figure 2****:** illustrates Aftin-5 time-dependent effect on Aβ₄₀ and Aβ₄₂ levels. Cells were exposed to 100 µM Aftin-5 for various periods of time. Aβ₄₀ and Aβ₄₂ levels were measured by an ELISA assay.

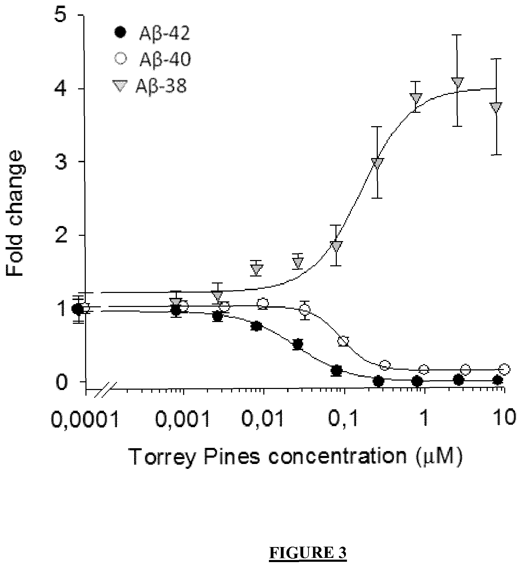

**Figure 3****:** illustrates GSM 'Torrey Pines' dose-dependent effect on Aβ₃₈, Aβ₄₀ and Aβ₄₂ levels in N2a-APP695 cells. Cells were exposed to various concentrations of 'Torrey Pines' compound for 18 hours. Aβ levels were measured by an ELISA assay and are expressed as fold change over the level of control, vehicle-treated cells.

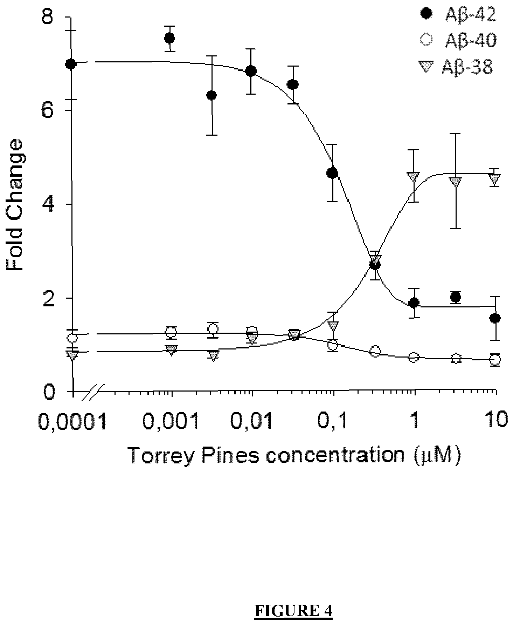

**Figure 4****:** illustrates GSM 'Torrey Pines' dose-dependently inhibition on AFTIN-5 up-regulation of Aβ₄₂ and down-regulation of Aβ₃₈. Cells were exposed to various concentrations of 'Torrey Pines' compound and 1 hr later to 100 µM AFTIN-5. Incubation was carried out for 18 hrs. Aβ levels were measured by an ELISA assay and are expressed as fold change over the level of control, AFTIN-5 treated cells.

The examples which follow illustrate the preparation of a number of compounds of the invention. These examples are not limiting and serve merely to illustrate the invention. The NMR spectra confirm the structures and the purities of the compounds obtained.

Mp represents the melting point in degrees Celsius.

The numbers or names between brackets in the title of the examples correspond to those in the 1^{st} column of Table 1.

### EXAMPLES

### Example 1: Preparation of (2R)-2-[[9-isopropyl-6-(N-methylanilino)purin-2-yl]amino]butan-1-ol (Aftin-5)

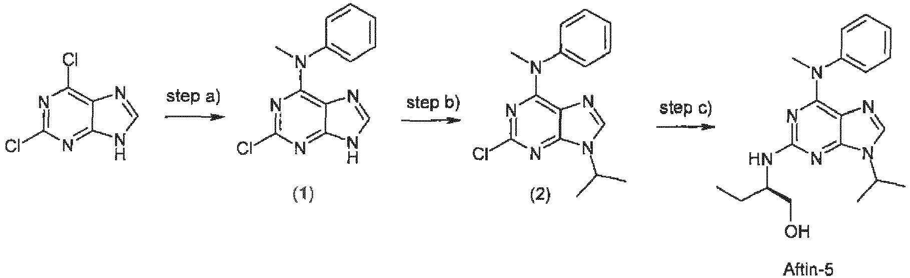

Step a): N-methylaniline (8.6 mL, 79.36 mmol) and triethylamine (29 mL, 211.63 mmol) were added to a solution of 2,6-dichloropurine (10.0 g, 52.91 mmol) in n-butanol (80 mL). The mixture was heated at 100 °C for 3 hours. The reaction was cooled to 40 °C, the precipitate was filtered at this temperature, washed with 10 mL of cold (15°C) water and dried overnight under vacuum.

Step b): 2-Bromopropane (25.77 mL, 275.37 mmol) was added to a solution of (1) (10.22 g, 39.34 mmol) and K₂CO₃ (21.74 g, 157.35 mmol) in 80 mL DMSO. After 12 h of stirring, the mixture was diluted with 200 mL of water and extracted with AcOEt (3x20 mL). After concentration, the remaining solid was triturated with 5 mL AcOEt filtered and dried overnight in vacuo.
Yield: 65%
¹H-NMR(DMSO-d₆) δ ppm: 1.47(d, 6H, (CH₃)₂CH); 3.12 and 3.60(2 brs, 3H, CH₃N); 4.75(hept, 1H, J = Hz) 4.90 and 5.50(2 brs, 2H, CH₂C₆H₅); 75(s, 1H, 8-H).
¹³C-NMR δ ppm: 22.7, 46.62, 119.07, 127.46, 128.64; 136.12; 137.31; 153.65.

Step c): A mixture of the chloro derivative (2) (11.5 g, 37.99 mmol) and (R)-2-aminobutanol (28.47 mL, 304 mmol) was heated 4 hours at 160 °C. After cooling, the mixture was extracted with CH₂Cl₂ washed with water (2x20 mL). The product obtained crystallized upon trituration with AcOEt.
Yield: 63%.
¹H-NMR(CDCl₃) δ ppm: 0.94(t, 3H, CH₃); 1.48(d, 6H, (CH₃)₂CH); 1.50(m, 2H, CH₂CH₃); 3.50 and 3.67(2m, 1H and 2H); 3.72(s, 3H, CH₃N); 4.56(hept, 1H, (CH₃)₂CH); 7.24-7.38(m, 5H, C₆H₄).

### Example 2: Preparation of (2R)-2-[[9-isopropyl-6-[(4-methoxyphenyl)-methyl-amino]purin-2-yl]amino]butan-1-ol (44)

The procedure used for the synthesis of Aftin-5 was applied to prepare 44. The only difference consists in the fact that N-Methyl-p-anisidine was used instead of N-methylaniline in step a). 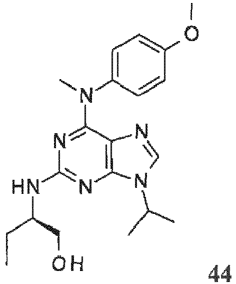 Yield: 65%.
RMN ¹H (CDCl₃); δ ppm: 0.95 (t, 3H, CH₃-CH₂); 1,4 (m, 2H, CH₃-CH₂); 1,45 (d, 6H, 2 x CH₃-CH); 3,55 (m, 1H, CH-NH); 3,65 (s, 3H, CH₃-N); 3,7 (m, 2H, CH₂-OH); 3,8 (s, 3H, O-CH₃); 4,55 (hept, 1H, CH-(CH₃)₂); 6.9 (d, 2H, C₆H₄); 7,15(d, 2H, C₆H₄); 7,4 (s, 1H, 8-H).

### Example 3: Preparation of (2R)-2-[[6-[benzyl(isopropyl)amino]-9-isopropylpurin-2-yl]amino]butan-1-ol (23)

The procedure used for the synthesis of Aftin-5 was applied to prepare **23.** The only difference consists in the fact that N-isopropylbenzylamine was used instead of N-methylaniline in step a). 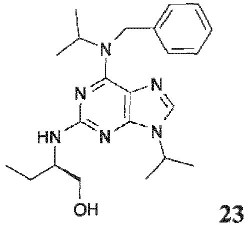 Yield: 55%.
Mp: 108-111 °C.
¹H NMR (CDCl₃); δ ppm: 0.85 (t, 1H, CH₃); 1.20 (m, 6H, 2 CH₃); 1.51 (d, 6H, 2 CH₃); 3.40 and 3.60 (2m, 3H, CHCH₂); 4.51 (hept, 1H,); 4.85 (m, 3H); 7.15 (brs, 5H, C₆H₅); 7.52 (s, 1H, 8-H).

### Example 4: Preparation of N6-benzyl-9-isopropyl-N6-methyl-purine-2,6-diamine (Aftin-1)

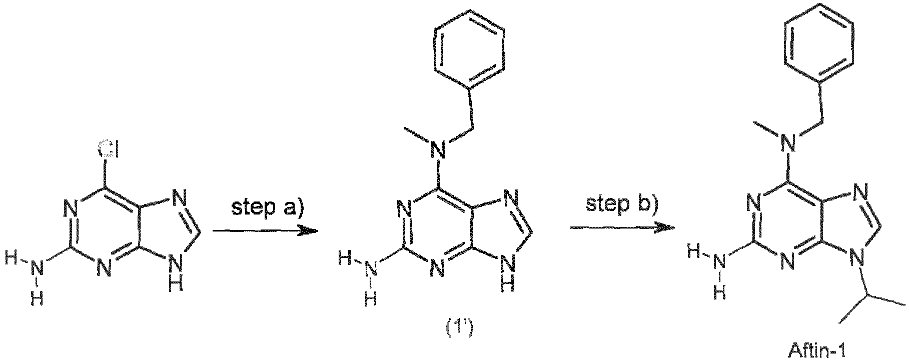

Step a): obtention of N6-Benzyl-N6-methyl-9H-purine-2,6-diamine (1'):
N-Methylbenzylamine (1.92 mL, 15 mmol) and 2 mL NEt₃ were added to a solution of 2-amino-6-chloropurine (1.69, 10 mmol) in 30 mL *n*-butanol. The mixture was stirred at 85°C for 6 h. After cooling to 20°C, (1') separated upon filtration of the precipitate.
   Yield: 74%.
   Mp: 130°C.
   ¹H-NMR (CDCl₃); δ ppm: 3.35 (brs 3H); 4.75 and 5.25 (2 brs, CH₂); 7.35 (m, 5H, C₆H₅); 7.65 (s, 1H, 8-H).

Step b): obtention of N6-Benzyl-9-isopropyl-N6-methyl-purine-2,6-diamine (Aftin-1). A mixture of N6-benzyl-N6-methyl-9H-purine-2,6-diamine (1.60 g, 5 mmol), K₂CO₃ (3.45 g, 25 mmol), was stirred in 50 mL DMSO at 10°C for 5 min. To this mixture, *iso*-propylbromide (2.4 mL, 25 mmol) was added slowly and the mixture was stirred for 24 h at rt. After addition of 500 mL H₂O and was extracted with AcOEt (3x20 mL). The organic layers were concentrated in vacuo and Aftin-1 was isolated upon trituration with Et₂O.
Yield: 72%.
Mp: 110°C.
¹H-NMR (CDCl₃); δ ppm: 1.45 (d, 6H, CH(CH₃)₂); 3.30 (brs, CH₃N); 4.51(brs, CH₂); 4.62 (hept, 1H, CH(CH₃)₂); 5.23 (brs, NH₂); 7.2 (m, 5H, C₆H₅); 7.48 (s, 1H, 8-H). ¹³C-NMR (CDCl₃); δ ppm: 22.74 (CHCH₃); 35.83 (brs, CH₃N); 45.76 (CH); 53.26 (CH₂N); 115.25 (CH); 127.07 (CH); 127.65 (CH); 128.49 (CH); 129.05; 133.53 (CH); 138.33 (CH); 152.54 (CH); 155.27 (CH); 159.08 (CH).

### Example 5: Preparation of N6-Benzyl-9-cyclopentyl-N6-methyl-purine-2,6-diamine (Aftin-2)

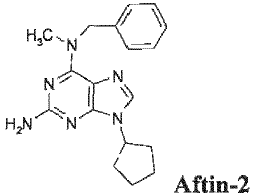

The procedure used for the synthesis of Aftin-1 was applied to prepare Aftin-2. The only difference consists in the fact that 2-bromocyclopentane was used instead of isopropylbromide in step b).
Yield: 71 %.
Mp: 160°C.
¹H NMR (CDCl₃); δ ppm: 1.70 (m, 2H, cyclopentyl); 1.83 (m, 4H, cyclopentyl); 2.16 (m, 2H, cyclopentyl); 3.28 (brs, 3H, CH₃); 4.61 (brs, 2H, CH₂-C₆H₅); 4.73 (quint, 1H, cyclopentyl); 4.61 (brs, 2H, CH₂); 5.21 (brs, NH₂); 7.25 (m, 5H); 7.46 (s, 1H).

### Example 6: Preparation of 9-Iso-propyl-2,6-dibenzyloxypurine (46)

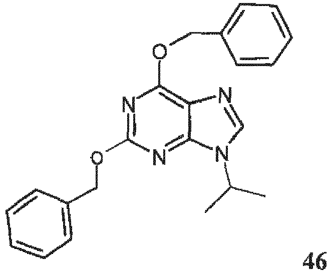

9-Iso-propyl-2,6-dibenzyloxypurine 46 was prepared from 2,6-dichloro-9-isopropylpurine. Benzyl alcohol (4 g, 37 mmol) in 60 mL THF was added at 0 °C under nitrogen to a suspension of NaH (1.31g, 54.6 mmol) in 60 mL THF. The mixture was stirred at 20 °C for 1 h. A solution of 2,6-dichloro-9-isopropylpurine (3.64 g, 15.8 mmol) in 30 mL was then added. The mixture was stirred at 60 °C for 2h. After cooling at 0 °C, 1 g of NH₄Cl was added with caution followed by 50 g of ice. The mixture was extracted with CH₂Cl₂ (3x50 mL).
Yield 65%.
¹H-NMR (CDCl₃); δ ppm: 1.50 (d, 6H, 2CH₃); 4.80(hept, 1H, CH(CH₃)₂); 5.38 and 5.60 (2s, 2x2H, 2CH₂); 7.20-7.40 (m, 10H, 2 C₆H₅); 7.85(s, 1H, 8-H).
¹³C-NMR (CDCl₃) δ ppm: 22.50, 47.18, 68.53, 69.51, 117.70, 127.96, 128.08, 128.23, 128.41, 128.43, 136.17, 136.90, 138.58, 140.48, 153.39, 160.61, 161.31.

### Example 7: Preparation of N-benzyl-9-isopropyl-N-methyl-purin-6-amine (9)

Compound 9 was prepared *via* a two step procedure starting from 6-chloropurine.

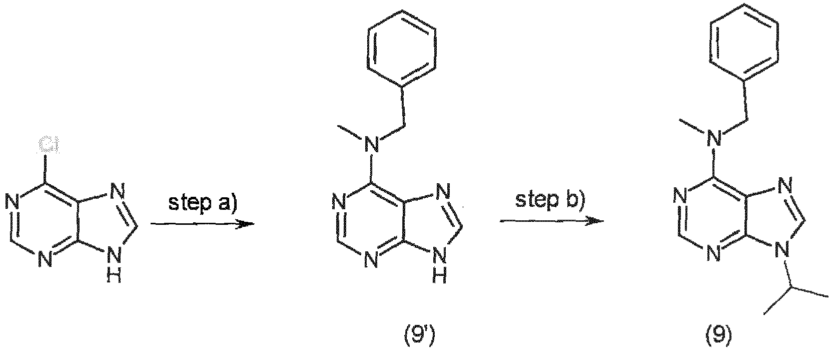

A mixture of 6-chloropurine (1.54 g, 10 mmol), N-methylbenzylamine (1.92 mL, 15 mmol) and 2 mL NEt₃ in n-butanol 30 mL is heated at 100 °C for 2h. After cooling, the intermediate N-benzyl-N-methyl-purin-6-amine (**9'**) which crystallized was filtered, washed with 5 mL cold (0 °C) butanol. After drying it was used for the next step. A mixture of N-benzyl-N-methyl-purin-6-amine (1.20 g, 5 mmol) and K₂CO₃ (3.45 g, 25 mmol) was stirred in 50 mL DMSO at 10°C for 5 min. To this mixture, iso-propylbromide (2.4 mL, 25 mmol) was added slowly and the mixture was stirred for 24 h at room temperature. After addition of 500 mL of water, the mixture was extracted with AcOEt (3x20 mL). The organic layers were concentrated in vacuo and compound **9** was isolated upon trituration with Et₂O.
Yield of the two step procedure: 45%.
¹H-NMR (CDCl₃); δ ppm: 1.52(d, 6H, 2CH₃); 3.56(brs, 3H, CH₃); 4.80(hept 1H, CH); 5.30(brs, 2H, CH₂); 7.24(m, 5H, C₆H₅); 7.72(s, 1H, purine), 8.34(s, 1H, purine)

### Example 8: Preparation of (2R)-2-[(6-benzylsulfanyl-9-isopropyl-purin-2-yl)amino]butan-1-ol (47)

Compound 47 was prepared via a three step procedure starting from 2,6-dichloropurine.

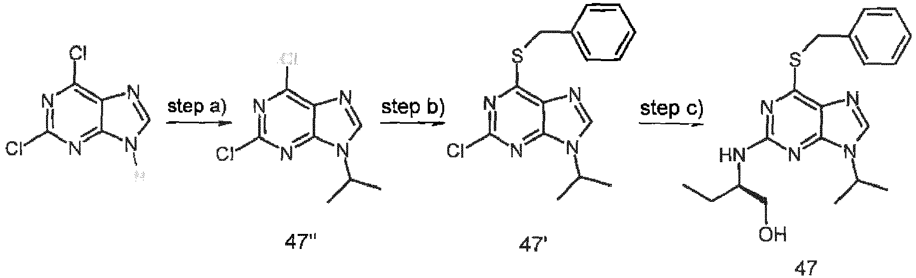

Step a): Alkylation of 2,6-chloropurine (10g, 52.9 mmol) in 200 mL of DMSO in the presence of K₂CO₃ (21.9 g, 158.7 mmol) was achieved upon adding slowly (within 1 hour) 2-bromopropane (32.5 g 264.5 mmol) at 15-18 °C. The temperature should be carefully adjusted between 15 and 18°C to avoid excessive formation of the 7-regiosiomer. After 2 days stirring, 1000 mL of water was added and the mixture was extracted with AcOEt (5x20 mL). The organic layer was washed with water dried and evaporated. The residue was purified by column chromatography using (cyclohexane:AcOEt 7:3) for elution.

Step b): A mixture of compound 47" (10 mmol), benzyl mercaptan (20 mmol) and 2 mL NEt₃ in butanol was heated at 85 °C for 2 h. The reaction was monitored by thin layer chromatography. After cooling at 10 °C, compound 47' which precipitates was filtered, washed with 3-5 mL of cold (0 °C) *iso*-propanol and used for the next step without any further purification.

Step c): Compound 47' (2 mmol) was heated in (R)-2-aminobutanol (16 mmol), without any added solvent, at 120 °C for 4 h. After cooling to 20 °C, the mixture was extracted with CH₂Cl₂, the organic layer was washed twice with water and dried over anhydrous Na₂SO₄. Compound 47 was isolated by column chromatography after concentration in vacuo of the organic layer.
Yield: 55 %
¹H-NMR (CDCl₃); δ(ppm): 0.99 (t, 3H, CH₃); 1.57 (d, 6H, (CH₃)₂CH); 1.65 (m, 2H, CH₂-CH₃); 3.60, 3.75, 3.90 (3m, 3H, CHCH₂OH); 4.48 (s, 2H, CH₂C₆H₅); 4.56 (hept, 1H, (CH₃)₂CH); 4.96 (d, 1H, NH); 7.20 and 7.37 (m, 5H); 7.61 (s, 1H, 8-H).

### Example 9: Measurement of Aβ38, Aβ40 and Aβ42 levels in mouse neuroblastoma cells treated with different compounds according to the invention through an ELISA assay

Mouse neuroblastoma cells (N2a cells) or N2a-APP695 cells (N2a cell line stably transfected with APP695, an amyloid precursor protein) were cultured in an essential medium composed of Dulbecco's minimal essential medium (DMEM) and of OptiMEM^{®} (1:1, v/v) (Gibco^{®}) containing 0.2 mg/mL Geniticin (Geneticin^{®} Selective Antibiotic, Gibco^{®}) and 5 % fetal bovine serum (FBS, Gibco^{®}, EU approved origin) in a humidified, 5% CO₂ incubator at 37°C. Routinely, cells were split without use of Trypsin every 4/5 days. They were first rinsed with PBS (Gibco) and detached from the plate bottom using 4 mL Versene (Gibco) at room temperature for 3-4 min. 8 mL of fresh medium were added to the cell suspension, and the mix was centrifuged for 3 min at 1,000 rpm. The cell pellet was resuspended in fresh medium before seeding (1/10 dilution) in new flasks.

The N2a-APP695 cells were seeded at 10.000 cells/well in a 96 well plate with modified media (0.5% FBS) and incubated overnight. Cells were treated with fresh media comprising different compounds according to the invention (equal quantity of DMSO sold by Sigma^{®}). They were then incubated for 18h in a humidified, 5% CO₂ incubator.

Purines according to the invention were added at various concentrations to N2a-APP695.

The plate was finally centrifuged to remove cell fragments before collecting supernatant samples for amyloids levels determination.

Then, Aβ38, Aβ40 and Aβ42 levels were measured in a sandwich ELISA (Enzyme-Linked ImmunoSorbent Assay) using a combination of monoclonal antibody 6E10 (mAb 6E10) (sold by Covance^{®} under the reference SIG-39320) and *biotinylated polyclonal Aβ38, Aβ40 or Aβ42 antibodies (provided by Dr. P.D. Mehta, IBR, Staten Island, USA).*

A solution of 100 µl mAb 6E10 diluted in carbonate-bicarbonate buffer, pH 9.6, was coated in the wells of microtiter plates (Maxisorp^{™} sold by Nunc^{®}) and incubated at 4°C overnight. The plates were then washed with PBST (PBS tablets sold by Gibco^{®}) containing 0.05% Tween-20 (Sigma^{®}), and blocked for 1 h with 1% BSA (Sigma^{®}) in PBST (PBS tablets sold by Gibco^{®}) containing 0.05 Tween-20 (Sigma^{®}) to avoid nonspecific binding.

Following a washing step, samples were added and incubated for 2 hours at room temperature (RT) and then at 4°C overnight. Plates were then washed before incubation with biotinylated polyclonal antibody diluted in *PBST* (PBS tablets sold by Gibco^{®}) containing 0.05 Tween-20 (Sigma^{®}) + 0.5% BSA at room temperature for 75 minutes.

After a washing step, streptavidin-horseradish peroxidase conjugate (Streptavidin poly-HRP, Thermo Scientific Pierce^{®}), diluted in PBS + 1% BSA, was added and incubation was carried out for 45 min at room temperature. After washing, 100 µl of o-phenylenediamine dihydrochloride (OPD tablets, Invitrogen^{®}) diluted in citrate buffer pH 5.0 were added. The reaction was stopped after 15 min with the addition of 100 µl 1 N sulfuric acid.

A range of known concentrations of Aβ38, Aβ40 and Aβ42, ranging from 0.01 to 10 ng/mL for Aβ38 and Aβ42, and ranging from 0.001 to 10 ng/mL for Aβ40, were use as a reference scale to determine the concentrations of Aβ38, Aβ40 and Aβ42 obtained.

The optical density was finally measured at 490 nm in a microELISA reader (sold by BioTek Instrument, reference E1 800, Gen 5 software).

The EC₅₀ of the molecules tested were thus calculated. Those obtained for the Aβ42 production are indicated in Table 1.

It can be seen from the results presented in Table 1 that a lot of compounds according to the invention possess a very low EC₅₀ and are thus very effective in order to increase the Aβ42 production of the treated cells.

More particularly, dose-response curves of Figure 1 shows that Aftin-5, the purvalanol equivalent of Aftin-4 (N6-methyl-roscovitine), induces an increase of the levels of Aβ42 production (*up to* > *900%*), while the levels of Aβ40 remains essentially stable (maximum 40% increase).

In contrast the levels of Aβ38 were found to decrease (> 70%) in parallel to the increase of Aβ42. Time-course experiments showed that Aβ42 production reached a plateau after about 12 hours after 100 µM Aftin-5 addition (Figure 2). It remained stable even after 48 hours.

### Example 10: Measurement of the toxicity of different compounds according to the invention on mouse neuroblastoma cells following the ELISA assay of example 9

To measure cell viability, the MTS assay (CellTiter 96^{®} AQueous One Solution Cell Proliferation Assay, Promega) was used in the same wells as the capture ELISA assay. 20µl of MTS reagent (3-(4,5-dimethylthiazol-2-yl)-5-(3-carboxymethoxyphenyl)-2-(4-sulfophenyl)-2H-tetrazolium) was added in each well containing 100µl of media and incubation was then proceeded for 3 hours (37°C, 5% CO2 and 95% humidity). Measurements were made on OD 490Δ630 nm (excitation and emission wavelengths) using a microELISA reader (BioTek Instrument, El 800, Gen 5 software).

The toxicity on the N2a-APP695 cells of the tested compounds of example 1 are indicated in Table 1 here after.

Concerning more particularly Aftin-5, it did not show any cytotoxic effects on cells even at the highest concentration tested (150 µM) or at the longest exposure (5 days).

**Table 1. Summary of results EC₅₀ (µM) at inducing Aβ42 production as measured by the ELISA assay in example 9 and toxicity as measured by the MTS assay**

| | | | |
|---|---|---|---|
| In the following Table, "-" means that the tested compound is non toxic with a concentration higher than 100 µM and "+" means toxic. | | | |
| Compounds No. 1 and 2 are different from the compounds according to the invention and are thus used as negative controls. | | | |

| No | Structure | EC₅₀ | Toxicity |
|---|---|---|---|
| 1 (negative control) | 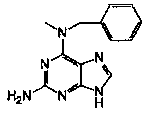 | 100 | - |
| 2 (negative control) | 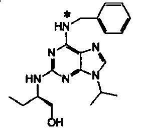 | 100 | - |
| Aftin-1 | 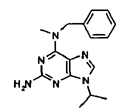 | 20 | + |
| 9 | 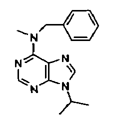 | 12 | - |
| Aftin-2 | 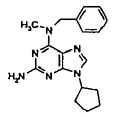 | 11 | - |
| 12 | 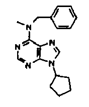 | 12 | + (>31µM) |
| 13 | 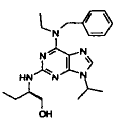 | 3,8 | + (>31µM) |
| 14 | 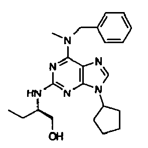 | 2,7 | + (>20µM) |
| 15 | 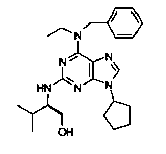 | 0,5 | + |
| 16 | 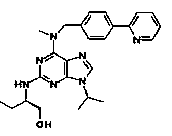 | 3,2 | +(>31µM) |
| 17 | 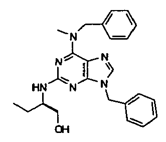 | 0,7 | +(>12µM) |
| 18 | 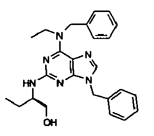 | 1,3 | +(>12µM) |
| 19 | 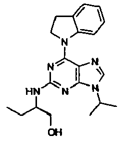 | 8 | +(>31µM) |
| 20 | 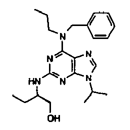 | 1,35 | +(>12µM) |
| 21 | 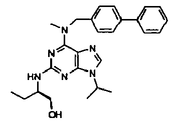 | 0,45 | + |
| 22 | 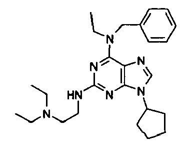 | 0,8 | +(>12µM) |
| 23 | 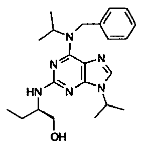 | 2,5 | +(>12µM) |
| 24 | 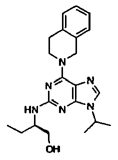 | 3,5 | +(>31µM) |
| Aftin-4 | 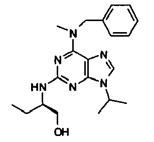 | 10 | +(>31µM) |
| 28 | 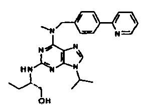 | 2,8 | +(>12µM) |
| 29 | 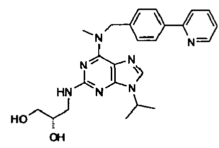 | 10 | - |
| 30 | 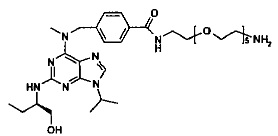 | >100 | - |
| Aftin-3 | 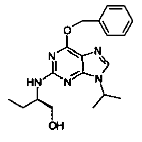 | 13 | - |
| 32 | 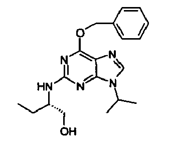 | 21 | - |
| 33 | 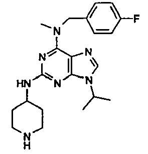 | 10(2 fold) | +(>12µM) |
| 35 | 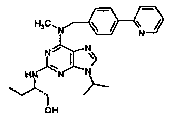 | 1,4 | +(>12µM) |
| 26 | 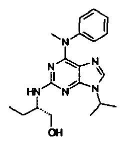 | 17 | - |
| Aftin-5 | 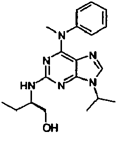 | 10,6 | - |
| 34 | 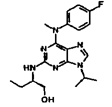 | 30 | - |
| 35 | 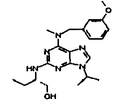 | 90 | +(>12µM) |
| 36 | 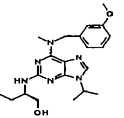 | 105 | +(>100µM) |
| 37 | 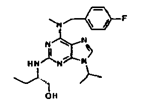 | 52 | +(>12µM) |
| 38 | 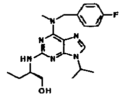 | 55 | +(>12µM) |
| 39 | 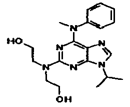 | >150 | - |
| 40 | 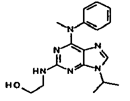 | >150 | - |
| 41 | 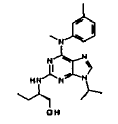 | 15 | - |
| 42 | 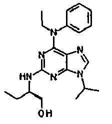 | 25 | +(>100µM) |
| 43 | 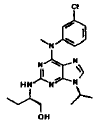 | 6 | +(>12µM) |
| 44 | 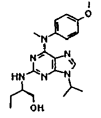 | 4,5 | +(>21µM) |
| 45 | 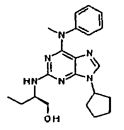 | 25 | +(>21µM) |
| 46 | 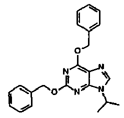 | 3,5 | +(>12µM) |
| 47 | 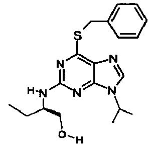 | >10 | + |

### Example 11: Aftins-induced up-regulation of Aβ42 requires active γ-secretases

To investigate the importance of γ-secretases in the effects of Aftins according to the invention, two potent pharmacological inhibitors of γ-secretases, DAPT (*N*-[(3,5-Difluorophenyl)acetyl]-L-alanyl-2-phenyl]glycine-1,1-dimethylethyl ester) and BMS 299897 were used.

Both drugs dose-dependently inhibited the Aftin-4 and Aftin-5 induced production of Aβ₄₂. Both drugs also reduced the level of basal Aβ₄₀ and Aβ₃₈.

IC50 values (Aβ38/Aβ40/Aβ42: 112/61.8/42.5 nM and 88/55.9/28.8 nM, for DAPT and BMS 299897, respectively) are in agreement with reported IC50 values on γ-secretases (25-56 nM and 8-300 nM).

It can thus be observed that up-regulation of Aβ₄₂ and down-regulation of Aβ₃₈ by Aftin-5 are counteracted by γ-secretase inhibitors.

A recently reported γ-secretase modulator (GSM), the "Torrey Pines' compound (Cheng et al in WO2004/110350), was also used.

The chemical structure of the 'Torrey Pines' compound is the following :

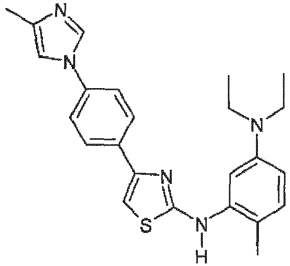

As demonstrated in Figure 3, this compound down-regulates Aβ₄₂ and Aβ₄₀ production, in a dose-dependent manner, while it triggers the parallel up-regulation of Aβ₃₈ level in N2a-APP695 cells.

When increasing concentrations of the 'Torrey Pines' compound were added to N2a-APP695 cells 1 h prior to 100 µM Aftin-5, an inhibition of Aftin-5 effect was observed inducing Aβ₄₂ up-regulation and Aβ₃₈ down-regulation in a dose dependant manner (Figure 4).

This shows that Aftin-5 and the GSM 'Torrey Pines' have opposite effects on the production of Aβ peptides in N2a-APP695 cells. This was confirmed in a parallel experiment in which increasing concentrations of Aftin-5 were added to N2a-APP695 cells simultaneously with 1 µM of the 'Torrey Pines' compound.

Under these conditions, it has been observed that Torrey Pines compound induced Aβ₄₂ down-regulation and Aβ₃₈ up-regulation in a dose-dependent manner.

## Claims

1. Use of a compound of formula (I): 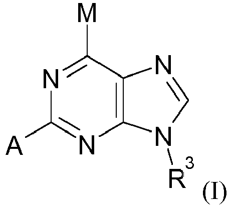 in which
M represents a NR¹R² group, an OR¹ group or a SR¹ group,
A represents a NR⁴R⁵ group, an OR¹⁰ group or a hydrogen atom,
R¹ is an aryl group, a heteroaryl group, a -CH₂-aryl group, a -CH₂-heteroaryl group, said aryl and heteroaryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group, an azido group (-N₃), an aryl group, a heteroaryl group and a (C₁-C₆)alkyl group,
R² is a (C₁-C₆)alkyl group or a (C₁-C₆)cycloalkyl group said alkyl and cycloalkyl being optionally substituted with one or more substituents chosen from a halogen atom , a hydroxyl group and a NH₂ group,
or R¹ forms together with R² and with the nitrogen atom that bears R¹ and R² an heterobicyclic ring,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group, said alkyl being optionally substituted with one or more substituents chosen from a halogen atom, a hydroxyl group and a R⁸ group,
R⁸ represents a hydroxyl group, a NH₂ group, a N(CH₃)₂ group, a 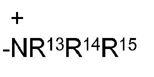 group or a 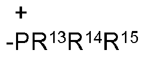 group,
R¹³, R¹⁴ and R¹⁵ represent independently of each other an aryl group or a (C₁-C₆)alkyl group,
x is an integer ranging from 0 to 4,
y is an integer ranging from 2 to 3,
z is an integer ranging from 1 to 10,
p is an integer ranging from 2 to 9,
q is an integer ranging from 2 to 8,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom , a hydroxyl group and a NH₂ group,
R⁴ is a hydrogen atom, a (C₁-C₆)alkyl group, a (C₁-C₆)cycloalkyl group, an aryl group or a heteroaryl group, said alkyl and cycloalkyl being optionally substituted with one or more substituents chosen from a halogen atom, a hydroxyl group and a NH₂ group, said aryl and heteroaryl being optionally substituted with one or more substituents chosen from a halogen atom, a hydroxyl group, a NH₂ group and a NH-R⁹ group,
R⁹ is a heteroaryl group optionally substituted with one or more substituents chosen from a halogen atom, a hydroxyl group, a NH₂ group and a heteroaryl group,
R⁵ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group, an azido group and a NH₂ group, one or more of the carbon atoms of said alkyl or cycloalkyl being optionally replaced by a nitrogen atom,
R¹⁰ is a hydrogen atom, a (C₁-C₆)alkyl group, a (C₁-C₆)cycloalkyl group, an aryl group, a heteroaryl group, a -CH₂-aryl group or -CH₂-heteroaryl group, said aryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group and an azido group (N₃),
in particular Aftin-3, Aftin-4 and Aftin-5, 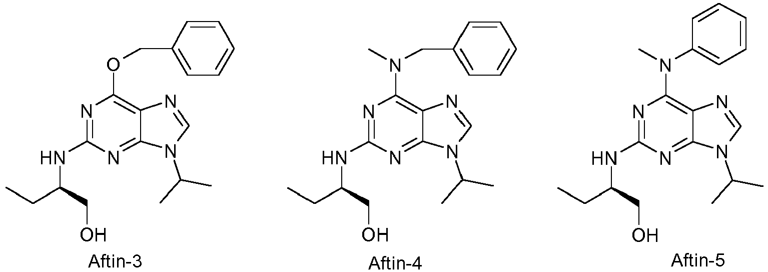 and their addition salts with pharmaceutically acceptable acids,
as an inducer agent that increases the Aβ₄₂ amyloid β peptides production for *in vitro* screening and *in vivo* screening in animal models, said animal being different from a human being, of compounds that modulate the production of Aβ₄₂ amyloid β peptides.

2. Use according to claim 1 of a compound of formula (Ia): 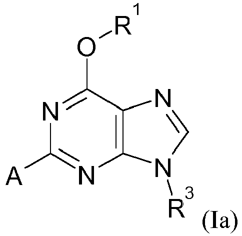 in which
A represents a NR⁴R⁵ group or an OR¹⁰ group,
R¹ is as defined in claim 1, preferably R¹ is a -CH₂-aryl group and more preferably a benzyl group,
R⁵ is as defined in claim 1, preferably R⁵ is a (C₁-C₆)alkyl group or a (C₁-C₆)cycloalkyl group, said alkyl and cycloalkyl being optionally substituted with one hydroxyl group, more preferably R⁵ is a methyl, ethyl, cyclopropyl, cyclobutyl, methylcyclopropyl, methylcyclobutyl or 1-hydroxybutan-2-yl group in particular a 1-hydroxybutan-2-yl group,
R¹⁰ is as defined in claim 1, preferably R¹⁰ is a -CH₂-aryl group, more preferably a benzyl group,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom , a hydroxyl group and a NH₂ group, preferably R³ is a (C₁-C₆)alkyl group or a (C₁-C₆)cycloalkyl group, more preferably a methyl, ethyl, cyclopropyl, cyclobutyl, methylcyclopropyl, methylcyclobutyl or isopropyl group, in particular an isopropyl group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group, preferably R⁴ is a hydrogen atom,
and their addition salts with pharmaceutically acceptable acids.

3. Use according to claim 1 of a compound of formula (Ib): 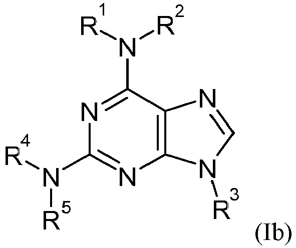 in which
R¹, R² and R⁵ are as defined in claim 1,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom, a hydroxyl group and a NH₂ group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group,
and their addition salts with pharmaceutically acceptable acids.

4. Use according to the preceding claim of a compound in which, the NR¹R² radicals are chosen from the following radicals: 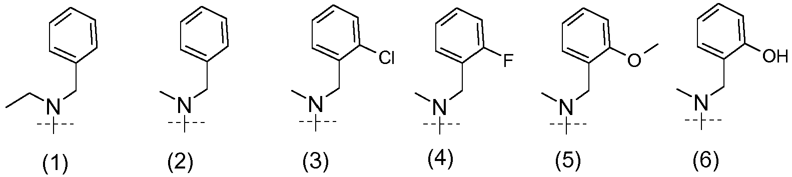 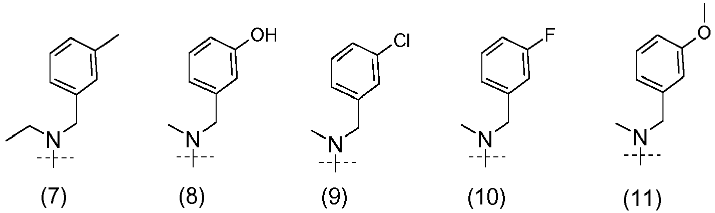 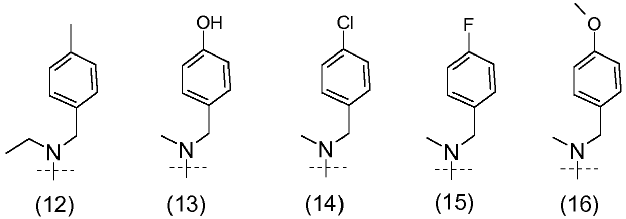 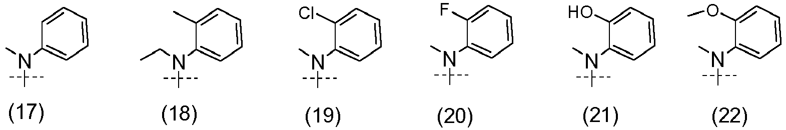 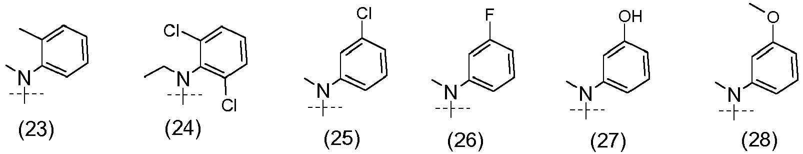 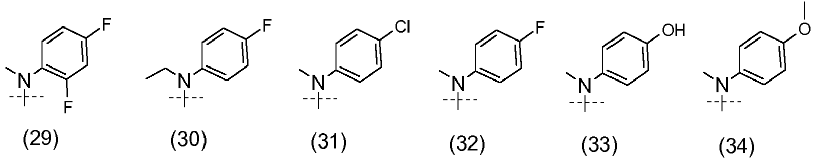 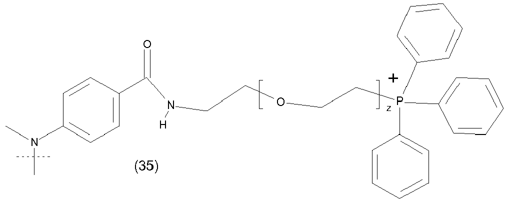 wherein z is ranging from 2 to 9, 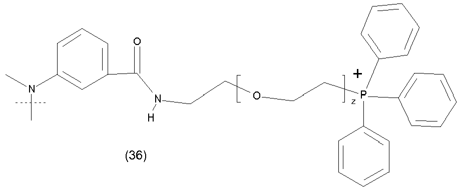 wherein z is ranging from 2 to 9, 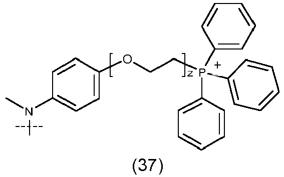 wherein z is ranging from 2 to 9, 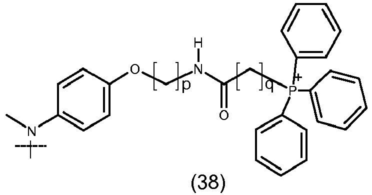 wherein p is ranging from 2 to 9 and q is ranging from 2 to 8, 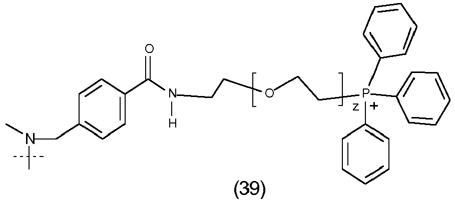 wherein z is ranging from 2 to 9, 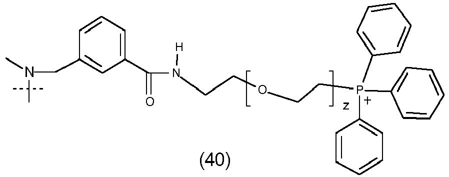 wherein z is ranging from 2 to 9, 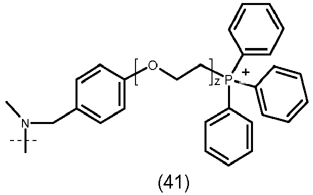 wherein z is ranging from 2 to 9, 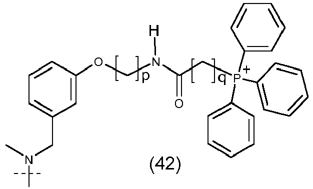 wherein p is ranging from 2 to 9 and q is ranging from 2 to 8,

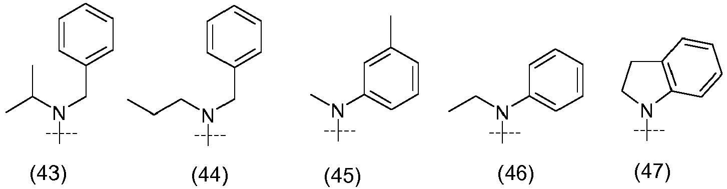

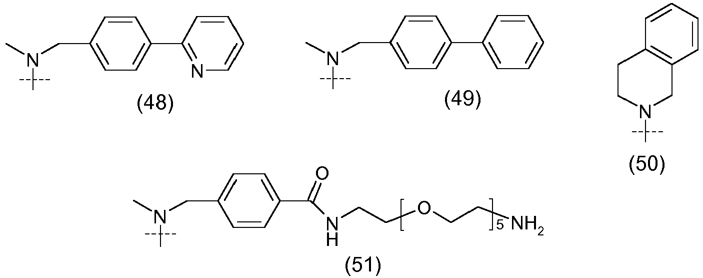

5. Use according to the preceding claim of a compound in which,
the NR¹R² radical is as defined in preceding claim, preferably, NR¹R² radical is chosen from radicals of formulae (1), (2), (11), (15), (17), (25), (32), (34) and (43) to (51) as defined in claim 4,
R³ is a (C₁-C₆)alkyl group, a (C₁-C₆)cycloalkyl group or a -CH₂-aryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R⁴ is a hydrogen atom, a (C₁-C₆)alkyl group or a (C₁-C₆)cycloalkyl group, said alkyl and cycloalkyl being optionally substituted with one or more hydroxyl group, preferably R⁴ is a methyl, ethyl, cyclopropyl, cyclobutyl, methylcyclopropyl, methylcyclobutyl or 2-hydroxyethyl group or a hydrogen atom, in particular R⁴ is a 2-hydroxyethyl group or a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said alkyl and cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl, 1-hydroxy-3-methylbutan-2-yl, 1,2-dihydroxypropan-3-yl, N-ditehylaminoeth-2-yl, piperidin-4-yl or 2-hydroxyethyl group, or a hydrogen atom, in particular R⁵ is a 1-hydroxybutan-2-yl, 1-hydroxy-3-methylbutan-2-yl, 1,2-dihydroxypropan-3-yl, N-diethylaminoeth-2-yl, piperidin-4-yl or 2-hydroxyethyl group, or a hydrogen atom, and their addition salts with pharmaceutically acceptable acids.

6. Use according to claim 4 of a compound of formula (Ic): 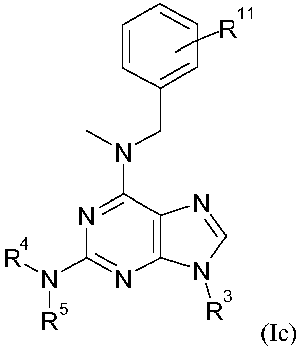 in which,
R³ is a (C₁-C₆)alkyl group, a (C₁-C₆)cycloalkyl group, a -CH₂-aryl group or a -CH₂-heteroaryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R⁴ is a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl, 1,2-dihydroxypropan-3-yl or piperidin-4-yl group, or a hydrogen atom, in particular R⁵ is a 1-hydroxybutan-2-yl, 1,2-dihydroxypropan-3-yl, or piperidin-4-yl group, or a hydrogen atom,
R¹¹ is chosen from a halogen or a hydrogen atom, an OR⁶ group, a CONR⁶R⁷ group, an aryl group and a heteroaryl group, preferably R¹¹ is an OR⁶, CONR⁶R⁷, pyridinyl, phenyl group, or a fluorine or a hydrogen atom,
R⁶ and R⁷ represent independently of each other a hydrogen atom, a (C₁-C₆)alkyl group or a (CH₂)ₓ[O(CH₂)_{y}]_{z}-R⁸ group as defined in claim 1, preferably R⁶ and R⁷ represent a hydrogen atom, or a methyl or a (CH₂)₂[O(CH₂)₂]₅-NH₂ group,
and their addition salts with pharmaceutically acceptable acids.

7. Use according to claim 4 of a compound of formula (Id): 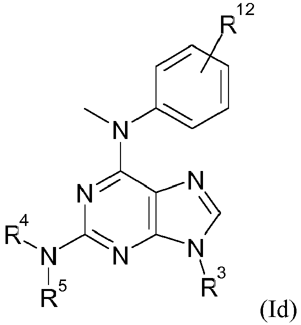 in which,
R³ is a (C₁-C₆)alkyl group, a CH₂-aryl group, a -CH₂-heteroaryl group or a (C₁-C₆)cycloalkyl group, preferably R³ is a methyl, ethyl, cyclopropyl, cyclobutyl, methylcyclopropyl, methylcyclobutyl, isopropyl, cyclopentyl group, in particular R³ is an isopropyl or cyclopentyl group,
R⁴ is a hydrogen atom, a (C₁-C₆)alkyl group or a (C₁-C₆)cycloalkyl group, said alkyl and cycloalkyl being optionally substituted with one or more hydroxyl group, preferably R⁴ is a methyl, ethyl, cyclopropyl, cyclobutyl, methylcyclopropyl, methylcyclobutyl or 2-hydroxyethyl group or a hydrogen atom, in particular R⁴ is a 2-hydroxyethyl group or a hydrogen atom,
R⁵ is a (C₁-C₆)alkyl group or a (C₁-C₆)cycloalkyl group, said alkyl and cycloalkyl being optionally substituted with one or more hydroxyl group, preferably R⁵ is a methyl, ethyl, cyclopropyl, cyclobutyl, methylcyclopropyl, methylcyclobutyl, 1-hydroxybutan-2-yl or 2-hydroxyethyl group, in particular R⁵ is a 1-hydroxybutan-2-yl or 2-hydroxyethyl group,
R¹² is chosen from a halogen or a hydrogen atom, an OR⁶ group and a (C₁-C₆)alkyl group, preferably R¹² is a hydrogen, fluorine or chloride atom, or an OR⁶ or methyl group,
R⁶ is a (C₁-C₆)alkyl group, preferably, R⁶ is a methyl group,
and their addition salts with pharmaceutically acceptable acids.

8. Use according to claim 1 of a compound of formula (Ie): 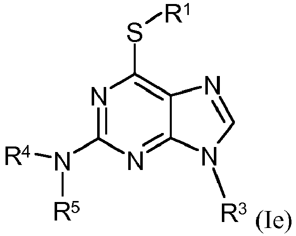 in which
R¹ and R⁵ are as defined in claim 1,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom, a hydroxyl group and a NH₂ group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group,
and their addition salts with pharmaceutically acceptable acids.

9. Use according to any one of the preceding claims **characterized in that** said inducer agent is intended to screen compounds aimed at treating Alzheimer's disease.

10. Compound of formula (Ia): 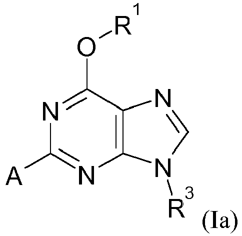 in which
A represents a NR⁴R⁵ group or an OR¹⁰ group,
R¹ is an aryl group, a heteroaryl group, a -CH₂-aryl group, a -CH₂-heteroaryl group, said aryl and heteroaryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group, an azido group (-N₃), an aryl group, a heteroaryl group and a (C₁-C₆)alkyl group, preferably R¹ is a -CH₂-aryl group, more preferably a benzyl group,
R⁵ is a (C₁-C₆)alkyl group or a (C₁-C₆)cycloalkyl group, said alkyl and cycloalkyl being optionally substituted with one or more substituents chosen from a halogen atom, a hydroxyl group, an azido group and a NH₂ group, one or more of the carbon atoms of said alkyl or cycloalkyl being optionally replaced by a nitrogen atom, preferably R⁵ is a (C₁-C₆)alkyl group or a (C₁-C₆)cycloalkyl group, said alkyl and cycloalkyl being optionally substituted with one hydroxyl group, more preferably R⁵ is a methyl, ethyl, cyclopropyl, cyclobutyl, methylcyclopropyl, methylcyclobutyl or 1-hydroxybutan-2-yl group in particular a 1-hydroxybutan-2-yl group,
R¹⁰ is a hydrogen atom, a (C₁-C₆)alkyl group, a (C₁-C₆)cycloalkyl group, an aryl group, a heteroaryl group, a -CH₂-aryl group or -CH₂-heteroaryl group, said aryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group and an azido group (N₃), preferably R¹⁰ is a -CH₂-aryl group, more preferably a benzyl group,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom , a hydroxyl group and a NH₂ group, preferably R³ is a (C₁-C₆)alkyl group or a (C₁-C₆)cycloalkyl group, more preferably a methyl, ethyl, cyclopropyl, cyclobutyl, methylcyclopropyl, methylcyclobutyl or isopropyl group, in particular an isopropyl group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group, preferably R⁴ is a hydrogen atom,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group, said alkyl being optionally substituted with one or more substituents chosen from a halogen atom, a hydroxyl group and a R⁸ group,
R⁸ represents a hydroxyl group, a NH₂ group, a N(CH₃)₂ group, a 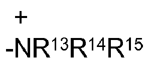 group or a 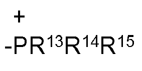 group,
R¹³, R¹⁴ and R¹⁵ represent independently of each other an aryl group or a (C₁-C₆)alkyl group,
x is an integer ranging from 0 to 4,
y is an integer ranging from 2 to 3,
z is an integer ranging from 1 to 10,
p is an integer ranging from 2 to 9,
q is an integer ranging from 2 to 8,
with the proviso that the compound of formula (Ia) is different from Aftin-3, 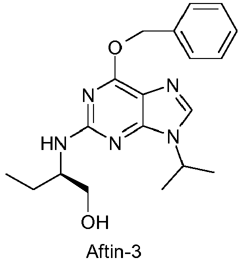 and their addition salts with pharmaceutically acceptable acids.

11. Compound of formula (Ib) 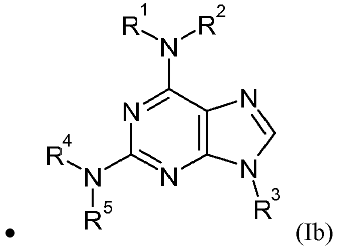 in which,
the NR¹R² radical is chosen from radicals of formulae (1), (2), (11), (15), (17), and (43) to (48), (50) and (51) as defined in claim 4,
R³ is a (C₁-C₆)alkyl group, a (C₁-C₆)cycloalkyl group or a -CH₂-aryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R⁴ is a hydrogen atom, a (C₁-C₆)alkyl group or a (C₁-C₆)cycloalkyl group, said alkyl and cycloalkyl being optionally substituted with one or more hydroxyl group, preferably R⁴ is a methyl, ethyl, cyclopropyl, cyclobutyl, methylcyclopropyl, methylcyclobutyl or 2-hydroxyethyl group or a hydrogen atom, in particular R⁴ is a 2-hydroxyethyl group or a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said alkyl and cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl, 1-hydroxy-3-methylbutan-2-yl, 1,2-dihydroxypropan-3-yl, N-ditehylaminoeth-2-yl, piperidin-4-yl or 2-hydroxyethyl group, or a hydrogen atom, in particular R⁵ is a 1-hydroxybutan-2-yl, 1-hydroxy-3-methylbutan-2-yl, 1,2-dihydroxypropan-3-yl, N-diethylaminoeth-2-yl, piperidin-4-yl or 2-hydroxyethyl group, or a hydrogen atom,
with the proviso that the compound of formula (Ib) is different from Aftin-4 and compound 30, 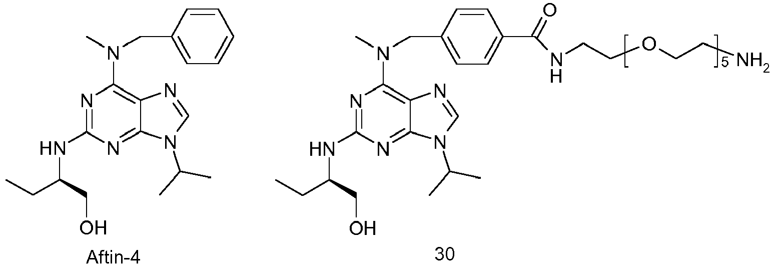 and their addition salts with pharmaceutically acceptable acids.

12. Compound of formula (Ic): 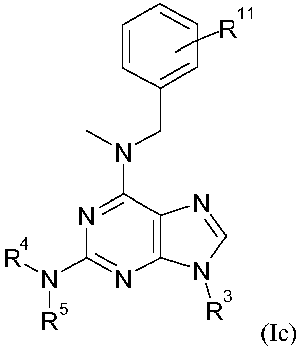 in which,
R³ is a (C₁-C₆)alkyl group, a (C₁-C₆)cycloalkyl group, a -CH₂-aryl group or a -CH₂-heteroaryl group, preferably R³ is a methyl, ethyl, cyclopropyl, cyclobutyl, methylcyclopropyl, methylcyclobutyl, isopropyl, cyclopentyl or benzyl group, in particular R³ is an isopropyl, cyclopentyl or benzyl group,
R⁴ is a hydrogen atom,
R⁵ is a hydrogen atom, a (C₁-C₆)alkyl group or a (C₁-C₆)cycloalkyl group, said alkyl and cycloalkyl being optionally substituted with one or more hydroxyl group, one or more of the carbon atoms of said cycloalkyl being optionally replaced by a nitrogen atom, preferably R⁵ is a methyl, ethyl, cyclopropyl, cyclobutyl, methylcyclopropyl, methylcyclobutyl, 1-hydroxybutan-2-yl, 1,2-dihydroxypropan-3-yl or piperidin-4-yl group, or a hydrogen atom, in particular R⁵ is a 1-hydroxybutan-2-yl, 1,2-dihydroxypropan-3-yl, or piperidin-4-yl group, or a hydrogen atom,
R¹¹ is chosen from a halogen or a hydrogen atom, an OR⁶ group, a CONR⁶R⁷ group, an aryl group and a heteroaryl group, preferably R¹¹ is an OR⁶, CONR⁶R⁷, pyridinyl, phenyl group, or a fluorine or a hydrogen atom,
R⁶ and R⁷ represent independently of each other a hydrogen atom, a (C₁-C₆)alkyl group or a (CH₂)ₓ[O(CH₂)_{y}]_{z}-R⁸ group as defined in claim 1, preferably R⁶ and R⁷ represent a hydrogen atom, or a methyl or a (CH₂)₂[O(CH₂)₂]₅-NH₂ group,
with the proviso that the compound of formula (Ic) is different from Aftin-4 and compound 30, 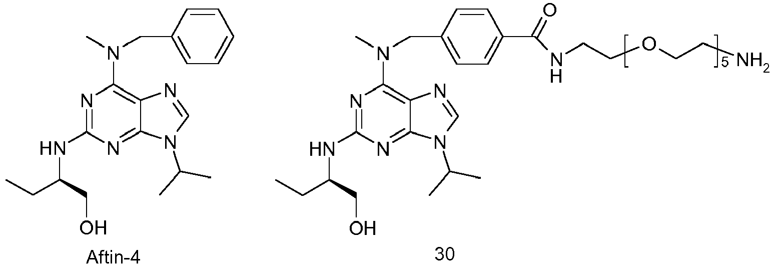 and their addition salts with pharmaceutically acceptable acids.

13. Compound of formula (Ie): 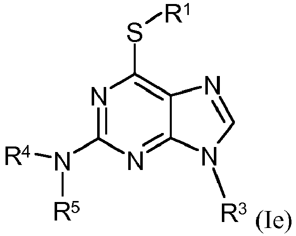 in which
R¹ is an aryl group, a heteroaryl group, a -CH₂-aryl group, a -CH₂-heteroaryl group, said aryl and heteroaryl being optionally substituted with one or more substituents chosen from a halogen atom, a hydroxyl group, an OR⁶ group, a SR⁶ group, a NR⁶R⁷ group, a CN group, a CONR⁶R⁷ group, a SOR⁶ group, a SO₂R⁶ group, an azido group (-N₃), an aryl group, a heteroaryl group and a (C₁-C₆)alkyl group,
R⁵ is a (C₁-C₆)alkyl group or a (C₁-C₆)cycloalkyl group, said alkyl and cycloalkyl being optionally substituted with one or more substituents chosen from a halogen atom, a hydroxyl group, an azido group and a NH₂ group, one or more of the carbon atoms of said alkyl or cycloalkyl being optionally replaced by a nitrogen atom,
R⁶ and R⁷ represent independently of each other a hydrogen atom, a (C₁-C₆)alkyl group, a (CH₂)ₓ[O(CH₂)_{y}]_{z}-R⁸ group or a (CH₂)ₚNH-CO-(CH₂)_{q}-R⁸ group, said alkyl being optionally substituted with one or more substituents chosen from a halogen atom, a hydroxyl group and a R⁸ group,
R⁸ represents a hydroxyl group, a NH₂ group, a N(CH₃)₂ group, a 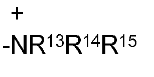 group or a 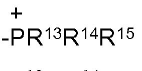 group,
R¹³, R¹⁴ and R¹⁵ represent independently of each other an aryl group or a (C₁-C₆)alkyl group,
R³ is a (C₁-C₆)alkyl group, a (C₁-C₆)cycloalkyl group, an aryl group, a -CH₂-aryl group or a -CH₂-heteroaryl group, said alkyl, cycloalkyl, aryl and heteroaryl group being optionally substituted with one or more substituents chosen from a halogen atom, a hydroxyl group and a NH₂ group,
R⁴ is a hydrogen atom, a (C₁-C₆)alkyl group or a (C₁-C₆)cycloalkyl group, said alkyl and cycloalkyl being optionally substituted with one or more substituents chosen from a halogen atom, a hydroxyl group and a NH₂ group,
with the proviso that the compound of formula (Ie) is different from the following compound, 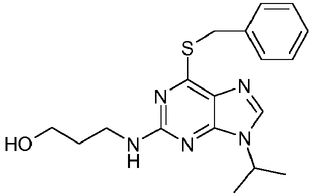 and their addition salts with pharmaceutically acceptable acids.

14. Compound according to anyone of claims 10 to 13, chosen from those disclosed in the following table:
| No | Structure | No | Structure |
|---|---|---|---|
| Aftin-1 | 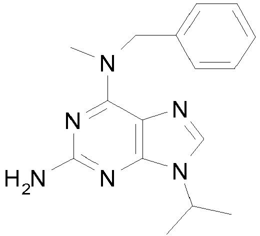 | 32 | 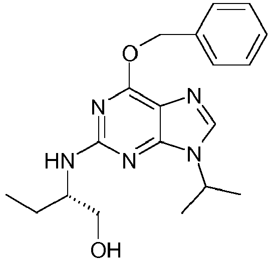 |
| 9 | 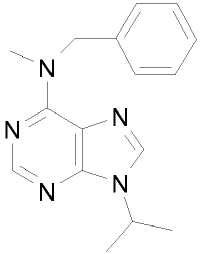 | 33 | 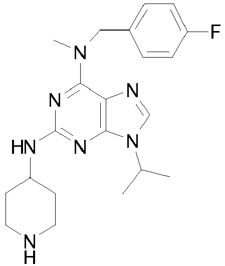 |
| Aftin-2 | 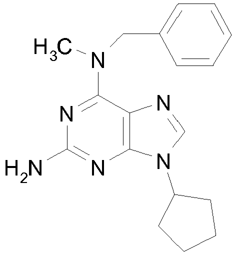 | 35 | 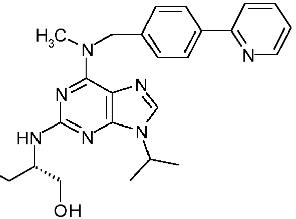 |
| 12 | 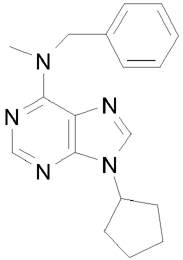 | 26 | 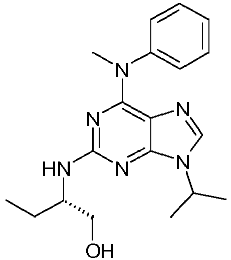 |
| 13 | 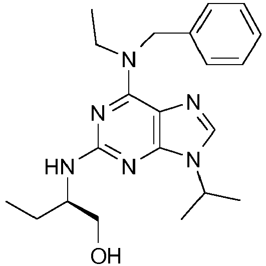 | Aftin-5 | 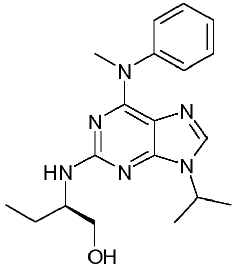 |
| 14 | 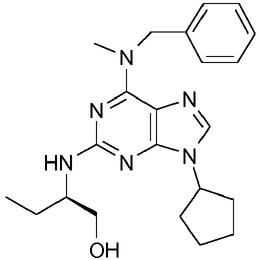 | 34 | 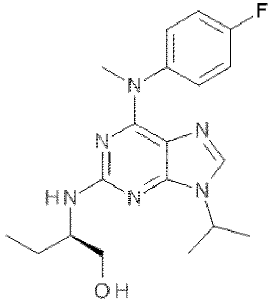 |
| 15 | 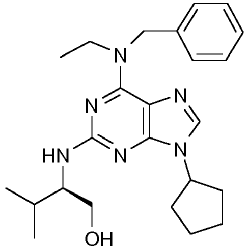 | 35 | 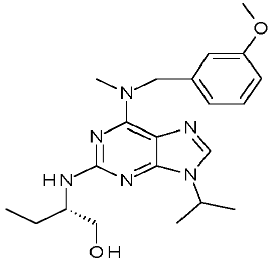 |
| 16 | 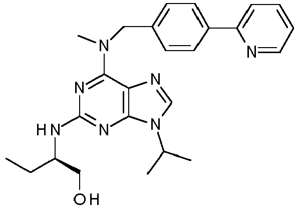 | 36 | 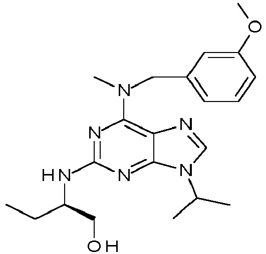 |
| 17 | 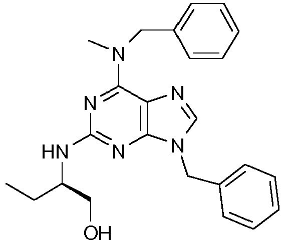 | 37 | 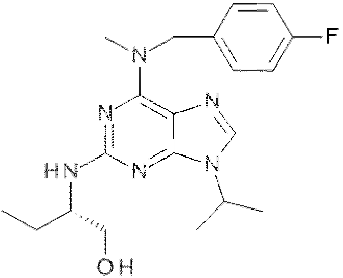 |
| 18 | 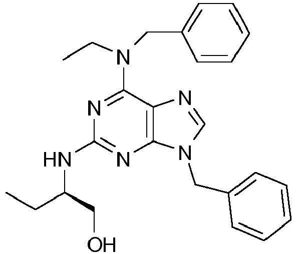 | 38 | 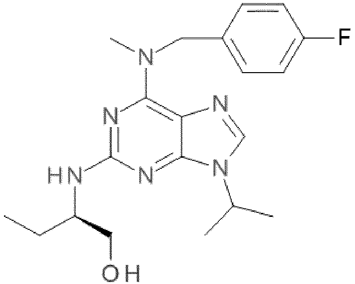 |
| 19 | 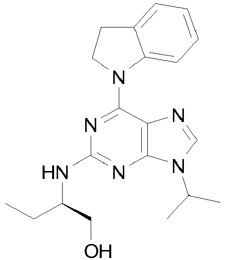 | 39 | 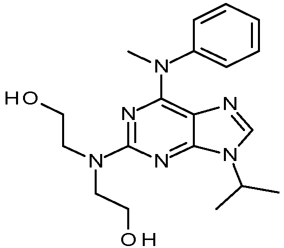 |
| 20 | 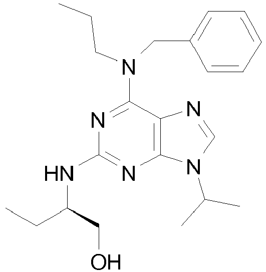 | 40 | 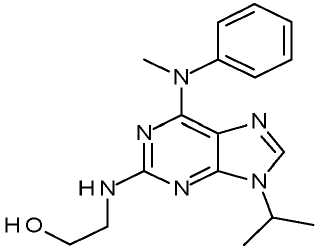 |
| | | 41 | 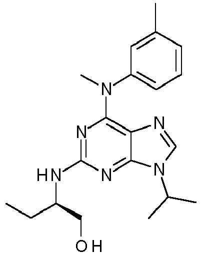 |
| 22 | 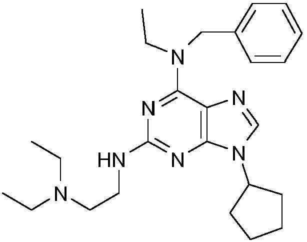 | 42 | 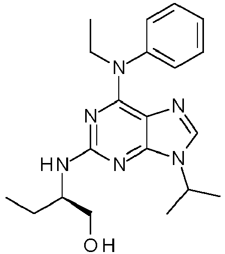 |
| 23 | 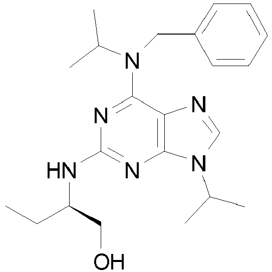 | | |
| 24 | 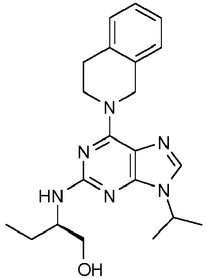 | 44 | 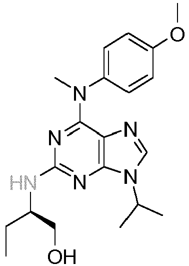 |
| 28 | 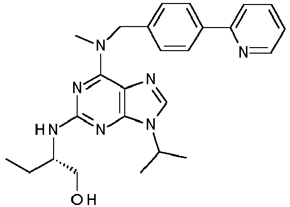 | 45 | 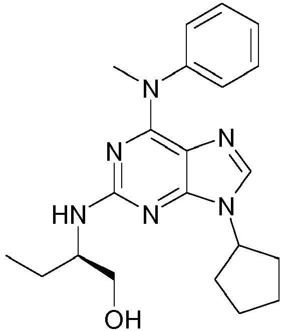 |
| 29 | 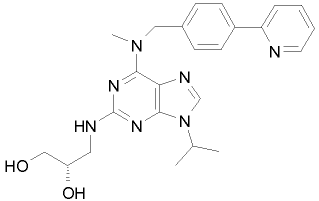 | 46 | 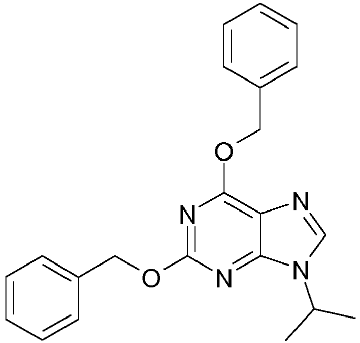 |
| 47 | 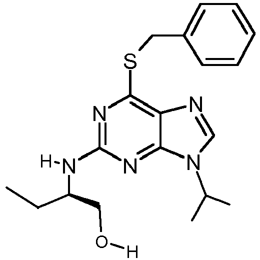 | | |
in particular, is Aftin-5.

15. Method for an *in vitro* screening of candidate compounds that modulate the production of a β-amyloid peptide, comprising the steps of :
a) incubating cells able to produce a β-amyloid peptide with an inducing compound selected from the group consisting of the compounds of formulae (I), (Ia), (Ib), (Ic), (Id) and (Ie) as disclosed in any one of claims 1 to 8, and further with a candidate compound to be tested, whereby a test sample is provided,
b) measuring the level of a β-amyloid peptide in the test sample obtained at the end of step a),
c) comparing the level of the said β-amyloid peptide measured at step b) with a reference value of the said β-amyloid peptide when step a) is performed in the absence of the said inducing compound,
d) selecting the candidate compound when the said candidate compound modulates the level of the said β-amyloid peptide.

16. Use of a compound of formula (I), (Ia), (Ib), (Ic), (Id) and/or (Ie) as disclosed in any one of claims 1 to 8 as reference compounds in *in vitro* screening methods and *in vivo* screening methods in animal models, said animal being different from a human being, designed to detect Alzheimer-inducing compounds among molecules constituting the human chemical exposome and to measure their potency.

## Patentansprüche

1. Verwendung einer Verbindung der Formel (I) 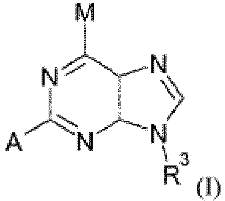
worin M eine NR¹R²-Gruppe, eine OR¹-Gruppe oder eine SR¹-Gruppe darstellt,
A eine NR⁴R⁵-Gruppe, eine OR¹⁰-Gruppe oder ein Wasserstoffatom darstellt,
R¹ eine Arylgruppe, eine Heteroarylgruppe, eine -CH₂-Arylgruppe, eine -CH₂-Heteroarylgruppe ist, wobei das Aryl und Heteroaryl gegebenenfalls mit einem oder mehreren unter einem Halogenatom, einer Hydroxygruppe, einer OR⁶-Gruppe, einer SR⁶-Gruppe, einer NR⁶R⁷-Gruppe, einer CN-Gruppe, einer CONR⁶R⁷-Gruppe, einer SOR⁶-Gruppe, einer SO₂R⁶-Gruppe, einer Azidogruppe (-N₃), einer Arylgruppe, einer Heterarylgruppe und einer (C₁-C₆)Alkylgruppe ausgewählten Substituenten subsituiert ist,
R² eine (C₁-C₆)-Alkylgruppe oder eine (C₁-C₆)-Cycloalkylgruppe ist, wobei Alkyl und Cycloalkyl gegebenenfalls mit einen oder mehreren unter einem Halogenatom, einer Hydroxygruppe und einer NH₂-Gruppe ausgewählten Substituenten subsituiert ist,
oder R¹ zusammen mit R² und mit dem Stickstoffatom, welches R¹ und R² trägt, einen heterobicyclischen Ring bildet,
R⁶ und R⁷ unabhängig voneinander ein Wasserstoffatom, eine (C₁-C₆)-Alkylgruppe, eine (CH₂)ₓ[O(CH₂)_{y}]_{z}-R⁸-Gruppe oder eine (CH₂)ₚNH-CO-(CH₂)_{q}-R⁸-Gruppe darstellen, wobei das Alkyl gegebenenfalls mit einem oder mehreren unter einem Halogenatom, einer Hydroxygruppe und einer R⁸-Gruppe ausgewählten Substituenten substituiert ist,
R⁸ eine Hydroxygruppe, eine NH₂-Gruppe, eine N(CH₃₎₂-Gruppe, eine 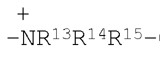 Gruppe oder eine 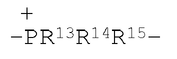 Gruppe darstellt,
R¹³, R¹⁴ und R¹⁵ unabhängig voneinander eine Arylgruppe oder eine (C₁-C₆)-Alkylgruppe darstellen,
x eine ganze Zahl im Bereich von 0 bis 4 ist,
y eine ganze Zahl im Bereich von 2 bis 3 ist,
z eine ganze Zahl im Bereich von 1 bis 10 ist,
p eine ganze Zahl im Bereich von 2 bis 9 ist,
q eine ganze Zahl im Bereich von 2 bis 8 ist,
R³ eine (C₁-C₆)-Alkylgruppe, eine (C₁-C₆)-Cycloalkylgruppe, eine Arylgruppe, eine -CH₂-Arylgruppe oder eine -CH₂-Heteroarylgruppe ist, wobei die Alkyl-, Cycloalkyl-, Aryl- und Heteroarylgruppe gegebenenfalls mit einem oder mehreren unter einem Halogenatom, einer Hydroxygruppe und einer NH₂-Gruppe ausgewählten Substituenten substituiert ist,
R⁴ ein Wasserstoffatom, eine (C₁-C₆)-Alkylgruppe, eine (C₁-C₆)-Cycloalkylgruppe, eine Arylgruppe oder eine Heteroarylgruppe ist, wobei das Alkyl und Cycloalkyl gegebenenfalls mit einem oder mehreren unter einem Halogenatom, einer Hydroxygruppe und einer NH₂-Gruppe ausgewählten Substituenten substituiert ist, wobei das Aryl und das Heteroaryl gegebenenfalls mit einem oder mehreren unter einem Halogenatom, einer Hydroxygruppe, einer NH₂-Gruppe und einer NH-R⁹-Gruppe ausgewählten Substituenten subsituiert ist,
R⁹ eine Heteroarylgruppe ist, die gegebenenfalls mit einem oder mehreren unter einem Halogenatom, einer Hydroxygruppe, einer NH₂-Gruppe und einer Heteroarylgruppe ausgewählten Substituenten substituiert ist,
R⁵ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einem oder mehreren unter einem Halogenatom, einer Hydroxygruppe, einer Azidogruppe und einer NH₂-Gruppe ausgewählten Substituenten subsituiert ist, wobei ein oder mehrere der Kohlenstoffatome des Alkyls oder Cycloalkyls gegebenenfalls durch ein Stickstoffatom ersetzt sind,
R¹⁰ ein Wasserstoffatom, eine (C₁-C₆)-Alkylgruppe, eine (C₁-C₆)-Cycloalkylgruppe, eine Arylgruppe, eine Heteroarylgruppe, eine -CH₂-Arylgruppe oder -CH₂-Heteroarylgruppe ist, wobei das Aryl gegebenenfalls mit einem oder mehreren unter einem Halogenatom, einer Hydroxygruppe, einer OR⁶-Gruppe, einer SR⁶-Gruppe, einer NR⁶R⁷-Gruppe, einer CN-Gruppe, einer CONR⁶R⁷-Gruppe, einer SOR⁶-Gruppe, einer SO₂R⁶-Gruppe und einer Azidogruppe (N₃) ausgewählten Substituenten substituiert ist,
insbesondere Aftin-3, Aftin-4 und Aftin-5 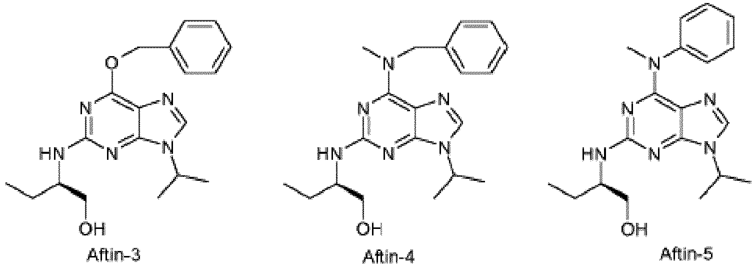 und von deren Additionssalzen mit pharmazeutisch geeigneten Säuren als ein Induktionsmittel, das die Produktion des Aβ₄₂-Amyloid-β-Peptids erhöht, für das in vitro-Screenen und das in vivo-Screenen in Tiermodellen, wobei das Tier sich von einem Menschen unterscheidet, von Verbindungen, welche die Produktion von Aβ₄₂-Amyloid-β-Peptiden modulieren.

2. Verwendung nach Anspruch 1 einer Verbindung der Formel (Ia): 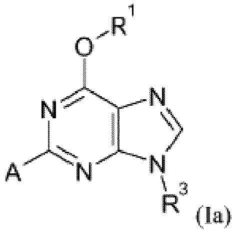
worin A eine NR⁴R⁵-Gruppe oder eine OR¹⁰-Gruppe darstellt,
R¹ wie in Anspruch 1 definiert ist, wobei R¹ vorzugsweise eine -CH₂-Arylgruppe und stärker bevorzugt eine Benzylgruppe ist,
R⁵ wie in Anspruch 1 definiert ist, wobei R⁵ vorzugsweise eine (C₁-C₆)-Alkylgruppe oder eine (C₁-C₆)-Cycloalkylgruppe ist, wobei das Alkyl und Cycloalkyl gegebenenfalls mit einer Hydroxygruppe subsituiert ist, wobei R⁵ stärker bevorzugt eine Methyl-, Ethyl-, Cyclopropyl-, Cyclobutyl-, Methylcyclopropyl-, Metyhlcyclobutyl- oder 1-Hydroxybutan-2-ylgruppe, insbesondere eine 1-Hydroxybutan-2-ylgruppe ist,
R¹⁰ wie in Anspruch 1 definiert ist, wobei R¹⁰ vorzugsweise eine -CH₂-Arylgruppe, stärker bevorzugt eine Benzylgruppe ist,
R³ eine (C₁-C₆)-Alkylgruppe, eine (C₁-C₆)-Cycloalkylgruppe, eine Arylgruppe, eine -CH₂-Arylgruppe oder eine -CH₂-Heteroarylgruppe ist, wobei die Alkyl-, Cycloalkyl-, Aryl- und Heteroarylgruppe gegebenenfalls mit einem oder mehreren unter einem Halogenatom, einer Hydroxygruppe und einer NH₂-Gruppe ausgewählten Substituenten substituiert sind, wobei R³ vorzugsweise eine (C₁-C₆)-Alkylgruppe oder eine (C₁-C₆)-Cycloalkylgruppe, stärker bevorzugt eine Methyl-, Ethyl-, Cyclopropyl-, Cyclobutyl-, Methylcyclopropyl-, Methylcyclobutyl- oder Isopropylgruppe, insbesondere eine Isopropylgruppe ist,
R⁴ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einem oder mehreren unter einem Halogenatom, einer Hydroxygruppe und einer NH₂-Gruppe ausgewählten Substituenten substituiert sind, wobei R⁴ vorzugsweise ein Wasserstoffatom ist,
und von deren Additionssalzen mit pharmazeutisch geeigneten Säuren.

3. Verwendung nach Anspruch 1 einer Verbindung der Formel (Ib): 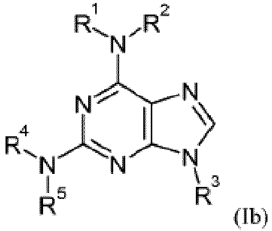 worin
R¹, R² und R⁵ wie in Anspruch 1 definiert sind,
R³ eine (C₁-C₆)-Alkylgruppe, eine (C₁-C₆)-Cycloalkylgruppe, eine Arylgruppe, eine -CH₂-Arylgruppe oder eine -CH₂-Heteroarylgruppe ist, wobei die Alkyl-, Cycloalkyl-, Aryl- und Heteroarylgruppe gegebenenfalls mit einem oder mehreren unter einem Halogenatom, einer Hydroxygruppe und einer NH₂-Gruppe ausgewählten Substituenten substituiert sind,
R⁴ ein Wasserstoffatom, eine (C₁-C₆)-Alkylgruppe oder eine (C₁-C₆)-Cycloalkylgruppe ist,
wobei das Alkyl und Cycloalkyl gegebenenfalls mit einem oder mehreren unter einem Halogenatom, einer Hydroxygruppe und einer NH₂-Gruppe ausgewählten Substituenten substituiert sind,
und von deren Additionssalzen mit pharmazeutisch geeigneten Säuren.

4. Verwendung nach dem vorstehenden Anspruch einer Verbindung, worin die NR¹R²-Radikale unter den folgenden Radikalen ausgewählt sind: 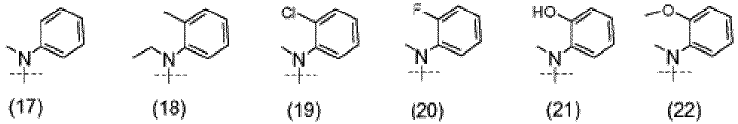 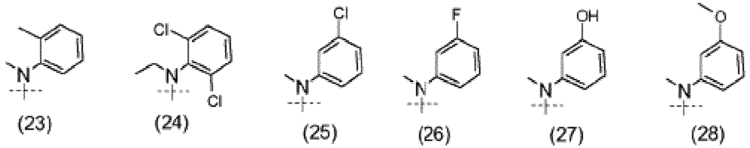 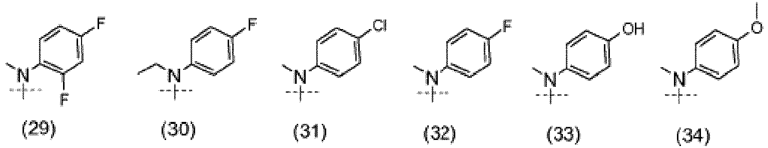 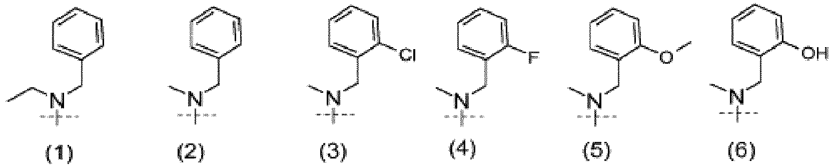 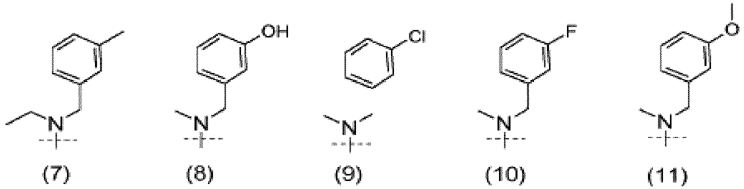 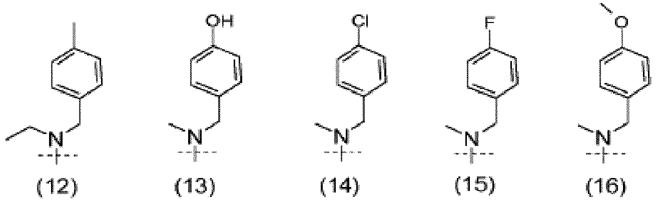 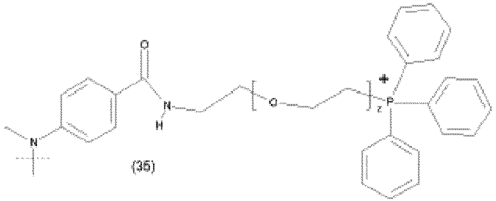
worin z im Bereich von 2 bis 9 ist, 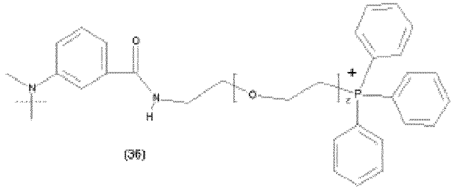
worin z im Bereich von 2 bis 9 ist, 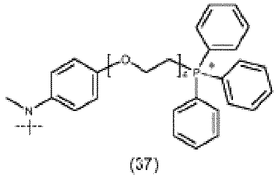
worin z im Bereich von 2 bis 9 ist, 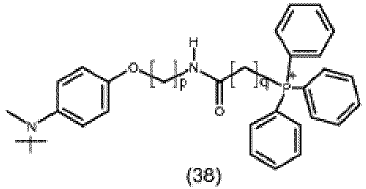
wobei p im Bereich von 2 bis 9 ist und q im Bereich von 2 bis 8 ist, 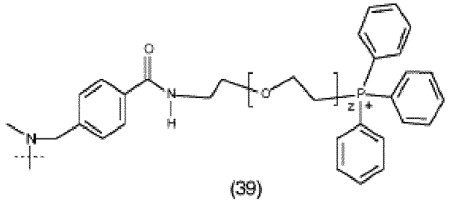
worin z im Bereich von 2 bis 9 ist, 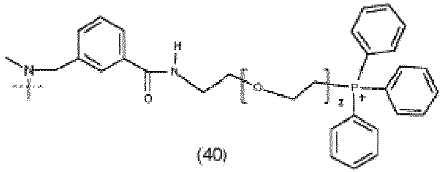
worin z im Bereich von 2 bis 9 ist, 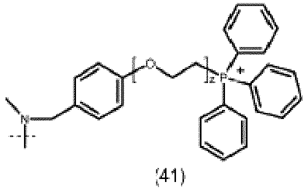
worin z im Bereich von 2 bis 9 ist, 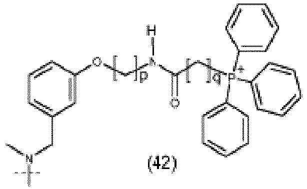
wobei p im Bereich von 2 bis 9 ist und q im Bereich von 2 bis 8 ist,

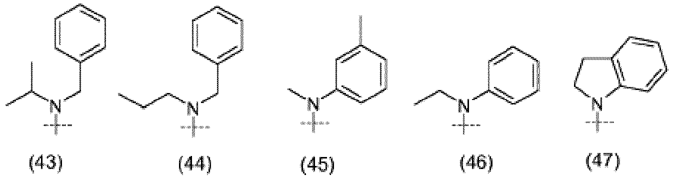

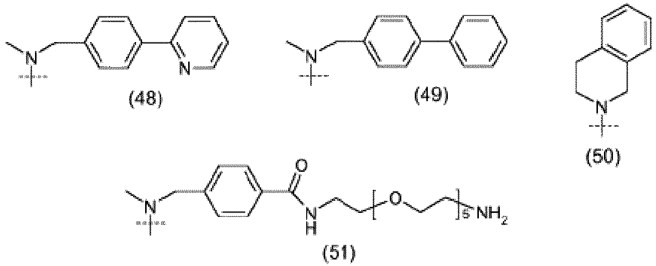

5. Verwendung nach dem vorstehenden Anspruch einer Verbindung, worin das NR¹R²-Radikal wie in dem vorstehenden Anspruch definiert ist, wobei das NR¹R²-Radikal vorzugsweise unter den Radikalen der in Anspruch 4 definierten Formeln (1), (2), (11), (15), (17), (25), (32), (34) und (43) bis (51) ausgewählt ist,
R³ eine (C₁-C₆)-Alkylgruppe, eine (C₁-C₆)-Cycloalkylgruppe oder eine CH₂-Arylgruppe, vorzugsweise eine Methyl-, Etyhl-, Cyclopropyl-, Cyclobutyl-, Methylcyclopropyl-, Methylcyclobutyl-, Isopropyl-, Cyclopentyl- oder Benzylgruppe, insbesondere eine Isopropyl-, Cyclopentyl- oder Benzylgruppe ist,
R⁴ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einer oder mehreren Hydroxygruppen substituiert ist, wobei R4 vorzugsweise eine Methyl-, Ethyl-, Cyclopropyl-, Cyclobutyl-, Methylcyclopropyl-, Methylcyclobutyl- oder 2-Hydroxyethylgruppe oder ein Wasserstoffatom, insbesondere eine 2-Hydroxyethylgruppe oder ein Wasserstoffatom ist,
R⁵ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einer oder mehreren Hydroxygruppen substituiert ist, wobei ein oder mehrere der Kohlenstoffatome des Alkyls und des Cycloalkyls gegebenenfalls durch ein Stickstoffatom ersetzt sind, wobei R5 vorzugsweise Methyl-, Ethyl-, Cyclopropyl-, Cyclobutyl-, Methylcyclopropyl-, Methylcyclobutyl-, 1-Hydroxybutan-2-yl-, 1-Hydroxy-3-methylbutan-2-yl-, 1,2-Dihydroxypropan-3-yl-, N-Ditehylaminoeth-2-yl-, Piperidin-4-yl- oder 2-Hydroxyethylgruppe oder ein Wasserstoffatom, vorzugsweise eine 1-Hydroxybutan-2-yl-, 1-Hydroxy-3-methylbutan-2-yl-, 1,2-Dihydroxypropan-3-yl-, N-Dethalaminoeth-2-yl-, Piperidian-4-yl- oder 2-Hydroxyethylgruppe oder ein Wasserstoffatom ist,
und von deren Additionssalzen mit pharmazeutisch geeigneten Säuren.

6. Verwendung nach Anspruch 4 einer Verbindung der Formel (Ic): 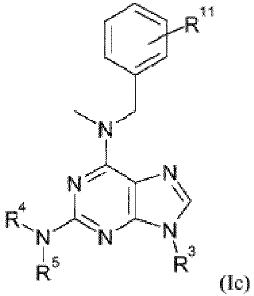 worin
R³ eine (C₁-C₆)-Alkylgruppe, eine (C₁-C₆)-Cycloalkylgruppe, ein -CH2-Arylgruppe oder eine -CH2-Heteroarylgruppe ist, wobei R³ vorzugsweise eine Methyl-, Ethyl-, Cyclopropyl-, Cyclobutyl-, Methylcyclopropyl-, Methylcyclobutyl-, Isopropyl-, Cyclopentyl- oder eine Benzylgruppe, vorzugsweise eine Isopropyl-, Cyclopentyl- oder Benzylgruppe ist,
R⁴ ein Wasserstoffatom ist,
R⁵ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einer oder mehreren Hydroxygruppen substituiert ist, wobei ein oder mehrere der Kohlenstoffatome des Cycloalkyls gegebenenfalls durch ein Stickstoffatom ersetzt sind, wobei R⁵ vorzugsweise eine Methyl-, Ethyl-, Cyclopropyl-, Cyclobutyl-, Methylcyclopropyl-, Methylcyclobutyl-, 1-Hyroxybutan-2-yl-, 1,2-Dihydroxypropan-3-yl- oder Piperidin-4-ylgruppe oder ein Wasserstoffatom, insbesondere eine 1-Hydroxybutan-2-yl-, 1,2-Dihydroxypropan-3-yl- oder Piperidin-4-ylgruppe oder ein Wasserstoffatom ist,
R¹¹ unter einem Halogen oder einem Wasserstoffatom, einer OR⁶-Gruppe, einer CONR⁶R⁷-Gruppe, einer Arylgruppe und einer Heteroarylgruppe ausgewählt ist, wobei R¹¹ vorzugsweise eine OR⁶-, CONR⁶R⁷-, Piperidinyl-, Phenylgruppe oder ein Fluor- oder Wasserstoffatom ist,
R⁶ und R⁷ unabhängig voneinander ein Wasserstoffatom, eine (C₁-C₆)-Alkylgruppe oder eine (CH₂)ₓ[O(CH₂)_{y}]_{z}-R⁸-Gruppe wie in Anspruch 1 definiert sind, wobei R⁶ und R⁷ vorzugsweise ein Wasserstoffatom oder eine Methyl- oder eine (CH₂)₂[O(CH₂)₂]₅-Gruppe sind,
und von deren Additionssalzen mit pharmazeutisch geeigneten Säuren.

7. Verwendung nach Anspruch 4 einer Verbindung der Formel (Id): 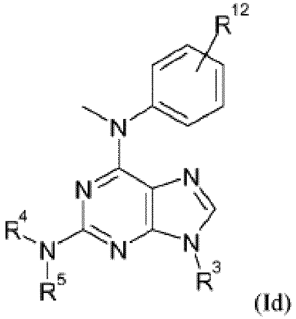 worin
R³ eine (C₁-C₆)-Alkylgruppe, eine CH₂-Arylgruppe, eine -CH₂-Heteroarylgruppe oder eine (C₁-C₆)-Cycloalkylgruppe ist, wobei R³ vorzugsweise eine Methyl-, Ethyl-, Cyclopropyl-, Cyclobutyl-, Methylcyclopropyl-, Methylcyclobutyl-, Isopropyl- oder Cyclopentylgruppe, insbesondere eine Isopropyl- oder Cyclopentylgruppe ist,
R⁴ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einer oder mehreren Hydroxygruppen substituiert sind, wobei R⁴ vorzugsweise eine Methyl-, Ethyl-, Cyclopropyl-, Cyclobutyl-, Methylcyclopropyl-, Methylcyclobutyl- oder 2-Hydroxyethylgruppe oder ein Wasserstoffatom, insbesondere eine 2-Hydroxyethylgruppe oder ein Wasserstoffatom ist,
R⁵ eine (C₁-C₆)-Alyklgruppe oder eine (C₁-C₆)-Cycloalkylgruppe ist, wobei das Alkyl und Cycloalkyl gegebenenfalls mit einer oder mehreren Hydroxygruppen substituiert ist, wobei R⁵ vorzugsweise eine Methyl-, Ethyl-, Cyclopropyl-, Cyclobutyl-, Methylcyclopropyl-, Methylcyclobutyl-, 1-Hydroxybutan-2-yl- oder 2-Hydroxyethylgruppe, vorzugsweise eine 1-Hydroxybutan-2-yl- oder 2-Hydroxyethylgruppe ist,
R¹² unter einem Halogen oder einem Wasserstoffatom, einer OR⁶-Gruppe und einer (C₁-C₆)-Alkylgruppe ausgewählt ist, wobei R¹² vorzugsweise ein Wasserstoff-, Fluor- oder Chloratom oder eine OR⁶- oder Methylgruppe ist,
R⁶ eine (C₁-C₆)-Alkylgruppe, vorzugsweise eine Methylgruppe ist,
und von deren Additionssalzen mit pharmazeutisch geeigneten Säuren.

8. Verwendung nach Anspruch 1 einer Verbindung der Formel (Ie): 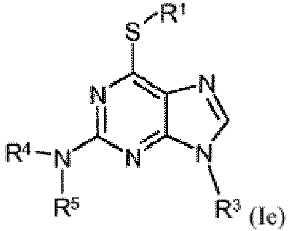 worin
R¹ und R⁵ wie in Anspruch 1 definiert sind, R³ eine (C₁-C₆)-Alkylgruppe, eine (C₁-C₆)-Cycloalkylgruppe, eine Arylgruppe, eine -CH₂-Arylgruppe oder eine -CH₂-Heteroarylgruppe ist, wobei die Alkyl-, Cycloalkyl-, Aryl- und Heteroarylgruppe gegebenenfalls mit einem oder mehreren unter einem Halogenatom, einer Hydroxygruppe und einer NH₂-Gruppe ausgewählten Substituenten substituiert sind,
R⁴ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einem oder mehreren unter einem Halogenatom, einer Hydroxygruppe und einer NH₂-Gruppe ausgewählten Substituenten substituiert ist,
und von deren Additionssalzen mit pharmazeutisch geeigneten Säuren.

9. Verwendung nach einem der vorstehenden Ansprüche, **dadurch gekennzeichnet, dass** das Induktionsmittel zum Screenen von auf die Behandlung der Alzheimerkrankheit abzielende Verbindungen vorgesehen ist.

10. Verbindung der Formel (Ia): 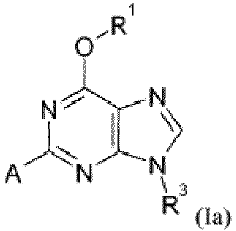 worin
A eine NR⁴R⁵-Gruppe oder eine OR¹⁰-Gruppe darstellt,
R¹ eine Arylgruppe, eine Heteroarylgruppe, eine -CH₂-Arylgruppe, eine -CH₂-Heteroarylgruppe ist, wobei das Aryl und das Heteroaryl gegebenenfalls mit einem oder mehreren unter einem Halogenatom, einer Hydroxygruppe, einer OR⁶-Gruppe, einer SR⁶-Gruppe, einer NR⁶R⁷-Gruppe, einer CN-Gruppe, einer CONR⁶R⁷-Gruppe einer SOR⁶-Gruppe, einer SO₂R⁶-Gruppe, einer Azidogruppe(-N₃), einer Arylgruppe, einer Heteraoarylgruppe und einer (C₁-C₆)-Alkylgruppe ausgewählten Substituenten subsituiert sind, wobei R¹ vorzugsweise eine - CH₂-Arylgruppe, stärker bevorzugt eine Benzylgruppe ist,
R⁵ eine (C₁-C₆)-Alkylgruppe oder eine (C₁-C₆)-Cycloalkylgruppe ist, wobei das Alkyl und Cycloalkyl gegebenenfalls mit einem oder mehreren unter einem Halogenatom, einer Hydroxygruppe, einer Azidogruppe und einer NH₂-Gruppe ausgewählten Substituenten substituiert sind, wobei ein oder mehrere der Kohlenstoffatome des Alkyls oder Cycloalkyls gegebenenfalls durch ein Stickstoffatom ersetzt sind, wobei R⁵ vorzugsweise eine (C₁-C₆)-Alkylgruppe oder eine (C₁-C₆)-Cycloalkylgruppe ist, wobei das Alkyl und Cycloalkyl gegebenenfalls mit einer Hydroxygruppe subsituiert ist, wobei R⁵ stärker bevorzugt eine Methyl-, Ethyl-, Cyclopropyl-, Cyclobutyl-, Methylcyclopropyl-, Methylcyclobutyl- oder eine 1-Hydroxybutan-2-ylgruppe, insbesondere eine 1-Hydroxybutan-2-ylgruppe ist,
R¹⁰ ein Wasserstoffatom, eine (C₁-C₆)-Alkylgruppe, eine (C₁-C₆)-Cycloalkylgruppe, eine Arylgruppe, eine Heteroarylgruppe, eine -CH₂-Arylgruppe oder eine -CH₂-Heteroarylgruppe ist, wobei das Aryl gegebenenfalls mit einem oder mehreren unter einem Halogenatom, einer Hydroxygruppe, einer OR⁶-Gruppe, einer SR⁶-Gruppe, einer NR⁶R⁷-Gruppe, einer CN-Gruppe, einer CONR⁶R⁷-Gruppe einer SOR⁶-Gruppe, einer SO₂R⁶-Gruppe und einer Azidogruppe(N₃) ausgewählten Substituenten substituiert sind, wobei R¹⁰ vorzugsweise eine -CH₂-Arylgruppe, stärker bevorzugt eine Benzylgruppe ist,
R³ eine (C₁-C₆)-Alkylgruppe, eine (C₁-C₆)-Cycloalkylgruppe, eine Arylgruppe, eine CH₂-Arylgruppe oder eine -CH₂-Heteroarylgruppe ist, wobei die Alkyl-, Cycloalkyl-, Aryl- und Heteroarylgruppe gegebenenfalls mit einem oder mehreren unter einem Halogenatom, einer Hydroxygruppe, und einer NH₂-Gruppe ausgewählten Substituenten substituiert ist, wobei R³ vorzugsweise eine (C₁-C₆)-Alkylgruppe oder eine (C₁-C₆)-Cycloalkylgruppe, stärker bevorzugt eine Methyl-, Ethyl-, Cyclopropyl-, Cyclobutyl-, Methylcyclopropyl-, Methylcyclobutyl- oder Isopropylgruppe, insbesondere eine Isopropylgruppe ist,
R⁴ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einem oder mehreren unter einem Halogenatom, einer Hydroxygruppe und einer NH₂-Gruppe ausgewählten Substituenten substituiert sind, wobei R⁴ vorzugsweise ein Wasserstoffatom ist,
R⁶ und R⁷ unabhängig voneinander ein Wasserstoffatom, eine (C₁-C₆)-Alkylgruppe, eine (CH₂)ₓ[O(CH₂)_{y}]_{z}-R⁸-Gruppe oder eine (CH₂)ₚNH-CO-(CH₂)_{q}-R⁸-Gruppe sind, wobei das Alkyl gegebenenfalls mit einem oder mehreren unter einem Halogenatom, einer Hydroxygruppe und einer R⁸-Gruppe ausgewählten Substituenten substituiert ist,
R⁸ eine Hydroxygruppe, eine NH₂-Gruppe, eine + + -NR¹³R¹⁴R¹⁵-Gruppe oder eine -PR¹³R¹⁴R¹⁵-Gruppe ist,
R¹³, R¹⁴ und R¹⁵ unabhängig voneinander eine Arylgruppe oder eine (C₁-C₆)-Alkylgruppe darstellen,
x eine ganze Zahl im Bereich von 0 bis 4 ist,
y eine ganze Zahl im Bereich von 2 bis 3 ist,
z eine ganze Zahl im Bereich von 1 bis 10 ist,
p eine ganze Zahl von 2 bis 9 ist,
q eine ganze Zahl von 2 bis 8 ist,
mit der Maßgabe, dass die Verbindung der Formel (Ia) sich von Aftin-3 unterscheidet, 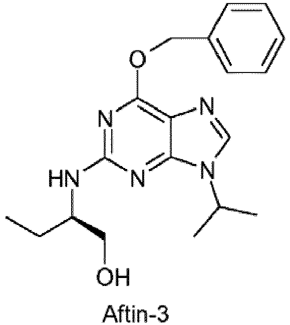 und deren Additionssalze mit pharmazeutisch geeigneten Säuren.

11. Verbindung der Formel (Ib) 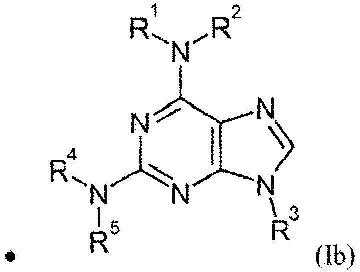 worin
das NR¹R²-Radikal unter Radikalen in Anspruch 4 definierten Formeln (1), (2), (11), (15), (17) und (43) bis (48), (50) und (51) ausgewählt ist,
R³ eine (C₁-C₆)-Alkylgruppe, eine (C₁-C₆)-Cycloalkylgruppe oder eine -CH₂-Arylgruppe ist, wobei R³ vorzugsweise eine Methyl-, Ethyl-, Cyclopropyl-, Cyclobutyl-, Methylcyclopropyl-, Methylcyclobutyl-, Isopropyl-, Cyclopentyl- oder Benzylgruppe, insbesondere eine Isopropyl-, Cyclopentyl- oder Benzylgruppe ist,
R⁴ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einer oder mehreren Hydroxygruppen substituiert ist, wobei R⁴ vorzugsweise eine Methyl-, Ethyl-, Cyclopropyl-, Cyclobutyl-, Methylcyclopropyl-, Methylcyclobutyl- oder eine 2-Hydroxyethylgruppe oder ein Wasserstoffatom, insbesondere eine 2-Hydroxyethylgruppe oder ein Wasserstoffatom ist,
R⁵ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einer oder mehreren Hydroxygruppen substituiert sind, ein oder mehrere der Kohlenstoffatome des Alkyls und Cycloalkyls gegebenenfalls durch ein Stickstoffatom ersetzt sind, wobei R⁵ vorzugsweise Methyl-, Ethyl-, Cyclopropyl-, Cyclobutyl-, Methylcyclopropyl-, Methylcyclobutyl-, 1-Hydroxybutan-2-yl-, 1-Hydroxy-3-methylbutan-2-yl-, 1,2-Dihydroxypropan-3-yl-, N-Diethyl-aminoeth-2-yl-, Piperidin-4-yl- oder eine 2-Hydroxyethylgruppe oder ein Wasserstoffatom, insbesondere eine 1-Hydroxybutan-2-yl-, 1-Hydroxy-3-methylbutan-2-yl-, 1,2-Di-hydroxypropan-3-yl-, N-Diethylaminoeth-2-yl-, Piperidin-4-yl- oder eine 2-Hydroxyethylgruppe oder ein Wasserstoffatom ist,
mit der Maßgabe, dass die Verbindung der Formel (Ib) sich von Aftin-4 und der Verbindung 30 unterscheidet 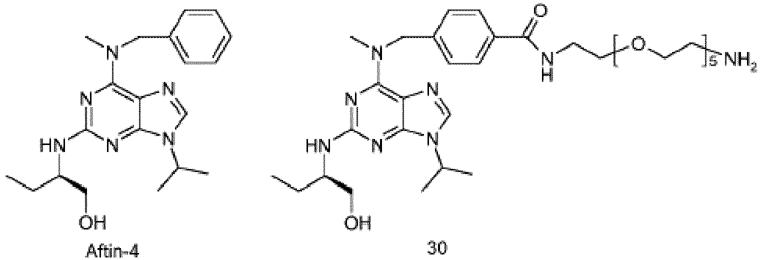 und deren Additionssalze und pharmazeutisch geeigneten Säuren.

12. Verbindung der Formel (Ic) 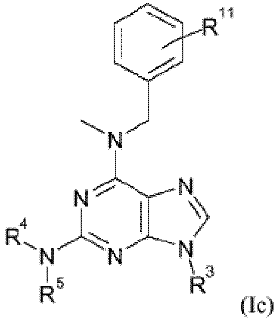 worin
R³ eine (C₁-C₆)-Alkylgruppe, eine (C₁-C₆)-Cycloalkylgruppe, eine -CH₂-Arylgruppe oder eine CH₂-Heteroarylgruppe ist, wobei R³ vorzugsweise eine Methyl-, Ethyl-, Cyclopropyl-, Cyclobutyl-, Methylcyclopropyl-, Methylcyclobutyl-, Isopropyl-, Cyclopentyl- oder Benzylgruppe, insbesondere eine Isopropylgruppe, Cyclopentyl- oder Benzylgruppe ist,
R⁴ ein Wasserstoffatom ist,
R⁵ ein Wasserstoffatom, eine (C₁-C₆)-Alkylgruppe oder eine (C₁-C₆)-Cycloalkylgruppe ist, wobei das Alkyl und Cycloalkyl gegebenenfalls mit einer oder mehreren Hydroxygruppen subsituiert ist, wobei ein oder mehrere Kohlenstoffatome des Cycloalkyls gegebenenfalls durch ein Stickstoffatom ersetzt sind, wobei R⁵ vorzugsweise eine Metyhl-, Ethyl-, Cyclopropyl-, Cyclobutyl-, Methylcyclopropyl-, Methylcyclobutyl-, 1-Hydroxybutan-2-yl-, 1,2-Dihydroxypropan-3-yl- oder eine Piperidin-4-ylgruppe oder ein Wasserstoffatom, insbesondere eine 1-Hydroxybutan-2-yl-, 1,2-Dihydroxypropan-3-yl- oder eine Piperidin-4-ylgruppe oder ein Wasserstoffatom ist,
R¹¹ unter einem Halogen- oder einem Wasserstoffatom, einer OR⁶-Gruppe, einer CONR⁶R⁷-Gruppe, einer Arylgruppe und einer Heteroarylgruppe ausgewählt ist, wobei R¹¹ vorzugsweise eine OR⁶-, CONR⁶R⁷-, Pyridinyl-, Phenylgruppe oder ein Fluor- oder Wasserstoffatom ist,
R⁶ und R⁷ unabhängig voneinander ein Wasserstoffatom, eine (C₁-C₆)-Alkylgruppe oder eine (CH₂)ₓ[O(CH₂)_{y}]_{z}-R⁸-Gruppe wie in Anspruch 1 definiert sind, wobei R⁶ und R⁷ vorzugsweise ein Wasserstoffatom oder eine Methyl- oder eine (CH₂)₂[O(CH₂)₂]₅-NH₂-Gruppe darstellen,
mit der Maßgabe, dass die Verbindung Formel (Ic) sich von Aftin-4 und Verbindung 30 unterscheidet, 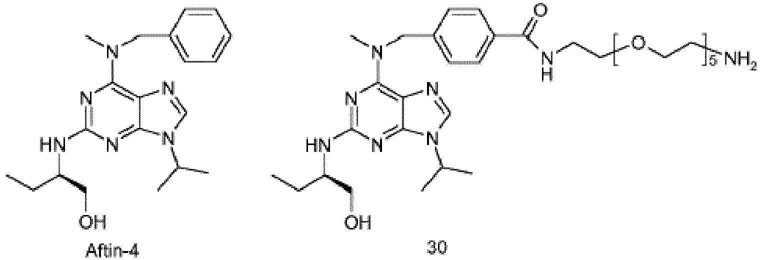 und deren Additionssalze mit pharmazeutisch geeigneten Säuren.

13. Verbindung der Formel (Ie): 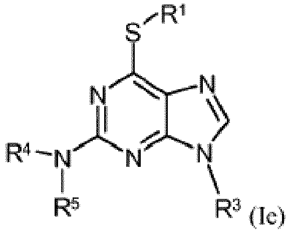 worin
R¹ eine Arylgruppe,
eine Heteroarylgruppe, eine -CH₂-Arylgruppe, eine -CH₂-Heteroarylgruppe ist, wobei das Aryl und Heteroaryl gegebenenfalls mit einem oder mehreren unter einem Halogenatom, einer Hydroxygruppe, einer OR⁶-Gruppe, einer SR⁶-Gruppe, einer NR⁶R⁷-Gruppe, ein CN-Gruppe, einer CONR⁶R⁷-Gruppe, einer SOR⁶-Gruppe, einer SO₂R⁶-Gruppe, einer Azidogruppe (-N₃), einer Arylgruppe, einer Heteroarylgruppe und einer (C₁-C₆)-Alkylgruppe ausgewählten Substituenten substituiert ist,
R⁵ eine (C₁-C₆)-Alkylgruppe oder eine (C₁-C₆)-Cycloalkylgruppe ist, wobei das Alkyl und das Cycloalkyl gegebenenfalls mit einem oder mehreren unter einem Halogenatom, einer Hydroxygruppe, einer Azidogruppe und einer NH₂-Gruppe ausgewählten Substituenten substituiert ist, ein oder mehrere der Kohlenstoffatome des Alkyls oder Cycloalkyls gegebenenfalls durch ein Stickstoffatom ersetzt sind,
R⁶ und R⁷ unabhängig voneinander ein Wasserstoffatom, eine (C₁-C₆)-Alkylgruppe, eine (CH₂)ₓ[O(CH₂)_{y}]_{z}-R⁸-Gruppe oder eine (CH₂)ₚNH-CO-(CH₂)_{q}-R⁸-Gruppe sind, wobei das Alkyl gegebenenfalls mit einem oder mehreren unter einem Halogenatom, einer Hydroxygruppe und einer R⁸-Gruppe ausgewählten Substituenten substituiert ist,
R⁸ eine Hydroxygruppe, eine NH₂-Gruppe, eine N(CH₃)₂-Gruppe, eine 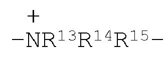 Gruppe oder eine 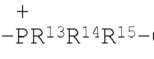 Gruppe ist,
R¹³, R¹⁴ und R¹⁵ unabhängig voneinander eine Arylgruppe oder eine (C₁-C₆)-Alkylgruppe sind,
R³ eine (C₁-C₆)-Alkylgruppe, eine (C₁-C₆)-Cycloalkylgruppe, eine Arylgruppe, eine -CH₂-Arylgruppe oder eine -CH₂-Heteroarylgruppe ist, wobei die Alkyl-, Cycloalkyl-, Aryl- und Heteroarylgruppe gegebenenfalls mit einem oder mehreren unter einem Halogenatom, einer Hydroxygruppe und einer NH₂-Gruppe ausgewählten Substituenten substituiert ist,
R⁴ ein Wasserstoffatom, eine (C₁-C₆)-Alkylgruppe oder eine (C₁-C₆)-Cycloalkylgruppe ist, wobei das Alkyl und das Cycloalkyl gegebenenfalls mit einem oder mehreren unter einem Halogenatom, einer Hydroxygruppe und einer NH₂-Gruppe ausgewählten Substituenten substituiert sind,
mit der Maßgabe, dass die Verbindung der Formel (Ie) sich von der folgenden Formel unterscheidet, 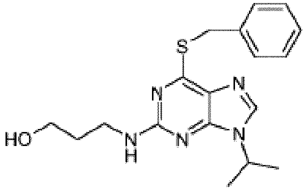 und deren Additionssalze mit pharmazeutisch geeigneten Säuren.

14. Verbindung nach einem der Ansprüche 10 bis 13, die unter den Verbindungen der folgenden Tabelle ausgewählt ist:
| Nr. | Struktur | Nr. | Struktur |
|---|---|---|---|
| | | | |
| Aftin-1 | 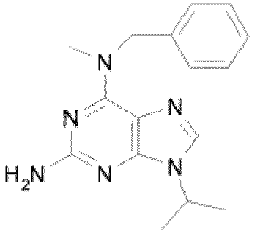 | 32 | 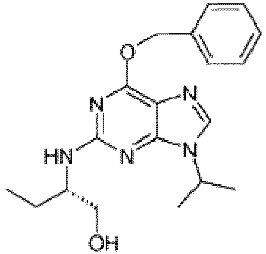 |
| 9 | 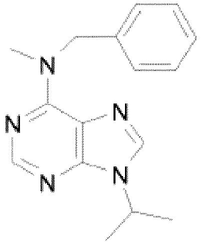 | 33 | 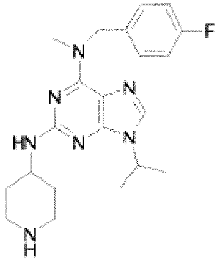 |
| Aftin-2 | 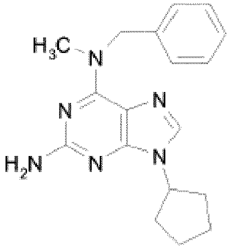 | 35 | 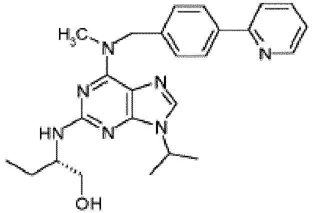 |
| 12 | 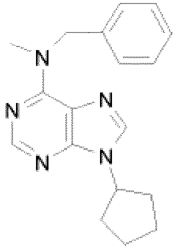 | 26 | 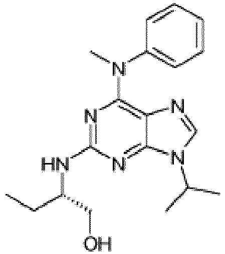 |
| 13 | 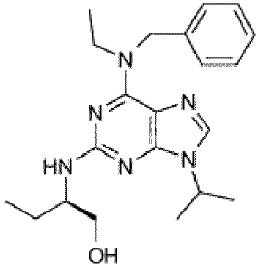 | Aftin-5 | 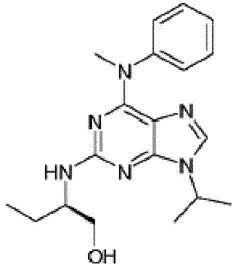 |
| 14 | 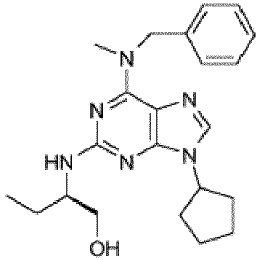 | 34 | 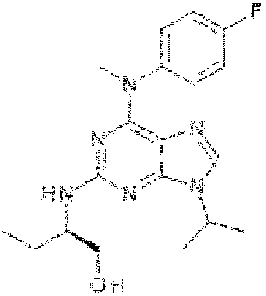 |
| 15 | 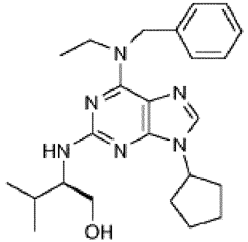 | 35 | 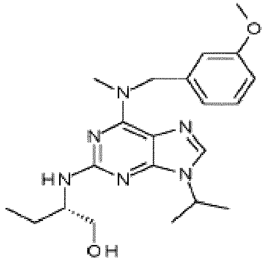 |
| 16 | 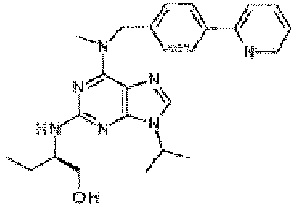 | 36 | 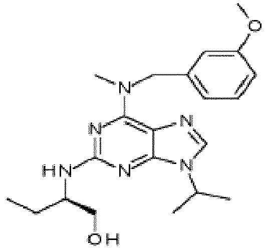 |
| 17 | 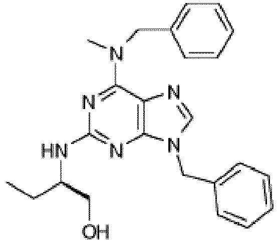 | 37 | 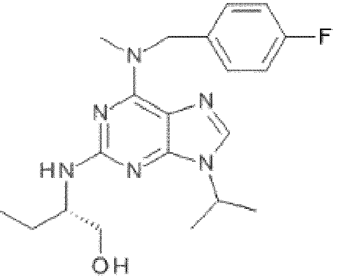 |
| 18 | 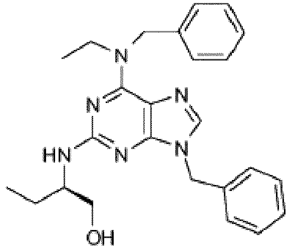 | 38 | 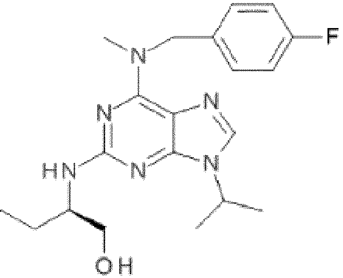 |
| 19 | 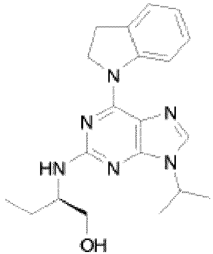 | 39 | 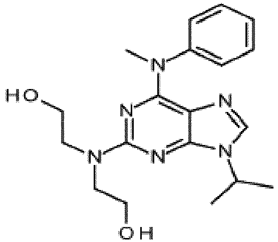 |
| 20 | 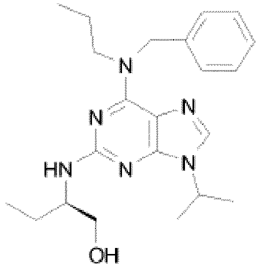 | 40 | 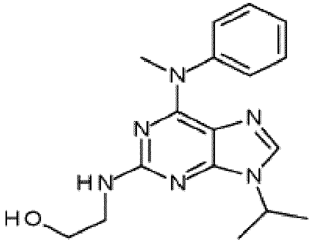 |
| | | 41 | 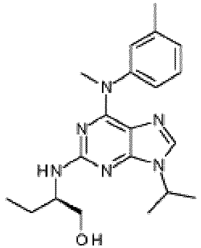 |
| 22 | 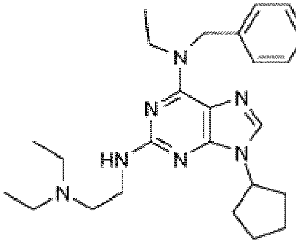 | 42 | 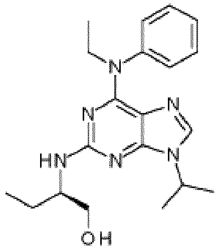 |
| 23 | 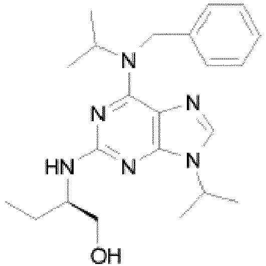 | | |
| 24 | 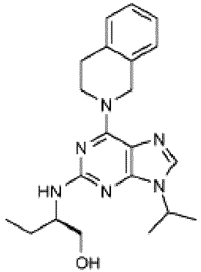 | 44 | 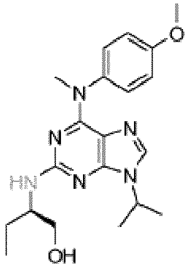 |
| 28 | 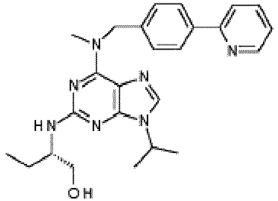 | 45 | 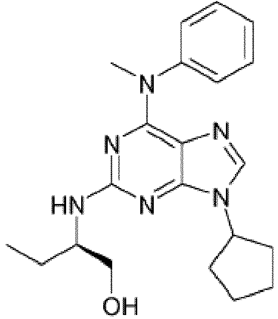 |
| 29 | 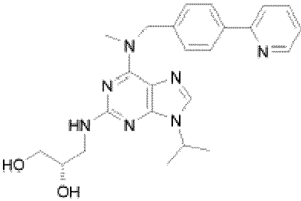 | 46 | 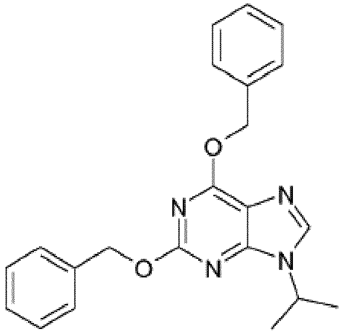 |
| 47 | 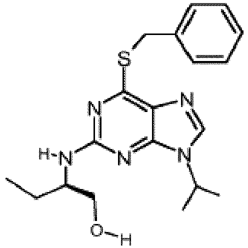 | | |
insbesondere Aftin 5.

15. Verfahren zum in vitro-Screenen von die Produktion eines β-Amyloidpeptids modulierenden Kandidatenverbindungen, welches die folgenden Stufen umfasst:
a) das Inkubieren von Zellen, die ein β-Amyloidpeptid produzieren können, mit einer induzierenden Verbindung, die aus der Gruppe ausgewählt ist, die aus den Verbindungen der in einem der Ansprüche 1 bis 8 offenbarten Verbindungen der Formeln (I), (Ia), (Ib), (Ic), (Id) und (Ie) besteht, und außerdem mit einer zu testenden Kandidatenverbindung, wobei eine Testprobe bereitgestellt wird,
b) das Messen der Konzentration eines β-Amyloidpeptids in der am Ende der Stufe a) erhaltenen Testprobe,
c) das Vergleichen der in Stufe b) gemessenen Konzentration des β-Amyloidpeptids mit einem Referenzwert des β-Amyloidpeptids, wenn Stufe a) in Abwesenheit der induzierenden Verbindung durchgeführt wird,
d) das Selektieren der Kandidatenverbindung, wenn die Kandidatenverbindung die Konzentration des β-Amyloidpeptids moduliert.

16. Verwendung einer in einem der Ansprüche 1 bis 8 offenbarten Verbindung der Formel (I), (Ia), (Ib), (Ic), (Id) und/oder (Ie) als Referenzverbindung in in vitro-Screeningverfahren und in vivo-Screeningverfahren in Tiermodellen, wobei sich das Tier von einem Menschen unterscheidet, die so ausgestaltet sind, dass sie Alzheimer-induzierende Verbindungen unter im menschlichen chemischen Exposom enthaltenden Molekülen nachweisen und deren Potenz messen.

## Revendications

1. Utilisation d'un composé de formule (I) : 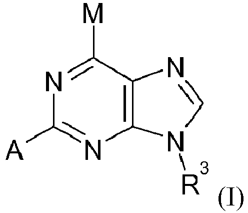 dans laquelle
M représente un groupe NR¹R², un groupe OR¹ ou un groupe SR¹,
A représente un groupe NR⁴R⁵, un groupe OR¹⁰ ou un atome d'hydrogène,
R¹ est un groupe aryle, un groupe hétéroaryle, un groupe -CH₂-aryle, un groupe -CH₂-hétéroaryle, lesdits aryle et hétéroaryle étant éventuellement substitués par un ou plusieurs substituants choisis parmi un atome d'halogène, un groupe hydroxyle, un groupe OR⁶, un groupe SR⁶, un groupe NR⁶R⁷, un groupe CN, un groupe CONR⁶R⁷, un groupe SOR⁶, un groupe SO₂R⁶, un groupe azido (-N₃), un groupe aryle, un groupe hétéroaryle et un groupe alkyle en C₁ à C₆,
R² est un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle et un groupe NH₂,
ou bien R¹ forme, conjointement avec R² et avec l'atome d'azote qui porte R¹ et R², un cycle hétérobicyclique,
R⁶ et R⁷, indépendamment l'un de l'autre, représentent un atome d'hydrogène, un groupe alkyle en C₁ à C₆, un groupe (CH₂)ₓ[O(CH₂)_{y}]_{z}-R⁸ ou un groupe (CH₂)ₚNH-CO-(CH₂)_{q}-R⁸, ledit alkyle étant éventuellement substitué par un ou plusieurs substituants choisis parmi un atome d'halogène, un groupe hydroxyle et un groupe R⁸,
R⁸ représente un groupe hydroxyle, un groupe NH₂, un groupe N(CH₃)₂, un groupe -N⁺R¹³R¹⁴R¹⁵ ou un groupe -P⁺R¹³R¹⁴R¹⁵, R¹³, R¹⁴ et R¹⁵, indépendamment les uns des autres, représentent un groupe aryle ou un groupe alkyle en C₁ à C₆,
x est un entier situé dans la plage allant de 0 à 4,
y est un entier situé dans la plage allant de 2 à 3,
z est un entier situé dans la plage allant de 1 à 10,
p est un entier situé dans la plage allant de 2 à 9,
q est un entier situé dans la plage allant de 2 à 8,
R³ est un groupe alkyle en C₁ à C₆, un groupe cycloalkyle en C₁ à C₆, un groupe aryle, un groupe -CH₂-aryle ou un groupe -CH₂-hétéroaryle, lesdits groupes alkyle, cycloalkyle, aryle et hétéroaryle étant éventuellement substitués par un ou plusieurs substituants choisis parmi un atome d'halogène, un groupe hydroxyle et un groupe NH₂, R⁴ est un atome d'hydrogène, un groupe alkyle en C₁ à C₆, un groupe cycloalkyle en C₁ à C₆, un groupe aryle ou un groupe hétéroaryle, lesdits alkyle et cycloalkyle étant éventuellement substitués par un ou plusieurs substituants choisis parmi un atome d'halogène, un groupe hydroxyle et un groupe NH₂, lesdits aryle et hétéroaryle étant éventuellement substitués par un ou plusieurs substituants choisis parmi un atome d'halogène, un groupe hydroxyle, un groupe NH₂ et un groupe NH-R⁹,
R⁹ est un groupe hétéroaryle éventuellement substitué par un ou plusieurs substituants choisis parmi un atome d'halogène, un groupe hydroxyle, un groupe NH₂ et un groupe hétéroaryle,
R⁵ est un atome d'hydrogène, un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle, un groupe azido et un groupe NH₂, un ou plusieurs des atomes de carbone dudit alkyle ou cycloalkyle étant éventuellement remplacés par un atome d'azote,
R¹⁰ est un atome d'hydrogène, un groupe alkyle en C₁ à C₆, un groupe cycloalkyle en C₁ à C₆, un groupe aryle, un groupe hétéroaryle, un groupe -CH₂-aryle ou un groupe -CH₂-hétéroaryle, ledit aryle étant éventuellement substitué par un ou plusieurs substituants choisis parmi un atome d'halogène, un groupe hydroxyle, un groupe OR⁶, un groupe SR⁶, un groupe NR⁶R⁷, un groupe CN, un groupe CONR⁶R⁷, un groupe SOR⁶, un groupe SO₂R⁶ et un groupe azido (N₃),
en particulier Aftin-3, Aftin-4 et Aftin-5, 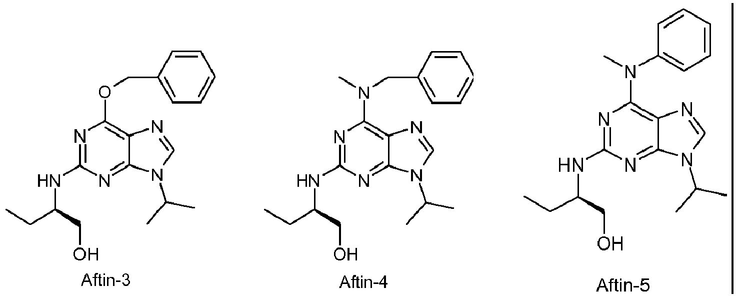 et de ses sels d'addition avec des acides pharmaceutiquement acceptables,
en tant qu'agent inducteur qui augmente la production de peptides β amyloïdes Aβ₄₂ pour le criblage in vitro et le criblage in vivo dans des modèles animaux, ledit animal étant différent d'un être humain, de composés qui modulent la production de peptides β amyloïdes Aβ₄₂.

2. Utilisation selon la revendication 1 d'un composé de formule (Ia) : 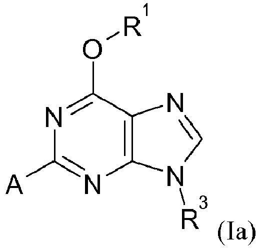 dans laquelle
A représente un groupe NR⁴R⁵ ou un groupe OR¹⁰,
R¹ est tel que défini dans la revendication 1, de préférence R¹ est un groupe -CH₂-aryle et mieux encore un groupe benzyle,
R⁵ est tel que défini dans la revendication 1, de préférence R⁵ est un groupe alkyle en C₁ à C₆ ou un groupe cycloalkyle en C₁ à C₆, lesdits alkyle et cycloalkyle étant éventuellement substitués par un seul groupe hydroxyle, mieux encore R⁵ est un groupe méthyle, éthyle, cyclopropyle, cyclobutyle, méthylcyclopropyle, méthylcyclobutyle ou 1-hydroxybutan-2-yle, en particulier un groupe 1-hydroxybutan-2-yle,
R¹⁰ est tel que défini dans la revendication 1, de préférence R¹⁰ est un groupe -CH₂-aryle, mieux encore un groupe benzyle,
R³ est un groupe alkyle en C₁ à C₆, un groupe cycloalkyle en C₁ à C₆, un groupe aryle, un groupe -CH₂-aryle ou un groupe -CH₂-hétéroaryle, lesdits groupes alkyle, cycloalkyle, aryle et hétéroaryle étant éventuellement substitués par un ou plusieurs substituants choisis parmi un atome d'halogène, un groupe hydroxyle et un groupe NH₂, de préférence R³ est un groupe alkyle en C₁ à C₆ ou un groupe cycloalkyle en C₁ à C₆, mieux encore un groupe méthyle, éthyle, cyclopropyle, cyclobutyle, méthylcyclopropyle, méthylcyclobutyle ou isopropyle, en particulier un groupe isopropyle,
R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle et un groupe NH₂, de préférence R⁴ est un atome d'hydrogène,
et de ses sels d'addition avec des acides pharmaceutiquement acceptables.

3. Utilisation selon la revendication 1 d'un composé de formule (Ib) : 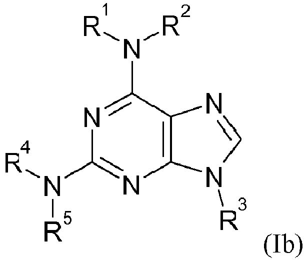 dans laquelle
R¹, R² et R⁵ sont tels que définis dans la revendication 1, R³ est un groupe alkyle en C₁ à C₆, un groupe cycloalkyle en C₁ à C₆, un groupe aryle, un groupe -CH₂-aryle ou un groupe -CH₂-hétéroaryle, lesdits groupes alkyle, cycloalkyle, aryle et hétéroaryle étant éventuellement substitués par un ou plusieurs substituants choisis parmi un atome d'halogène, un groupe hydroxyle et un groupe NH₂, R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle et un groupe NH₂,
et de ses sels d'addition avec des acides pharmaceutiquement acceptables.

4. Utilisation selon la revendication précédente d'un composé, dans laquelle les radicaux NR¹R² sont choisis parmi les radicaux suivants : 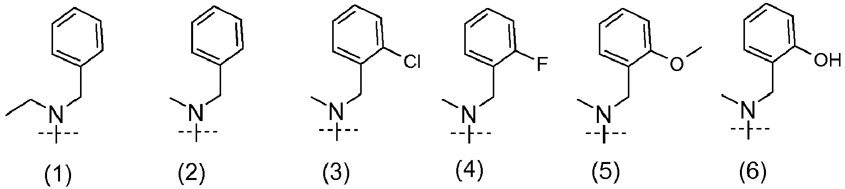 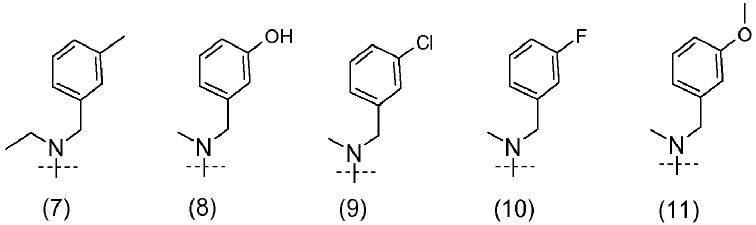 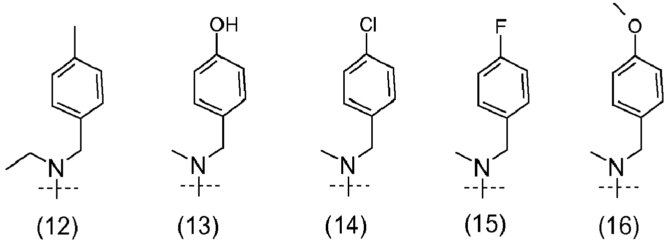 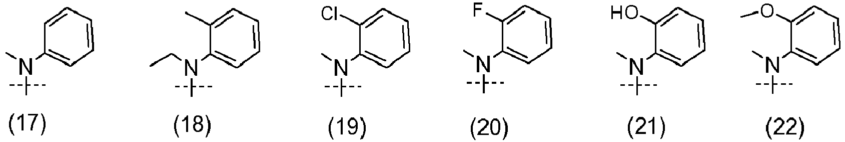 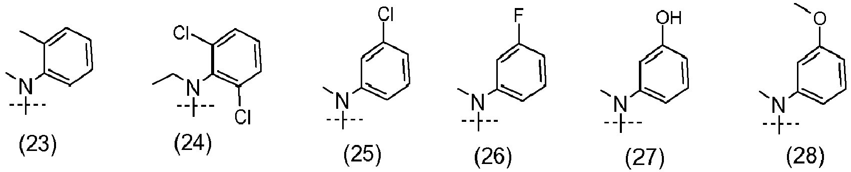 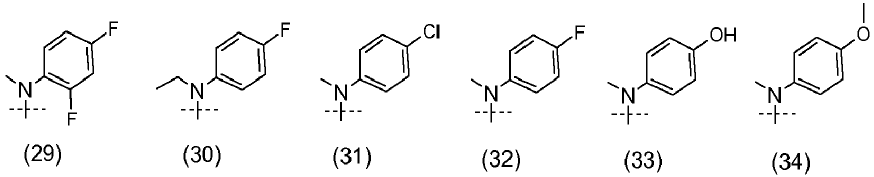 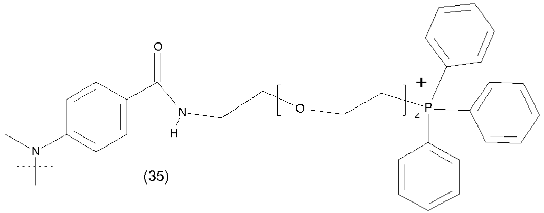 où z est situé dans la plage allant de 2 à 9, 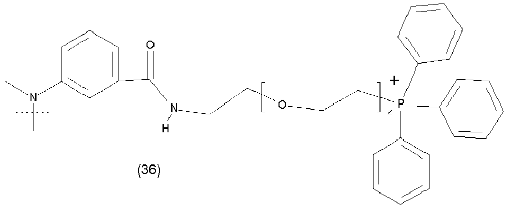 où z est situé dans la plage allant de 2 à 9, 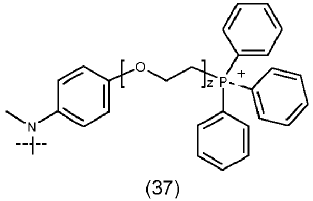 où z est situé dans la plage allant de 2 à 9, 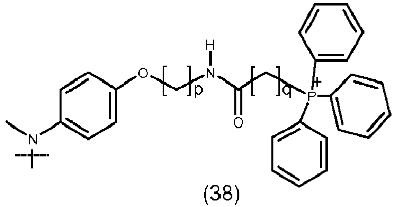 où p est situé dans la plage allant de 2 à 9 et q est situé dans la plage allant de 2 à 8, 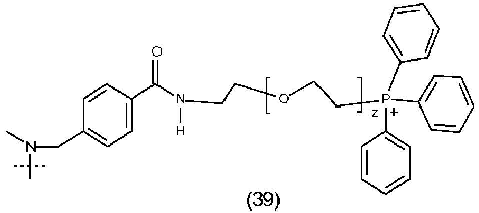 où z est situé dans la plage allant de 2 à 9, 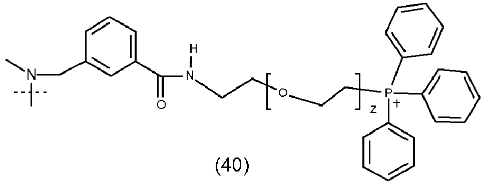 où z est situé dans la plage allant de 2 à 9, 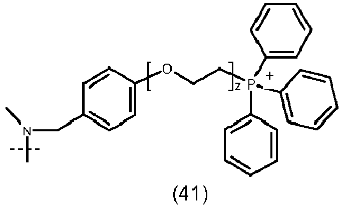 où z est situé dans la plage allant de 2 à 9, 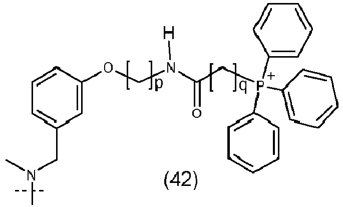 où p est situé dans la plage allant de 2 à 9 et q est situé dans la plage allant de 2 à 8,

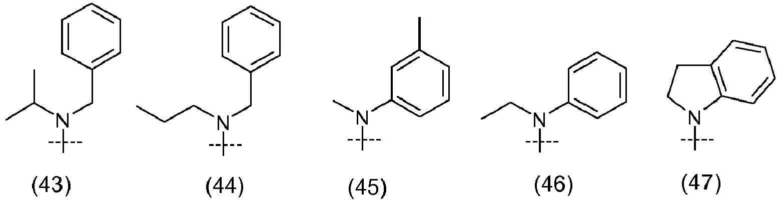

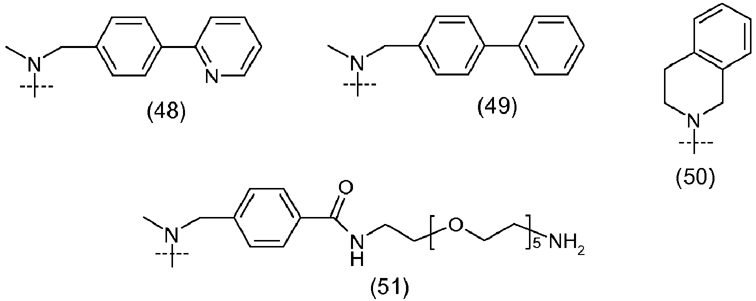

5. Utilisation selon la revendication précédente d'un composé, dans laquelle
le radical NR¹R² est tel que défini dans la revendication précédente, de préférence le radical NR¹R² est choisi parmi les radicaux de formules (1), (2), (11), (15), (17), (25), (32), (34) et (43) à (51), tels que définis dans la revendication 4,
R³ est un groupe alkyle en C₁ à C₆, un groupe cycloalkyle en C₁ à C₆ ou un groupe -CH₂-aryle, de préférence R³ est un groupe méthyle, éthyle, cyclopropyle, cyclobutyle, méthylcyclopropyle, méthylcyclobutyle, isopropyle, cyclopentyle ou benzyle, en particulier R³ est un groupe isopropyle, cyclopentyle ou benzyle,
R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs groupes hydroxyle, de préférence R⁴ est un groupe méthyle, éthyle, cyclopropyle, cyclobutyle, méthylcyclopropyle, méthylcyclobutyle ou 2-hydroxyéthyle ou un atome d'hydrogène, en particulier R⁴ est un groupe 2-hydroxyéthyle ou un atome d'hydrogène,
R⁵ est un atome d'hydrogène, un groupe alkyle en C₁ à C₆ ou un groupe cycloalkyle en C₁ à C₆, lesdits alkyle et cycloalkyle étant éventuellement substitués par un ou plusieurs groupes hydroxyle, un ou plusieurs des atomes de carbone desdits alkyle et cycloalkyle étant éventuellement remplacés par un atome d'azote, de préférence R⁵ est un groupe méthyle, éthyle, cyclopropyle, cyclobutyle, méthylcyclopropyle, méthylcyclobutyle, 1-hydroxybutan-2-yle, 1-hydroxy-3-méthylbutan-2-yle, 1,2-dihydroxypropan-3-yle, N-diéthylaminoéth-2-yle, pipéridin-4-yle ou 2-hydroxyéthyle, ou un atome d'hydrogène, en particulier R⁵ est un groupe 1-hydroxybutan-2-yle, 1-hydroxy-3-méthylbutan-2-yle, 1,2-dihydroxypropan-3-yle, N-diéthylaminoéth-2-yle, pipéridin-4-yle ou 2-hydroxyéthyle, ou un atome d'hydrogène,
et de ses sels d'addition avec des acides pharmaceutiquement acceptables.

6. Utilisation selon la revendication 4 d'un composé de formule (Ic) : 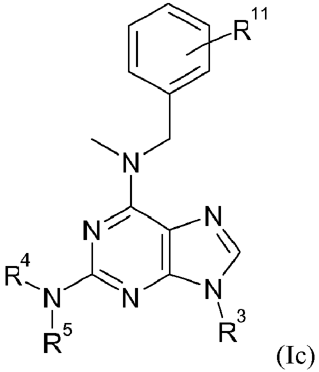 dans laquelle
R³ est un groupe alkyle en C₁ à C₆, un groupe cycloalkyle en C₁ à C₆, un groupe -CH₂-aryle ou un groupe -CH₂-hétéroaryle, de préférence R³ est un groupe méthyle, éthyle, cyclopropyle, cyclobutyle, méthylcyclopropyle, méthylcyclobutyle, isopropyle, cyclopentyle ou benzyle, en particulier R³ est un groupe isopropyle, cyclopentyle ou benzyle,
R⁴ est un atome d'hydrogène,
R⁵ est un atome d'hydrogène, un groupe alkyle en C₁ à C₆ ou un groupe cycloalkyle en C₁ à C₆, lesdits alkyle et cycloalkyle étant éventuellement substitués par un ou plusieurs groupes hydroxyle, un ou plusieurs des atomes de carbone dudit cycloalkyle étant éventuellement remplacés par un atome d'azote, de préférence R⁵ est un groupe méthyle, éthyle, cyclopropyle, cyclobutyle, méthylcyclopropyle, méthylcyclobutyle, 1-hydroxybutan-2-yle, 1,2-dihydroxypropan-3-yle ou pipéridin-4-yle, ou un atome d'hydrogène, en particulier R⁵ est un groupe 1-hydroxybutan-2-yle, 1,2-dihydroxypropan-3-yle, ou pipéridin-4-yle, ou un atome d'hydrogène,
R¹¹ est choisi parmi un atome d'halogène ou d'hydrogène, un groupe OR⁶, un groupe CONR⁶R⁷, un groupe aryle et un groupe hétéroaryle, de préférence R¹¹ est un groupe OR⁶, CONR⁶R⁷, pyridinyle, phényle, ou un atome de fluor ou d'hydrogène,
R⁶ et R⁷, indépendamment l'un de l'autre, représentent un atome d'hydrogène, un groupe alkyle en C₁ à C₆ ou un groupe (CH₂)ₓ[O(CH₂)_{y}]_{z}-R⁸ tel que défini dans la revendication 1, de préférence R⁶ et R⁷ représentent un atome d'hydrogène, ou un groupe méthyle ou (CH₂)₂[O(CH₂)₂]₅-NH₂,
et de ses sels d'addition avec des acides pharmaceutiquement acceptables.

7. Utilisation selon la revendication 4 d'un composé de formule (Id) : 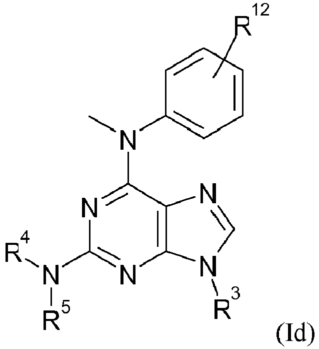 dans lequel
R³ est un groupe alkyle en C₁ à C₆, un groupe CH₂-aryle, un groupe -CH₂-hétéroaryle ou un groupe cycloalkyle en C₁ à C₆, de préférence R³ est un groupe méthyle, éthyle, cyclopropyle, cyclobutyle, méthylcyclopropyle, méthylcyclobutyle, isopropyle, cyclopentyle, en particulier R³ est un groupe isopropyle ou cyclopentyle,
R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs groupes hydroxyle, de préférence R⁴ est un groupe méthyle, éthyle, cyclopropyle, cyclobutyle, méthylcyclopropyle, méthylcyclobutyle ou 2-hydroxyéthyle ou un atome d'hydrogène, en particulier R⁴ est un groupe 2-hydroxyéthyle ou un atome d'hydrogène,
R⁵ est un groupe alkyle en C₁ à C₆ ou un groupe cycloalkyle en C₁ à C₆, lesdits alkyle et cycloalkyle étant éventuellement substitués par un ou plusieurs groupes hydroxyle, de préférence R⁵ est un groupe méthyle, éthyle, cyclopropyle, cyclobutyle, méthylcyclopropyle, méthylcyclobutyle, 1-hydroxybutan-2-yle ou 2-hydroxyéthyle, en particulier R⁵ est un groupe 1-hydroxybutan-2-yle ou 2-hydroxyéthyle,
R¹² est choisi parmi un atome d'halogène ou d'hydrogène, un groupe OR⁶ et un groupe alkyle en C₁ à C₆, de préférence R¹² est un atome d'hydrogène, de fluor ou de chlore, ou un groupe OR⁶ ou méthyle,
R⁶ est un groupe alkyle en C₁ à C₆, de préférence R⁶ est un groupe méthyle,
et de ses sels d'addition avec des acides pharmaceutiquement acceptables.

8. Utilisation selon la revendication 1 d'un composé de formule (Ie) : 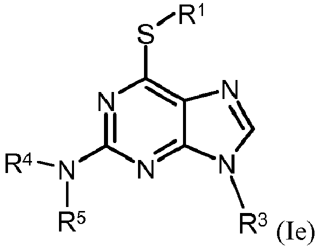 dans laquelle
R¹ et R⁵ sont tels que définis dans la revendication 1,
R³ est un groupe alkyle en C₁ à C₆, un groupe cycloalkyle en C₁ à C₆, un groupe aryle, un groupe -CH₂-aryle ou un groupe -CH₂-hétéroaryle, lesdits groupes alkyle, cycloalkyle, aryle et hétéroaryle étant éventuellement substitués par un ou plusieurs substituants choisis parmi un atome d'halogène, un groupe hydroxyle et un groupe NH₂,
R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle et un groupe NH₂,
et de ses sels d'addition avec des acides pharmaceutiquement acceptables.

9. Utilisation selon l'une quelconque des revendications précédentes, **caractérisée en ce que** ledit agent inducteur est destiné à cribler des composés visant à traiter la maladie d'Alzheimer.

10. Composé de formule (Ia) : 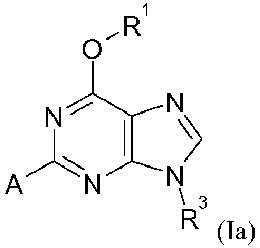 dans laquelle
A représente un groupe NR⁴R⁵ ou un groupe OR¹⁰,
R¹ est un groupe aryle, un groupe hétéroaryle, un groupe -CH₂-aryle, un groupe -CH₂-hétéroaryle, lesdits aryle et hétéroaryle étant éventuellement substitués par un ou plusieurs substituants choisis parmi un atome d'halogène, un groupe hydroxyle, un groupe OR⁶, un groupe SR⁶, un groupe NR⁶R⁷, un groupe CN, un groupe CONR⁶R⁷, un groupe SOR⁶, un groupe SO₂R⁶, un groupe azido (-N₃), un groupe aryle, un groupe hétéroaryle et un groupe alkyle en C₁ à C₆, de préférence R¹ est un groupe -CH₂-aryle, mieux encore un groupe benzyle,
R⁵ est un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle, un groupe azido et un groupe NH₂, un ou plusieurs des atomes de carbone dudit alkyle ou cycloalkyle étant éventuellement remplacés par un atome d'azote, de préférence R⁵ est un groupe alkyle en C₁ à C₆ ou un groupe cycloalkyle en C₁ à C₆, lesdits alkyle et cycloalkyle étant éventuellement substitués par un seul groupe hydroxyle, mieux encore R⁵ est un groupe méthyle, éthyle, cyclopropyle, cyclobutyle, méthylcyclopropyle, méthylcyclobutyle ou 1-hydroxybutan-2-yle, en particulier un groupe 1-hydroxybutan-2-yle,
R¹⁰ est un atome d'hydrogène, un groupe alkyle en C₁ à C₆, un groupe cycloalkyle en C₁ à C₆, un groupe aryle, un groupe hétéroaryle, un groupe -CH₂-aryle ou un groupe -CH₂-hétéroaryle, ledit aryle étant éventuellement substitué par un ou plusieurs substituants choisis parmi un atome d'halogène, un groupe hydroxyle, un groupe OR⁶, un groupe SR⁶, un groupe NR⁶R⁷, un groupe CN, un groupe CONR⁶R⁷, un groupe SOR⁶, un groupe SO₂R⁶ et un groupe azido (N₃), de préférence R¹⁰ est un groupe -CH₂-aryle, mieux encore un groupe benzyle,
R³ est un groupe alkyle en C₁ à C₆, un groupe cycloalkyle en C₁ à C₆, un groupe aryle, un groupe -CH₂-aryle ou un groupe -CH₂-hétéroaryle, lesdits groupes alkyle, cycloalkyle, aryle et hétéroaryle étant éventuellement substitués par un ou plusieurs substituants choisis parmi un atome d'halogène, un groupe hydroxyle et un groupe NH₂, de préférence R³ est un groupe alkyle en C₁ à C₆ ou un groupe cycloalkyle en C₁ à C₆, mieux encore un groupe méthyle, éthyle, cyclopropyle, cyclobutyle, méthylcyclopropyle, méthylcyclobutyle ou isopropyle, en particulier un groupe isopropyle,
R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle et un groupe NH₂, de préférence R⁴ est un atome d'hydrogène,
R⁶ et R⁷, indépendamment l'un de l'autre, représentent un atome d'hydrogène, un groupe alkyle en C₁ à C₆, un groupe (CH₂)ₓ[O(CH₂)_{y}]_{z}-R⁸ ou un groupe (CH₂)ₚNH-CO-(CH₂)_{q}-R⁸, ledit alkyle étant éventuellement substitué par un ou plusieurs substituants choisis parmi un atome d'halogène, un groupe hydroxyle et un groupe R⁸,
R⁸ représente un groupe hydroxyle, un groupe NH₂, un groupe N(CH₃)₂, un groupe -N⁺R¹³R¹⁴R¹⁵ ou un groupe -P⁺R¹³R¹⁴R¹⁵,
R¹³, R¹⁴ et R¹⁵, indépendamment les uns des autres, représentent un groupe aryle ou un groupe alkyle en C₁ à C₆,
x est un entier situé dans la plage allant de 0 à 4,
y est un entier situé dans la plage allant de 2 à 3,
z est un entier situé dans la plage allant de 1 à 10,
p est un entier situé dans la plage allant de 2 à 9,
q est un entier situé dans la plage allant de 2 à 8,
sous réserve que le composé de formule (Ia) soit différent d'Aftin-3, 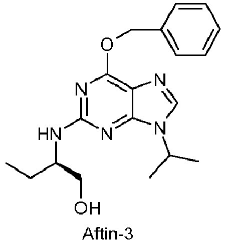 et ses sels d'addition avec des acides pharmaceutiquement acceptables.

11. Composé de formule (Ib) : 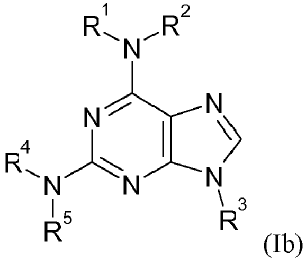 dans laquelle
le radical NR¹R² est choisi parmi les radicaux de formules (1), (2), (11), (15), (17) et (43) à (48), (50) et (51), tels que définis dans la revendication 4,
R³ est un groupe alkyle en C₁ à C₆, un groupe cycloalkyle en C₁ à C₆ ou un groupe -CH₂-aryle, de préférence R³ est un groupe méthyle, éthyle, cyclopropyle, cyclobutyle, méthylcyclopropyle, méthylcyclobutyle, isopropyle, cyclopentyle ou benzyle, en particulier R³ est un groupe isopropyle, cyclopentyle ou benzyle,
R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs groupes hydroxyle, de préférence R⁴ est un groupe méthyle, éthyle, cyclopropyle, cyclobutyle, méthylcyclopropyle, méthylcyclobutyle ou 2-hydroxyéthyle, ou un atome d'hydrogène, en particulier R⁴ est un groupe 2-hydroxyéthyle ou un atome d'hydrogène,
R⁵ est un atome d'hydrogène, un groupe alkyle en C₁ à C₆ ou un groupe cycloalkyle en C₁ à C₆, lesdits alkyle et cycloalkyle étant éventuellement substitués par un ou plusieurs groupes hydroxyle, un ou plusieurs des atomes de carbone desdits alkyle et cycloalkyle étant éventuellement remplacés par un atome d'azote, de préférence R⁵ est un groupe méthyle, éthyle, cyclopropyle, cyclobutyle, méthylcyclopropyle, méthylcyclobutyle, 1-hydroxybutan-2-yle, 1-hydroxy-3-méthylbutan-2-yle, 1,2-dihydroxypropan-3-yle, N-diéthylaminoéth-2-yle, pipéridin-4-yle ou 2-hydroxyéthyle, ou un atome d'hydrogène, en particulier R⁵ est un groupe 1-hydroxybutan-2-yle, 1-hydroxy-3-méthylbutan-2-yle, 1,2-dihydroxypropan-3-yle, N-diéthylaminoéth-2-yle, pipéridin-4-yle ou 2-hydroxyéthyle, ou un atome d'hydrogène,
sous réserve que le composé de formule (Ib) doit différent d'Aftin-4 et du composé 30, 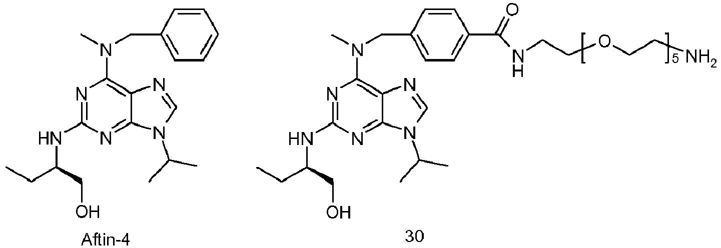 et ses sels d'addition avec des acides pharmaceutiquement acceptables.

12. Composé de formule (Ic) : 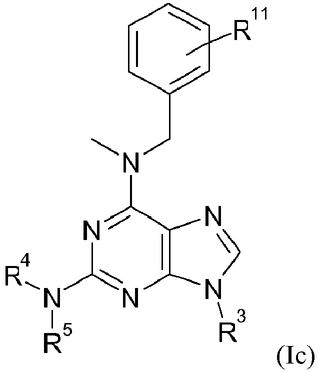 dans laquelle
R³ est un groupe alkyle en C₁ à C₆, un groupe cycloalkyle en C₁ à C₆, un groupe -CH₂-aryle ou un groupe -CH₂-hétéroaryle, de préférence R³ est un groupe méthyle, éthyle, cyclopropyle, cyclobutyle, méthylcyclopropyle, méthylcyclobutyle, isopropyle, cyclopentyle ou benzyle, en particulier R³ est un groupe isopropyle, cyclopentyle ou benzyle,
R⁴ est un atome d'hydrogène,
R⁵ est un atome d'hydrogène, un groupe alkyle en C₁ à C₆ ou un groupe cycloalkyle en C₁ à C₆, lesdits alkyle et cycloalkyle étant éventuellement substitués par un ou plusieurs groupes hydroxyle, un ou plusieurs des atomes de carbone dudit cycloalkyle étant éventuellement remplacés par un atome d'azote, de préférence R⁵ est un groupe méthyle, éthyle, cyclopropyle, cyclobutyle, méthylcyclopropyle, méthylcyclobutyle, 1-hydroxybutan-2-yle, 1,2-dihydroxypropan-3-yle ou pipéridin-4-yle, ou un atome d'hydrogène, en particulier R⁵ est un groupe 1-hydroxybutan-2-yle, 1,2-dihydroxypropan-3-yle, ou pipéridin-4-yle, ou un atome d'hydrogène,
R¹¹ est choisi parmi un atome d'halogène ou d'hydrogène, un groupe OR⁶, un groupe CONR⁶R⁷, un groupe aryle et un groupe hétéroaryle, de préférence R¹¹ est un groupe OR⁶, CONR⁶R⁷, pyridinyle, phényle, ou un atome de fluor ou d'hydrogène,
R⁶ et R⁷, indépendamment l'un de l'autre, représentent un atome d'hydrogène, un groupe alkyle en C₁ à C₆ ou un groupe (CH₂)ₓ[O(CH₂)_{y}]_{z}-R⁸ tel que défini dans la revendication 1, de préférence R⁶ et R⁷ représentent un atome d'hydrogène, ou un groupe méthyle ou (CH₂)₂[O(CH₂)₂]₅-NH₂,
sous réserve que le composé de formule (Ic) doit différent d'Aftin-4 et du composé 30, 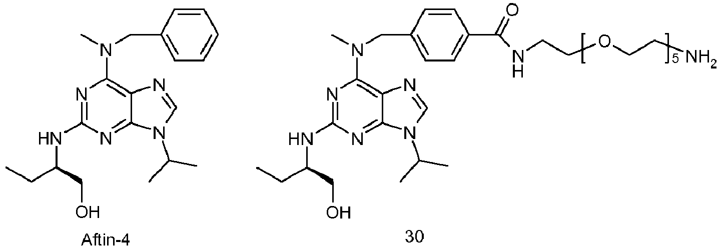 et ses sels d'addition avec des acides pharmaceutiquement acceptables.

13. Composé de formule (Ie) : 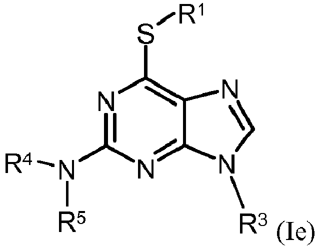 dans laquelle
R¹ est un groupe aryle, un groupe hétéroaryle, un groupe -CH₂-aryle, un groupe -CH₂-hétéroaryle, lesdits aryle et hétéroaryle étant éventuellement substitués par un ou plusieurs substituants choisis parmi un atome d'halogène, un groupe hydroxyle, un groupe OR⁶, un groupe SR⁶, un groupe NR⁶R⁷, un groupe CN, un groupe CONR⁶R⁷, un groupe SOR⁶, un groupe SO₂R⁶, un groupe azido (-N₃), un groupe aryle, un groupe hétéroaryle et un groupe alkyle en C₁ à C₆,
R⁵ est un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle, un groupe azido et un groupe NH₂, un ou plusieurs des atomes de carbone dudit alkyle ou cycloalkyle étant éventuellement remplacés par un atome d'azote,
R⁶ et R⁷, indépendamment l'un de l'autre, représentent un atome d'hydrogène, un groupe alkyle en C₁ à C₆, un groupe (CH₂)ₓ[O(CH₂)_{y}]_{z}-R⁸ ou un groupe (CH₂)ₚNH-CO-(CH₂)_{q}-R⁸, ledit alkyle étant éventuellement substitué par un ou plusieurs substituants choisis parmi un atome d'halogène, un groupe hydroxyle et un groupe R⁸,
R⁸ représente un groupe hydroxyle, un groupe NH₂, un groupe N(CH₃)₂, un groupe -N⁺R¹³R¹⁴R¹⁵ ou un groupe -P⁺R¹³R¹⁴R¹⁵,
R¹³, R¹⁴ et R¹⁵, indépendamment les uns des autres, représentent un groupe aryle ou un groupe alkyle en C₁ à C₆,
R³ est un groupe alkyle en C₁ à C₆, un groupe cycloalkyle en C₁ à C₆, un groupe aryle, un groupe -CH₂-aryle ou un groupe -CH₂-hétéroaryle, lesdits groupes alkyle, cycloalkyle, aryle et hétéroaryle étant éventuellement substitués par un ou plusieurs substituants choisis parmi un atome d'halogène, un groupe hydroxyle et un groupe NH₂,
R⁴ est un atome d'hydrogène, un groupe alkyle en C₁ à C₆ ou un groupe cycloalkyle en C₁ à C₆, lesdits alkyle et cycloalkyle étant éventuellement substitués par un ou plusieurs substituants choisis parmi un atome d'halogène, un groupe hydroxyle et un groupe NH₂,
sous réserve que le composé de formule (Ie) soit différent du composé suivant, 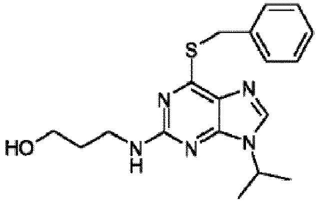 et ses sels d'addition avec des acides pharmaceutiquement acceptables.

14. Composé selon l'une quelconque des revendications 10 à 13, choisi parmi ceux divulgués dans le tableau suivant :
| N° | Structure | N° | Structure |
|---|---|---|---|
| Aftin-1 | 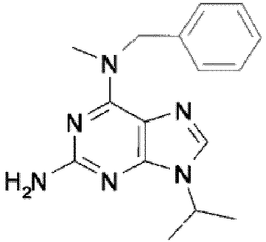 | 32 | 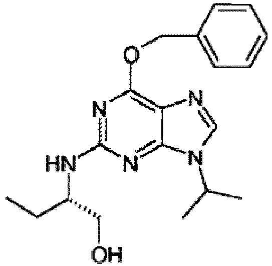 |
| 9 | 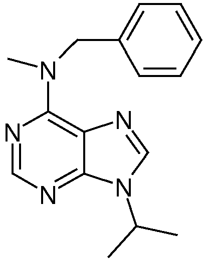 | 33 | 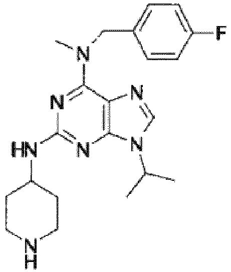 |
| Aftin-2 | 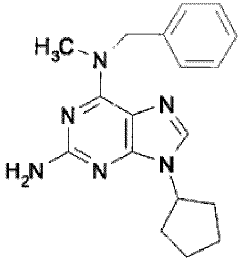 | 35 | 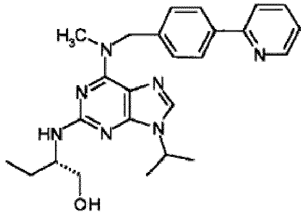 |
| 12 | 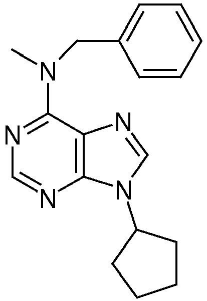 | 26 | 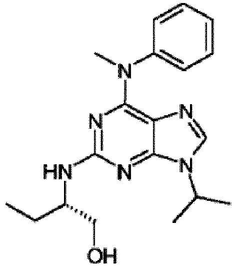 |
| 13 | 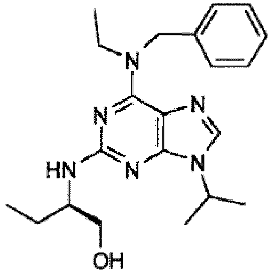 | Aftin-5 | 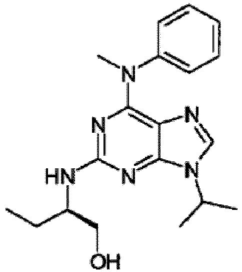 |
| 14 | 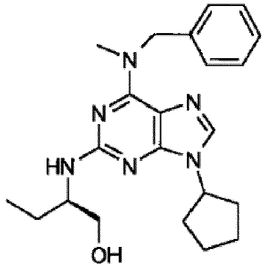 | 34 | 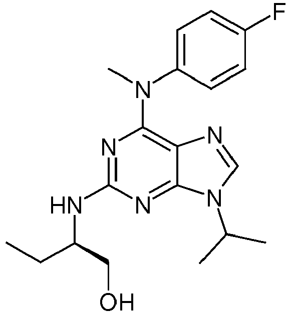 |
| 15 | 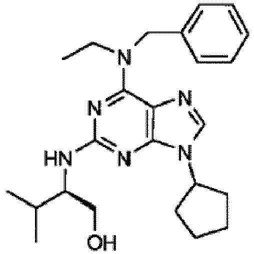 | 35 | 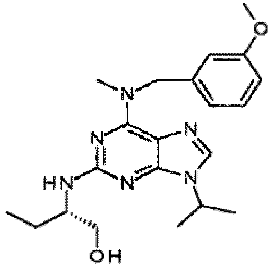 |
| 16 | 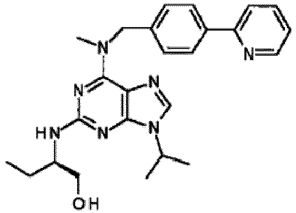 | 36 | 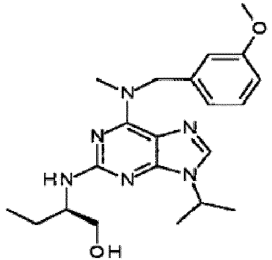 |
| 17 | 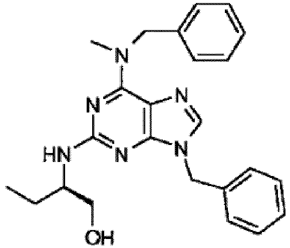 | 37 | 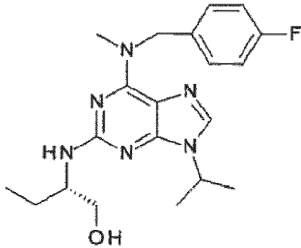 |
| 18 | 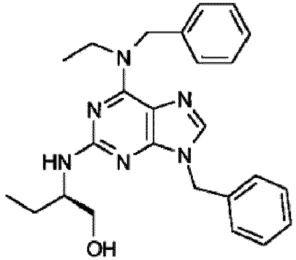 | 38 | 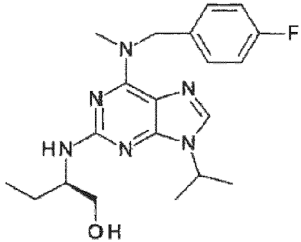 |
| 19 | 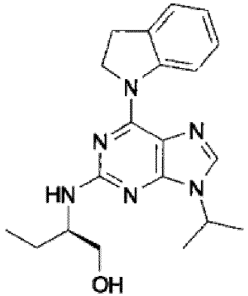 | 39 | 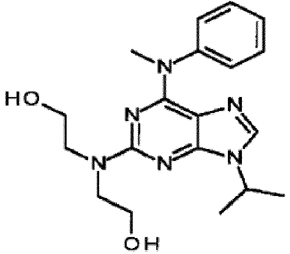 |
| 20 | 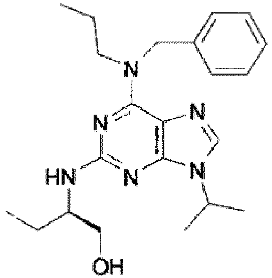 | 40 | 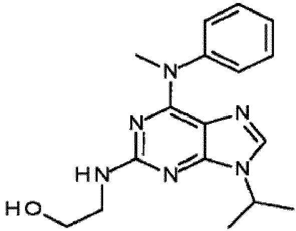 |
| | | 41 | 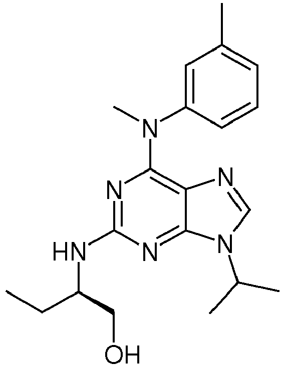 |
| 22 | 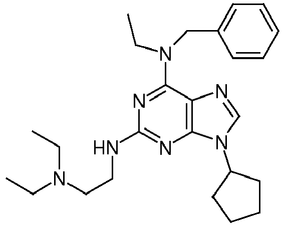 | 42 | 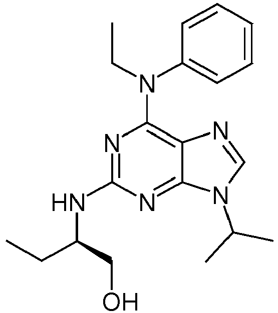 |
| 23 | 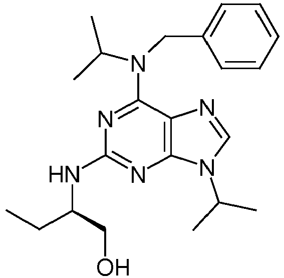 | | |
| 24 | 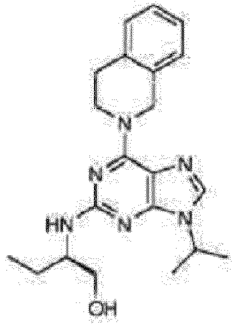 | 44 | 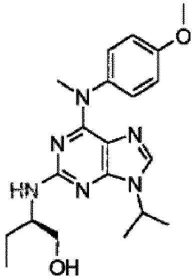 |
| 28 | 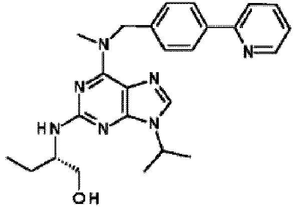 | 45 | 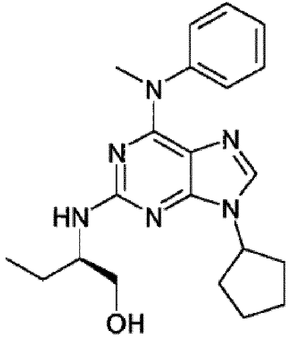 |
| 29 | 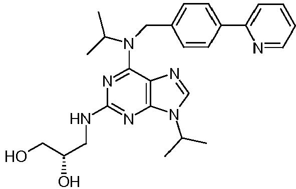 | 46 | 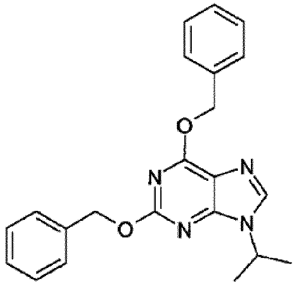 |
| 47 | 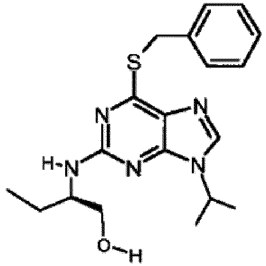 | | |
en particulier est Aftin-5.

15. Méthode de criblage in vitro de composés candidats qui modulent la production d'un peptide β-amyloïde, comprenant les étapes consistant à :
a) incuber des cellules capables de produire un peptide β-amyloïde avec un composé inducteur choisi dans l'ensemble constitué par les composés de formules (I), (Ia), (Ib), (Ic), (Id) et (Ie) tels que divulgués dans l'une quelconque des revendications 1 à 8, et en outre avec un composé candidat devant être testé, en conséquence de quoi un échantillon de test est obtenu,
b) mesurer le niveau d'un peptide β-amyloïde dans l'échantillon de test obtenu à la fin de l'étape a),
c) comparer le niveau dudit peptide β-amyloïde, mesuré à l'étape b), avec une valeur de référence dudit peptide β-amyloïde quand l'étape a) est effectuée en l'absence dudit composé inducteur,
d) sélectionner le composé candidat quand ledit composé candidat module le niveau dudit peptide β-amyloïde.

16. Utilisation d'un composé de formule (I), (Ia), (Ib), (Ic), (Id) et/ou (Ie) telle que divulguée dans l'une quelconque des revendications 1 à 8 en tant que composés de référence dans des méthodes de criblage in vitro et des méthodes de criblage in vivo dans des modèles animaux, ledit animal étant différent d'un être humain, conçues pour détecter des composés inducteurs d'Alzheimer parmi des molécules constituant l'exposome chimique humain et pour mesurer leur puissance.
